(19) Europäisches Patentamt
European Patent Office
Office européen des brevets

(11) EP 4 211 663 B1

(12) **EUROPEAN PATENT SPECIFICATION**

(45) Date of publication and mention of the grant of the patent:
**18.02.2026 Bulletin 2026/08**

(21) Application number: **21777496.7**

(22) Date of filing: **13.09.2021**

(51) International Patent Classification (IPC):
***G06F 18/22*** (2023.01) ***G06V 20/69*** (2022.01)
***G06V 10/44*** (2022.01) ***G06F 18/25*** (2023.01)
***G06F 18/2413*** (2023.01) ***C07K 16/28*** (2006.01)
***A61K 47/68*** (2017.01)

(52) Cooperative Patent Classification (CPC):
**C07K 16/2827; A61K 47/68037; A61K 47/6849; A61K 47/6851; A61K 47/6889; G06F 18/22; G06F 18/24133; G06F 18/254; G06V 10/454; G06V 20/695; G06V 20/698**

(86) International application number:
**PCT/EP2021/075116**

(87) International publication number:
**WO 2022/053685 (17.03.2022 Gazette 2022/11)**

(54) **A SCORING METHOD FOR AN ANTI-B7H4 ANTIBODY-DRUG CONJUGATE THERAPY**

BEWERTUNGSVERFAHREN FÜR EINE ANTI-B7H4-ANTIKÖRPER-WIRKSTOFF-KONJUGATTHERAPIE

MÉTHODE DE NOTATION ASSOCIÉE À UNE THÉRAPIE PAR CONJUGUÉ ANTICORPS-ANTI-B7H4-MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2020 US 202063077607 P**

(43) Date of publication of application:
**19.07.2023 Bulletin 2023/29**

(73) Proprietor: **MedImmune Limited**
**Cambridge CB2 0AA (GB)**

(72) Inventors:
- **SCHMIDT, Guenter**
  **80636 Munich (DE)**
- **BRIEU, Nicolas**
  **80636 Munich (DE)**
- **SPITZMUELLER, Andreas**
  **80636 Munich (DE)**
- **KAPIL, Ansh**
  **80636 Munich (DE)**
- **TAN, Tze, Heng**
  **80636 Munich (DE)**
- **WORTMANN, Philipp**
  **80636 Munich (DE)**

(74) Representative: **Mewburn Ellis LLP**
**Aurora Building**
**Counterslip**
**Bristol BS1 6BX (GB)**

(56) References cited:
**EP-A1- 2 832 845** **WO-A1-2015/124777**
**WO-A1-2019/197509** **WO-A1-2020/081343**

- **JOSEPH R. PODOJIL ET AL: "Potential targeting of B7-H4 for the treatment of cancer", IMMUNOLOGICAL REVIEWS, vol. 276, no. 1, 1 March 2017 (2017-03-01), US, pages 40 - 51, XP055510318, ISSN: 0105-2896, DOI: 10.1111/imr.12530**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

CROSS-REFERENCE TO RELATED APPLICATIONS

This application claims priority benefit of U.S. Provisional Patent Application No. 63/077,607, filed September 12, 2020.

INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 14,351 Byte ASCII (Text) file named " B7H4-400-WO-PCT_seq-listing.txt" created on September 2, 2021.

TECHNICAL FIELD

The present invention relates to a method for computing a score indicative of how a cancer patient will respond to a therapy that uses an anti-B7H4 antibody-drug conjugate.

BACKGROUND

Assessing a cancer patient's response probability to a given treatment is an essential step in determining a cancer patient's treatment regimen. Such an assessment is often based on histological analysis of tissue samples from a cancer patient and involves identifying and classifying cancers using standard grading schemes. Immunohistochemical (IHC) staining can be used to distinguish marker-positive cells that express a particular protein from marker-negative cells that do not express the protein. IHC staining typically involves multiple dyes, which includes one or more dyes connected to protein-specific antibodies and another dye that is a counterstain. A common counterstain is hematoxylin, which labels DNA and thus stains nuclei.

A protein specific stain or biomarker can be used to identify the regions of the tissue of the cancer patient that are likely to exhibit a response to a predetermined therapy. For example, a biomarker that stains epithelial cells can help to identify the suspected tumor regions. Then other protein specific biomarkers are used to characterize the cells within the cancerous tissue. The cells stained by a specific biomarker can be identified and quantified, and subsequently a score indicating the number of positively stained cells and negatively stained cells can be visually estimated by pathologists. This score can then be compared to scores of other cancer patients that have been calculated in the same way. If the response of these other patients to a given cancer treatment is known, the pathologist can predict, based on a comparison of the score calculated for the cancer patient with the scores of the other patients, how likely the cancer patient is to respond to a given treatment. However, visual assessment by pathologists is prone to variability and subjectivity.

One promising cancer treatment involves an antibody-drug conjugate (ADC) having a drug with cytotoxicity conjugated to an antibody, whose antigen is expressed on the surface of cancer cells. The ADC binds to the antigen and undergoes cellular internalization so as to deliver the drug selectively to cancer cells and to accumulate the drug within those cancer cells and kill them. A computer-based method is sought for generating a repeatable and objective score indicating a cancer patient's response to a treatment involving a therapeutic B7-H4 antibody-drug conjugate.

A relevant prior art document is WO 2015/124777 A which describes a medical image analysis method for identifying biomarker- positive tumour cells. The method comprises reading a first digital image of tumour cells stained with a nucleus stain and a second digital image of the same cells stained with a stain that selectively stains the biomarker. The former is used to identify the position of nuclei, and the latter is used to identify cell membranes that comprise the biomarker, the combination of a nucleus and a biomarker-stained cell membrane surrounding the identified nucleus is identified as a biomarker positive tumour cell

SUMMARY

A method for predicting how a cancer patient will respond to a therapy involving an antibody drug conjugate (ADC) involves computing a response score based on single-cell ADC scores for each cancer cell. The ADC includes an ADC payload and an ADC antibody that targets a protein on each cancer cell. A tissue sample is immunohistochemically stained using a dye linked to a diagnostic antibody that binds to the protein on the cancer cells in the tissue sample. A digital image of the tissue sample is acquired. Image analysis is performed on the digital image to detect the cancer cells using a convolutional neural network. For each cancer cell, a single-cell ADC score is computed based on the staining intensities of the dye in the membrane and/or cytoplasm of the cancer cell and/or in the membranes and cytoplasms of other cancer cells that are closer than a predefined distance to the cancer cell. A response score is generated that predicts the response

of the cancer patient to the ADC therapy by aggregating all single-cell ADC scores of the tissue sample using a statistical operation. Patients having a response score higher than a predetermined threshold are recommended for a therapy involving the ADC.

In one embodiment, a method of generating a score indicative of a survival probability of a cancer patient treated with an antibody drug conjugate (ADC) involves computing single-cell ADC scores. A tissue sample of the cancer patient is immunohistochemically stained using a dye linked to a diagnostic antibody. The ADC includes an ADC payload and an ADC antibody that targets the B7-H4 protein on cancer cells. The diagnostic antibody binds to the B7-H4 protein on cancer cells in the tissue sample. A digital image of the tissue sample is acquired, and cancer cells in the digital image are detected using image analysis. For each cancer cell, a single-cell ADC score is computed based on the staining intensity of the dye in the membrane. The single-cell ADC score may also optionally be based on the staining intensity of the dye in the cytoplasm of the cancer cell, as well as on the staining intensities of the dye in the membranes and the cytoplasms of other cancer cells that are closer than a predefined distance to the cancer cell. The resulting Quantitative Continuous Score (QCS) is indicative of the survival probability of the cancer patient and is generated by aggregating all single-cell ADC scores of the tissue sample using a statistical operation. The aggregating of all single-cell ADC scores is performed by determining the mean, determining the median, or determining a quantile with a predefined percentage. In another aspect, the aggregation of all single-cell ADC scores involves a threshold operation using a predefined threshold. All cells having a single-cell ADC score larger than the predefined threshold are labeled single-cell ADC positive. The aggregation is performed by determining the number of single-cell ADC positive cells divided by the number of all cancer cells.

In one embodiment, a method of predicting a response of a cancer patient to an antibody drug conjugate (ADC) involves detecting cancer cells and computing a single-cell ADC score for each cancer cell. The ADC includes an ADC payload and an ADC antibody that targets a protein on a cancer cell. The protein is B7-H4. A tissue sample is immunohistochemically stained using a dye linked to a diagnostic antibody. The diagnostic antibody binds to the protein on the cancer cells in the tissue sample. A digital image of the tissue sample is acquired, and cancer cells are detected in the digital image. For each cancer cell, a single-cell ADC score is computed based on the staining intensity of the dye in the membrane. The single-cell ADC score may also optionally be based on the staining intensity of the dye in the cytoplasm of the cancer cell, and based on the staining intensities of the dyes in the membranes and cytoplasms of other cancer cells that are closer than a predefined distance to the cancer cell. The staining intensity of each membrane is computed based on the average optical density of a brown diaminobenzidine (DAB) signal in pixels of the membrane, and the staining intensity of each cytoplasm is computed based on the average optical density of the brown DAB signal in pixels of the cytoplasm. The response of the cancer patient to the ADC is predicted based on an aggregation of all single-cell ADC scores of the tissue sample using a statistical operation.

In some embodiments, a patient is score-positive if at least 90% of tumor cells have a membrane optical density of 5 or greater. In some embodiments, a patient is score-positive if at least 90% of tumor cells have a membrane optical density of 6 or greater. In some embodiments, a patient is score-positive if at least 90% of tumor cells have a membrane optical density of 7 or greater. In some embodiments, a patient is score-positive if at least 90% of tumor cells have a membrane optical density of 8 or greater. In some embodiments, a patient is score-positive if at least 90% of tumor cells have a membrane optical density of 9 or greater. In some embodiments, a patient is score-positive if at least 90% of tumor cells have a membrane optical density of 10 or greater. In some embodiments, a patient is score-positive if at least 90% of tumor cells have a membrane optical density of 15 or greater. In some embodiments, a patient is score-positive if at least 90% of tumor cells have a membrane optical density of 20 or greater. In some embodiments a patient is score-positive if at least 90% of tumor cells have a membrane optical density of 25 or greater.

In some embodiments, a patient is score-positive if at least 50% of tumor cells have a membrane optical density of 5 or greater, 8 or greater, or 25 or greater. In some embodiments, a patient is score-positive if at least 50% of tumor cells have a membrane optical density of 8 or greater. In some embodiments, a patient is score-positive if at least 60% of tumor cells have a membrane optical density of 5 or greater, 8 or greater, or 25 or greater. In some embodiments, a patient is score-positive if at least 60% of tumor cells have a membrane optical density of 8 or greater. In some embodiments, a patient is score-positive if at least 70% of tumor cells have a membrane optical density of 5 or greater, 8 or greater, or 25 or greater. In some embodiments, a patient is score-positive if at least 70% of tumor cells have a membrane optical density of 8 or greater. In some embodiments, a patient is score-positive if at least 80% of tumor cells have a membrane optical density of 5 or greater, 8 or greater, or 25 or greater. In some embodiments, a patient is score-positive if at least 80% of tumor cells have a membrane optical density of 8 or greater. In some embodiments, a patient is score-positive if at least 90% of tumor cells have a membrane optical density of 5 or greater, 8 or greater, or 25 or greater. In some embodiments, a patient is score-positive if at least 90% of tumor cells have a membrane optical density of 8 or greater. In some embodiments, a patient is score-positive if at least 95% of tumor cells have a membrane optical density of 5 or greater, 8 or greater, or 25 or greater. In some embodiments, a patient is score-positive if at least 95% of tumor cells have a membrane optical density of 8 or greater.

In some embodiments, a patient is score-positive if the density of tumor cells with a membrane optical density of at least 8 is 500 cells/mm2 or greater. In some embodiments, a patient is score-positive if the density of tumor cells with a

membrane optical density of at least 8 is 1000 cells/mm2 or greater. In some embodiments, a patient is score-positive if the density of tumor cells with a membrane optical density of at least 8 is 1250 cells/mm2 or greater. In some embodiments, a patient is score-positive if the density of tumor cells with a membrane optical density of at least 8 is 1500 cells/mm2 or greater. In some embodiments, a patient is score-positive if the density of tumor cells with a membrane optical density of at least 8 is 1600 cells/mm2 or greater. In some embodiments, a patient is score-positive if the density of tumor cells with a membrane optical density of at least 8 is 1670 cells/mm2 or greater. In some embodiments, a patient is score-positive if the density of tumor cells with a membrane optical density of at least 8 is 1700 cells/mm2 or greater. In some embodiments, a patient is score-positive if the density of tumor cells with a membrane optical density of at least 8 is 1800 cells/mm2 or greater. In some embodiments, a patient is score-positive if the density of tumor cells with a membrane optical density of at least 8 is 1900 cells/mm2 or greater. In some embodiments, a patient is score-positive if the density of tumor cells with a membrane optical density of at least 8 is 2000 cells/mm2 or greater. In some embodiments, a patient is score-positive if the density of tumor cells with a membrane optical density of at least 8 is 3000 cells/mm2 or greater.

In some embodiments, a patient is score-positive if the spatial proximity score is 90 or greater. In some embodiments, a patient is score-positive if the spatial proximity score is 91 or greater. In some embodiments, a patient is score-positive if the spatial proximity score is 92 or greater. In some embodiments, a patient is score-positive if the spatial proximity score is 93 or greater. In some embodiments, a patient is score-positive if the spatial proximity score is 94 or greater. In some embodiments, a patient is score-positive if the spatial proximity score is 95 or greater. In some embodiments, a patient is score-positive if the spatial proximity score is 96 or greater. In some embodiments, a patient is score-positive if the spatial proximity score is 97 or greater. In some embodiments, a patient is score-positive if the spatial proximity score is 98 or greater. In some embodiments, a patient is score-positive if the spatial proximity score is 99 or greater. In some embodiments, a patient is score-positive if the spatial proximity score is 99.5 or greater. In some embodiments, a patient is score-positive if the spatial proximity score is 99.8 or greater. In the above embodiments, the spatial proximity score is calculated as follows: spatial proximity score = ([number of tumor cells with OD>8] + [number of tumor cells with OD<=8 which are in the 25um neighborhood of at least one tumor cell with OD>8])/[number of all tumor cells].

In another embodiment, a method of identifying a cancer patient who will exhibit a predetermined response to an antibody drug conjugate (ADC) involves generating a response score. The ADC includes an ADC payload and an ADC antibody that targets a protein on a cancer cell. A tissue sample of the cancer patient is immunohistochemically stained using a dye linked to a diagnostic antibody that binds to the protein on the cancer cells in the tissue sample. A digital image of the tissue sample is acquired, and cancer cells in the digital image are detected using a convolutional neural network. For each cancer cell, a single-cell ADC score is computed based on staining intensity of the dye in the membrane. The single-cell ADC score may also optionally be based on the staining intensity of the dye in the cytoplasm of the cancer cell, and on the staining intensities of the dye in the membranes and the cytoplasms of other cancer cells that are closer than a predefined distance to the cancer cell for which the single-cell score is computed. The QCS score is a response score generated by aggregating all single-cell ADC scores of the tissue sample using a statistical operation. The cancer patient is identified as one who will exhibit the predetermined response to the ADC based on whether the QCS score exceeds a threshold. Patients exhibiting a QCS score larger than the threshold are considered QCS Score Positive, and all other patients are considered QCS Score Negative. The predetermined response is a reduction in average tumor size. In another aspect, the difference between the QCS score to the threshold is indicative of the probability that the cancer patient is correctly identified as one who will exhibit the predetermined response to the ADC. A small difference indicates a low probability, whereas a large difference indicates a high probability that the cancer patient is accurately identified.

In one embodiment, a method of treating a cancer patient with an antibody drug conjugate (ADC) involves generating the QCS score in the form of a treatment score. The ADC includes an ADC payload and an ADC antibody that targets a protein on a cancer cell. The protein is B7-H4. A tissue sample of the cancer patient is immunohistochemically stained using a dye linked to a diagnostic antibody that binds to the protein on the cancer cells in the tissue sample. A digital image of the tissue sample is acquired, and cancer cells in the digital image are detected. For each cancer cell, a single-cell ADC score is calculated based on the staining intensity of the dye in the membrane. The single-cell ADC score may also optionally be based on the staining intensity of the dye in the cytoplasm of the cancer cell, as well as on the staining intensities of the dyes in the membranes and cytoplasms of other cancer cells that are closer than a predefined distance to the cancer cell. The treatment score is generated by aggregating all single-cell ADC scores of the tissue sample using a statistical operation. A therapy involving the ADC is administered to the cancer patient if the treatment score exceeds a predetermined threshold.

In another embodiment, a method of treating a cancer patient with an antibody drug conjugate (ADC) is performed by a clinician who administers a therapy. The ADC includes an ADC payload and an ADC antibody that targets a protein on a cancer cell. The protein is B7-H4. The therapy involving the ADC is administered to the cancer patient if a response score exceeds a predetermined threshold. The QCS response score was generated by aggregating single-cell ADC scores of a tissue sample of the cancer patient using a statistical operation. Each single-cell ADC score was computed for each cancer cell based on staining intensity of a dye in the membrane. The single-cell ADC score may also

optionally be based on the staining intensity of the dye in the cytoplasm of each cancer cell and also based on the staining intensities of the dye in the membranes and cytoplasms of other cancer cells that are closer than a predefined distance to each cancer cell for which the single-cell ADC score was computed. The cancer cells were detected in a digital image of the tissue sample of the cancer patient. The tissue sample was immunohistochemically stained using the dye linked to a diagnostic antibody that binds to the protein on the cancer cells in the tissue sample.

Other embodiments and advantages are described in the detailed description below. This summary does not purport to define the invention. The invention is defined by the claims.

## BRIEF DESCRIPTION OF THE DRAWINGS

The accompanying drawings, where like numerals indicate like components, illustrate embodiments of the invention.

FIG. 1 shows the full nucleotide sequence encoding for human B7-H4 (SEQ ID No: 1).
FIG. 2 shows nucleotide coding sequence for human B7-H4 (SEQ ID No: 2).
FIG. 3 shows the polypeptide sequence of human B7-H4 (SEQ ID No: 3).
FIG. 4 shows the amino acid sequence (SEQ ID No: 4) of the heavy chain HCDR1 of the anti-B7H4 antibody.
FIG. 5 shows the amino acid sequence (SEQ ID No: 5) of the heavy chain HCDR2 of the anti-B7H4 antibody.
FIG. 6 shows the amino acid sequence (SEQ ID No: 6) of the heavy chain HCDR3 of the anti-B7H4 antibody.
FIG. 7 shows the amino acid sequence (SEQ ID No: 7) of the light chain LCDR1 of the anti-B7H4 antibody.
FIG. 8 shows the amino acid sequence (SEQ ID No: 8) of the light chain LCDR2 of the anti-B7H4 antibody.
FIG. 9 shows the amino acid sequence (SEQ ID No: 9) of the light chain LCDR3 of the anti-B7H4 antibody.
FIG. 10 shows the amino acid sequence (SEQ ID No: 10) of a heavy chain variable region (VH) of the anti-B7H4 antibody.
FIG. 11 shows the amino acid sequence (SEQ ID No: 11) of a light chain variable region (VL) of the anti-B7H4 antibody.
FIG. 12 shows the amino acid sequence (SEQ ID No: 12) of a heavy chain of the anti-B7H4 antibody.
FIG. 13 shows the amino acid sequence (SEQ ID No: 13) of a light chain of the anti-B7H4 antibody.
FIG. 14 is a flowchart of steps by which an analysis system analyzes digital images of tissue from a cancer patient and predicts how the cancer patient will likely respond to a therapy involving an anti-B7H4 antibody-drug conjugate.
FIG. 15 shows digital images illustrating the image analysis process of step 2 of FIG. 14.
FIG. 16 illustrates image analysis steps in which nucleus objects of cancer cells are detected.
FIG. 17 illustrates image analysis steps in which nucleus objects are used to detect membranes.
FIG. 18 illustrates image analysis steps in which membrane objects of cancer cells are detected.
FIG. 19 is a screenshot of the results of the image analysis steps in an image analysis software environment.
FIG. 20 shows a script used for image analysis segmentation and the cell object measurements obtained using the script.
FIG. 21 shows sample quantitative results of staining intensities from image analysis using gray values of membrane and cytoplasm pixels.
FIG. 22 lists the exemplary quantitative amounts of staining on the membranes and in the cytoplasms of the image of FIG. 21.
FIG. 23 illustrates the mechanism by which an anti-B7H4 ADC therapy kills cancer cells.
FIG. 24 illustrates the calculation of the single-cell ADC score for each of the three cells shown in FIG. 22 based on cell separation to reflect the uptake of the ADC payload into neighboring cells.
FIG. 25 shows a formula by which a single-cell score is calculated.
FIG. 26 shows the correlation between actual outcome of 15 samples of a PDX Breast study and the manual H Score listed by response category.
FIG. 27 is a graph comparing a manual proportion score to a mean H Score.
FIG. 28 is a graph comparing a manual proportion score to the median QCS score for optical density of B7H4 membrane staining.
FIG. 29 is a graph comparing a manual intensity score to the mean H Score.
FIG. 30 is a graph comparing a manual intensity score to the median QCS score for optical density of B7H4 membrane staining.
FIG. 31 shows the mean H Score of samples of a PDX Breast study that fall within the three response categories PD, R and SD.
FIG. 32 shows the median QCS score of samples of a PDX Breast study that fall within the three response categories PD, R and SD.
FIG. 33 shows a box plot distribution of the manual H Score of the samples with respect to the response categories R, SD and PD.

FIG. 34 shows a box plot distribution of the QCS Score as defined by the median membrane optical density of the samples with respect to the response categories R, SD and PD.

FIG. 35 shows the relationship between tumor growth in percent and the QCS score (median/50% quantile of the membrane optical density of all tumor cells in the sample).

FIG. 36 shows the relationship between tumor growth in percent and the QCS score with an added correlation line.

FIG. 37 illustrates how a xenograft mouse model was used with image analysis and the quantitative continuous score to study the pharmacokinetics and pharmacodynamics of an anti-B7H4 ADC.

FIG. 38A is a graph showing the cell ratio of epithelial cells stained for ADC-IgG over all epithelial cells and demonstrates the concentration dependency and kinetics of ADC binding to target cells.

FIG. 38B is a graph showing the percent gH2AX positive epithelial cells asnd demonstrates increasing DNA damage over time after ADC treatment, and at different ADC concentrations.

FIG. 38C is a graph showing increasing DNA damage over time after ADC treatment based on the percent of cleaved caspase-3 (CC-3) positive tumor area.

FIG. 38D is a graph showing increasing tumor cell death over time after ADC treatment based on decreasing cell density of all epithelial cells.

FIG. 39A shows the median optical density of membrane staining of tumor tissue from 28 breast cancer patients that was cloned by implanting into mice.

FIG. 39B shows stained tissue of sample #21 from FIG. 39A, in which membrane staining by diagnostic antibody D11 was far less than the staining by diagnostic antibodies A57.1 and Cal63.

FIG. 39C shows stained tissue of sample #28 from FIG. 39A, in which membrane staining by diagnostic antibody A57.1 was significantly greater than the staining by diagnostic antibodies D11 and Cal63.

FIG. 40A is a digital image of sample #26 from FIG. 39A stained using the diagnostic antibody Cal63.

FIG. 40B is a digital image of sample #4 from FIG. 39A stained using the diagnostic antibody Cal63.

FIG. 41A shows the distribution of per-cell membrane staining for sample #26 before and after treatment with various concentrations of the ADC without payload.

FIG. 41B shows the distribution of per-cell membrane staining for sample #4 before and after treatment with the ADC E02-GL-SG3932.

FIG. 42A shows the per-cell distribution of bound IgG before and after ADC treatment for sample #26.

FIG. 42B shows the per-cell distribution of bound IgG before and after ADC treatment for sample #4.

FIG. 42C is a graph of the maximum membrane optical density of bound IgG at various dosage levels of the ADC E02-GL-SG3932.

FIG. 42D shows the percent of cleaved Caspase-3 (CC-3) positive tumor for sample #26 at various dosage levels of the ADC E02-GL-SG3932.

FIG. 43A is a graph showing the membrane optical density of IgG for clone samples #26 and #4 at various times after treatment with various dosages of the ADC.

FIG. 43B shows the median optical density of sample #26 at various times after treatment with 7 mg/kg of the ADC E02-GL-SG3932.

FIG. 44A shows, for sample #26, the density of stained epithelial cells at various times after treatment with the ADC as well as the increasing DNA damage over time after ADC treatment based on the percent of cleaved caspase-3 (CC-3) positive tumor cells.

FIG. 44B shows for sample #4, stained epithelial cell density at various times after ADC treatment as well as the increasing DNA damage over time after ADC treatment based on the percent of CC-3 positive tumor cells.

FIG. 45A shows the relationship of binary spatial proximity to membrane optical density at various times after ADC treatment for sample #26.

FIG. 45B shows the relationship of binary spatial proximity to membrane optical density at various times after ADC treatment for sample #4.

FIG. 46A shows the relationship of continuous spatial proximity to membrane optical density at various times after ADC treatment for sample #26.

FIG. 46B shows the relationship of continuous spatial proximity to membrane optical density at various times after ADC treatment for sample #4.

FIG. 47A shows the relationship of continuous spatial proximity to epithelial cell density at various times after ADC treatment for sample #26.

FIG. 47B shows the relationship of continuous spatial proximity to epithelial cell density at various times after ADC treatment for sample #4.

## DETAILED DESCRIPTION

The present invention provides a novel method for computing a score indicative of how a cancer patient will

respond to a therapy that uses a B7-H4 antibody-drug conjugate. Another aspect of the invention relates to a method for computing a score for a cancer patient indicative of a survival probability of a cancer patient treated with the ADC. Another aspect of the invention relates to a method for predicting a response of a cancer patient to the ADC. Another aspect of the invention relates to identifying a cancer patient who will exhibit a predetermined response to the ADC. Yet another aspect of the invention relates to a method of treating a cancer patient by administering a therapy involving the ADC if a treatment score exceeds a predetermined threshold.

**I. Definitions.**

To facilitate an understanding of the present invention, a number of terms and phrases are defined below. The term "cancer" is used to have the same meaning as that of the term "tumor".

In the present invention, "B7-H4" is synonymous with the V-set domain-containing T-cell activation inhibitor 1 that is encoded by the VTCN1 gene. B7-H4 is a transmembrane polypeptide of the B7 family of co-stimulatory proteins. B7-H4 is understood to be expressed on the surface of antigen-presenting cells for interactions with ligands of immune cells, such as T-lymphocytes, with CD28 being a potential ligand. The observation that B7-H4 is highly expressed on cells of various cancer types suggests that this molecule is a tumor-associated antigen. B7-H4 expression is not limited to a particular cancer type, but rather represents a target antigen for treating a broad spectrum of cancer types. The expression of the B7-H4 protein can be detected using a method well known to those skilled in the art, such as immunohistochemistry (IHC).

The RNA, DNA, and amino acid sequences of B7-H4 are known to those skilled in the art and can be found in many databases, for example, in the databases of the National Center for Biotechnology Information (NCBI) and UniProt. Examples of these sequences found at UniProt are at Q7Z7D3 (VTCN1_HUMAN) for human B7-H4; and Q7TSP5 (VTCN1_MOUSE) for mouse B7-H4. The nucleotide sequence encoding for human B7-H4 may be SEQ ID NO: 1 (FIG. 1) or more preferably SEQ ID NO: 2 (FIG. 2). The polypeptide sequence of human B7-H4 is preferably SEQ ID NO: 3 (FIG. 3).

In the present invention, "anti-B7H4 antibody" means an antibody that specifically binds to B7-H4, which preferably has an activity of binding to B7-H4 and thereby being internalized into B7-H4 expressing cells. In other words, an "anti-B7H4 antibody" means an antibody having an activity of binding to B7-H4 and thereafter moving into B7-H4 expressing cells.

The anti-B7H4 antibody comprises a heavy chain HCDR1 having the amino acid sequence of SEQ ID NO: 4 (FIG. 4), a heavy chain HCDR2 having the amino acid sequence of SEQ ID NO: 5 (FIG. 5), a heavy chain HCDR3 having the amino acid sequence of SEQ ID NO: 6 (FIG. 6), a light chain LCDR1 having the amino acid sequence of SEQ ID NO: 7 (FIG. 7), a light chain LCDR2 having the amino acid sequence of SEQ ID NO: 8 (FIG. 8), and a light chain LCDR3 having the amino acid sequence of SEQ ID NO: 9 (FIG. 9). The anti-B7H4 antibody comprises a variable heavy chain comprising the amino acid sequence of SEQ ID NO: 10 (FIG. 10), and a variable light chain comprising the amino acid sequence of SEQ ID NO: 11 (FIG. 11). The anti-B7H4 antibody comprises a heavy chain (e.g., comprising a VH and constant heavy chain) comprising the amino acid sequence of SEQ ID NO: 12 (FIG. 12) and a light chain (e.g., comprising a VL and constant light chain) comprising the amino acid sequence of SEQ ID NO: 13 (FIG. 13).

The terms "subject", "individual" and "patient" are used interchangeably herein to refer to a mammalian subject. In one embodiment the "subject" is a human, domestic animals, farm animals, sports animals, and zoo animals, e.g., humans, non-human primates, dogs, cats, guinea pigs, rabbits, rats, mice, horses, cattle, etc. In one embodiment, the subject is a cynomolgus monkey (Macaca fascicularis). In a preferable embodiment, the subject is a human. In methods of the invention, the patient may not have been previously diagnosed as having cancer. Alternatively, the patient may have been previously diagnosed as having cancer. The patient may also be one who exhibits disease risk factors, or one who is asymptomatic for cancer. The patient may also be one who is suffering from or is at risk of developing cancer. Thus, in one embodiment, a method of the invention may be used to confirm the presence of cancer in a patient. For example, the patient may previously have been diagnosed with cancer by alternative means. In one embodiment, the patient has been previously administered a cancer therapy.

In the present invention, the term "QCS Positive" refers to cancer that is likely to show a response to an anti-B7H4 ADC therapy. The term "QCS Negative" refers to cancer that is unlikely to show a response to an anti-B7H4 ADC therapy. The acronym QCS stands for Quantitative Continuous Score. The result of the novel predictive method of the present invention is generally referred to as a Quantitative Continuous Score and may be a response score or an indication of predicted survival time.

**II. The anti-B7H4 antibody-drug conjugate.**

The anti-B7H4 antibody-drug conjugate used in the present invention comprises an anti-B7H4 antibody or antigen binding fragment conjugated to one or more cytotoxins selected from a topoisomerase I inhibitor, tubulysin derivative, a pyrrolobenzodiazepine, or a combination thereof. For example, the antibody or antigen binding fragment thereof is conjugated to one or more cytotoxins selected from the group consisting of topoisomerase I inhibitor SG3932,

SG4010, SG4057 or SG4052 (the structures of which are provided below); tubulysin AZ1508, pyrrolobenzodiapezine SG3315, pyrrolobenzodiapezine SG3249, or a combination thereof.

It is preferred that the antibody or antigen binding fragment thereof is conjugated to a topoisomerase I inhibitor. Topoisomerase inhibitors are chemical compounds that block the action of topoisomerase (topoisomerase I and II), which is a type of enzyme that controls the changes in DNA structure by catalyzing the breaking and rejoining of the phosphodiester backbone of DNA strands during the normal cell cycle.

A general example of a suitable topoisomerase I inhibitor is represented by the following compound:

[Formula 1]

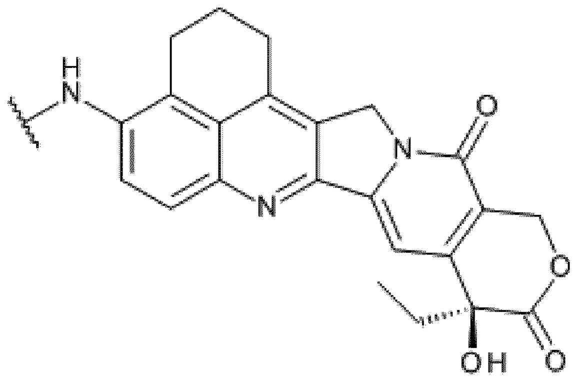

**A***

The compound shown above is denoted as A*, and may be referred to as a "Drug Unit" herein. The compound A* is preferably provided with a linker for connecting (preferably conjugating) to an antibody or antigen binding fragment described herein (which may be referred to as a "Ligand Unit"). Suitably, the linker is attached (e.g., conjugated) in a cleavable manner to an amino residue, for example, an amino acid of an antibody or antigen binding fragment described herein.

More particularly, an example of a suitable topoisomerase I inhibitor is represented by the following compound denoted as "I":

[Formula 2]

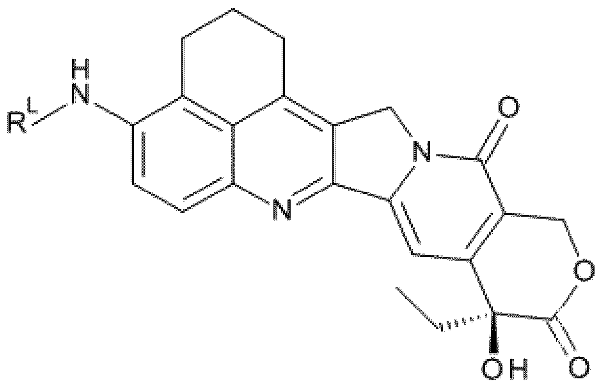

**I**

The compound I includes salts and solvates thereof, wherein $R^L$ is a linker for connection to an antibody or antigen binding fragment thereof described herein (e.g., the Ligand Unit), wherein said linker "Ia" is preferably selected from:

[Formula 3]

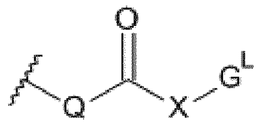

Ia

wherein Q is:

[Formula 4]

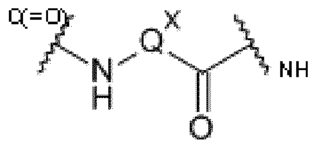

where $Q^X$ is such that Q is an amino-acid residue, a dipeptide residue, a tripeptide residue or a tetrapeptide residue, and X in Formula 3 is:

[Formula 5]

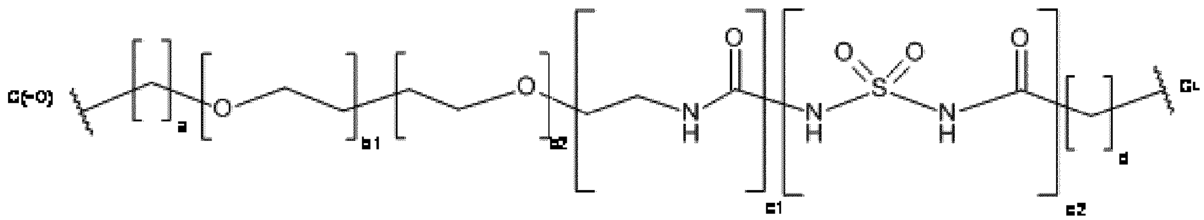

where a = 0 to 5, b1 = 0 to 16, b2 = 0 to 16, c1 = 0 or 1, c2 = 0 or 1, d = 0 to 5, wherein at least b1 or b2 = 0 (i.e., only one of b1 and b2 may not be 0) and at least c1 or c2 = 0 (i.e., only one of c1 and c2 may not be 0). $G^L$ in Formula 3 is a linker for connecting to an antibody or antigen binding fragment thereof as described herein (e.g., the Ligand Unit). Alternatively, $G^L$ in Formula 3 is the compound denoted as "Ib":

[Formula 6]

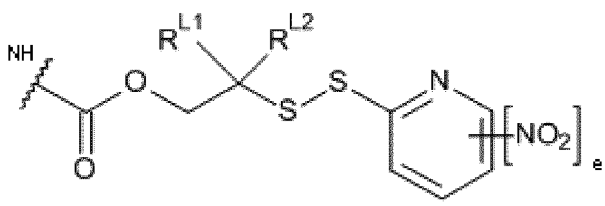

Ib

where $R^{L1}$ and $R^{L2}$ are independently selected from H and methyl, or together with the carbon atom to which they are bound form a cyclopropylene or cyclobutylene group. In Formula 6, e is 0 or 1.

It will be understood by the person skilled in the art that more than one of said agent(s) (e.g., topoisomerase I inhibitor) may be conjugated to the antibody or antigen binding fragment thereof. For example, a conjugate (e.g., antibody-drug conjugate) of the invention may be of the general formula $L-(D^L)_p$ or a pharmaceutically acceptable salt or solvate thereof, wherein L is an antibody or antigen binding fragment thereof described herein (e.g., the Ligand Unit), p is an integer from 1 to 20, and $D^L$ is a topoisomerase I inhibitor having a linker (e.g., Drug Linker unit) that is represented by the following formula:

[Formula 7]

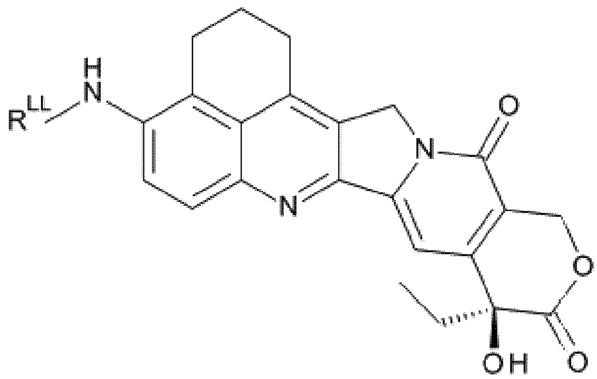

In Formula 7, $R^{LL}$ is a linker connected to an antibody or antigen binding fragment thereof described herein (e.g., the Ligand unit), wherein the linker is preferably selected from "Ia'" or "Ib'":

[Formula 8]

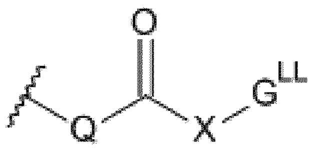

Ia'

where Q and X are as defined above, and $G^{LL}$ is a linker connected to an antibody or antigen binding fragment thereof as described herein (e.g., the Ligand Unit). Ib' is represented by the formula:

[Formula 9]

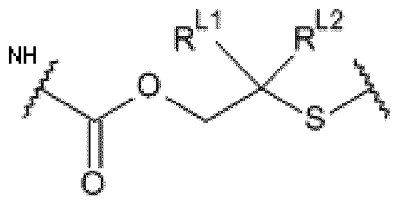

Ib'

where $R^{L1}$ and $R^{L2}$ are as defined above. The "p" in the formula L-$(D^L)_p$ for the antibody-drug conjugate represents the drug loading and is the number of topoisomerase I inhibitor(s) (e.g., Drug units) per antibody or antigen binding fragment thereof (e.g., Ligand unit). The drug loading ranges from 1 to 20 Drug units (D) per Ligand unit. Each Drug Linker unit is conjugated to the anti-B7H4 antibody. For compositions, "p" represents the average drug loading of the conjugates in the composition, and "p" ranges from 1 to 20. The meaning of "p" is the same as that of what is called the average number of conjugated drug molecules (the drug-to-antibody ratio DAR), and indicates the average number of units of the drug-linker conjugated per antibody molecule.

Accordingly, antibody-drug conjugates described herein embrace a conjugate comprising an antibody or antigen binding fragment thereof described herein (e.g., the Ligand Unit) covalently linked to at least one topoisomerase I inhibitor (e.g. Drug unit, such as A* illustrated above). Said inhibitor is preferably linked to the antibody or antigen binding fragment thereof by a linker (e.g., Linker unit), such as a linker described above as $R^L$ and/or $R^{LL}$. In other words, the antibody-drug conjugates described herein embrace an antibody or antigen binding fragment thereof described herein (e.g., the Ligand Unit) with one or more topoisomerase I inhibitors attached, preferably via a linker (e.g., Drug Linker units). The antibody or antigen binding fragment thereof (representing a Ligand unit), described more fully above, is a targeting agent that binds to a target moiety. More particularly, this Ligand unit can, for example, specifically bind to a B7-H4 protein on a target cell, to which the Drug unit is thus delivered. Accordingly, the present invention also provides methods for the treatment of, for example, various cancers and other disorders with an ADC (e.g., cancers/ disorders that are associated with the presence of cells, preferably cancerous cells, which express B7-H4).

After undergoing cellular internalization into cancer cells, the anti-B7H4 antibody-drug conjugate used in the present invention is cleaved at the Linker unit and releases the Drug unit into the cancer cell. The anti-B7H4 antibody-drug conjugate used in the present invention also has a bystander effect in which the anti-B7H4 antibody-drug conjugate is internalized into cancer cells that express the target protein B7-H4, and the Drug unit then also exerts an antitumor effect on neighboring cancer cells that do not express the target protein B7-H4.

**III. Further Preferences of the Preferred Embodiments.**

The following preferences may apply to all aspects of the invention as described above, or may relate to a single aspect. The preferences may be combined together in any combination. Various definitions that pertain to certain terms in this section are provided under the heading "Further Chemical Definitions" set forth below.

Certain features of the topoisomerase I inhibitors described above are particularly preferred and are defined in more detail below. By way of example, a preferred embodiment of feature $Q^X$ of the linker of Formula 4 are outlined. In one embodiment, Q is an amino acid residue. The amino acid may be a natural amino acid or a non-natural amino acid. For example, Q may be selected from: Phe, Lys, Val, Ala, Cit, Leu, Ile, Arg, and Trp, where Cit is citrulline. In one embodiment, Q comprises a dipeptide residue. The amino acids in the dipeptide may be any combination of natural amino acids and non-natural amino acids. In some embodiments, the dipeptide comprises natural amino acids. Where the linker is a cathepsin labile linker, the dipeptide is the site of action for cathepsin-mediated cleavage. The dipeptide then is a recognition site for cathepsin.

In one embodiment, Q is selected from:

$^{NH}$-Phe-Lys-$^{C=O}$,

$^{NH}$-Val-Ala-$^{C=O}$,

$^{NH}$-Val-Lys-$^{C=O}$,

$^{NH}$-Ala-Lys-$^{C=O}$,

$^{NH}$-Val-Cit-$^{C=O}$,

$^{NH}$-Phe-Cit-$^{C=O}$,

$^{NH}$-Leu-Cit-$^{C=O}$,

$^{NH}$-Ile-Cit-$^{C=O}$,

$^{NH}$-Phe-Arg-$^{C=O}$,

$^{NH}$-Trp-Cit-$^{C=O}$,

and

$^{NH}$-Gly-Val-$^{C=O}$;

where Cit is citrulline. Preferably, Q is selected from:

$^{NH}$-Phe-Lys-$^{C=O}$,

$^{NH}$-Val-Ala-$^{C=O}$,

$^{NH}$-Val-Lys-$^{C=O}$,

$^{NH}$-Ala-Lys-$^{C=O}$,

and

$^{NH}$-Val-Cit-$^{C=O}$.

More preferably, Q is selected from $^{NH}$-Phe-Lys-$^{C=O}$, $^{NH}$-Val-Cit-$^{C=O}$ or $^{NH}$-Val-Ala-$^{C=O}$. Other suitable dipeptide combinations include:

$^{NH}$-Gly-Gly-$^{C=O}$,

$^{NH}$-Gly-Val-$^{C=O}$,

$^{NH}$-Pro-Pro-$^{C=O}$,

and

$^{NH}$-Val-Glu-$^{C=O}$.

Other dipeptide combinations may be used, including those described by Dubowchik et al., Bioconjugate Chemistry, 2002, 13,855-869,

In some embodiments, Q is a tripeptide residue. The amino acids in the tripeptide may be any combination of natural amino acids and non-natural amino acids. In some embodiments, the tripeptide comprises natural amino acids. Where the linker is a cathepsin labile linker, the tripeptide is the site of action for cathepsin-mediated cleavage. The tripeptide then is a recognition site for cathepsin. Tripeptide linkers of particular interest are:

$^{NH}$-Glu-Val-Ala-$^{C=O}$,

$^{NH}$-Glu-Val-Cit-$^{C=O}$,

$^{NH}$-$\alpha$Glu-Val-Ala-$^{C=O}$ ,

and

$^{NH}$-$\alpha$Glu-Val-Cit-$^{C=O}$.

In some embodiments, Q is a tetrapeptide residue. The amino acids in the tetrapeptide may be any combination of natural amino acids and non-natural amino acids. In some embodiments, the tetrapeptide comprises natural amino acids. Where the linker is a cathepsin labile linker, the tetrapeptide is the site of action for cathepsin-mediated cleavage. The tetrapeptide then is a recognition site for cathepsin. Tetrapeptide linkers of particular interest are:

$^{NH}$-Gly-Gly-Phe-Gly $^{C=O}$,

and

$^{NH}$-Gly-Phe-Gly-Gly $^{C=O}$.

In some embodiments, the tetrapeptide is:

$^{NH}$-Gly-Gly-Phe-Gly $^{C=O}$.

In the above representations of peptide residues, $^{NH}$- represents the N-terminus, and -$^{C=O}$ represents the C-terminus of the residue. The C-terminus binds to the NH of the compound A* shown in Formula 1. Glu represents the residue of glutamic acid, i.e.:

[Formula 10]

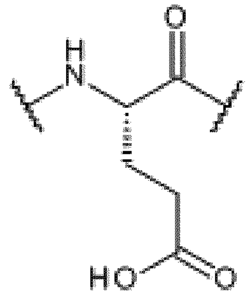

αGlu represents the residue of glutamic acid when bound via the α-chain, i.e.:

[Formula 11]

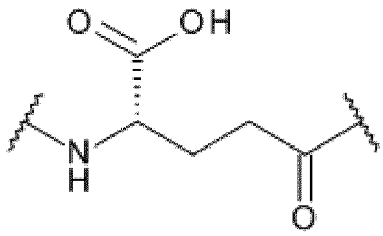

In one embodiment, the amino acid side chain is chemically protected, where appropriate. The side chain protecting group may be a group as discussed above. Protected amino acid sequences are cleavable by enzymes. For example, a dipeptide sequence comprising a Boc side chain-protected Lys residue is cleavable by cathepsin.

Protecting groups for the side chains of amino acids are well known in the art and are described in the Novabiochem Catalog as well as above.

$G^L$ of the linker of Formula 3 may be selected from:

| | | | |
|---|---|---|---|
| ($G^{L1-1}$) | 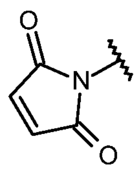 | ($G^{L6}$) | 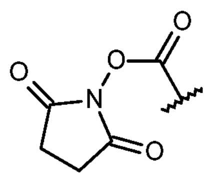 |
| ($G^{L1-2}$) | 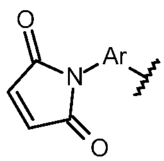 | ($G^{L7}$ ) | 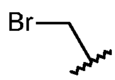 |
| ($G^{L2}$) | 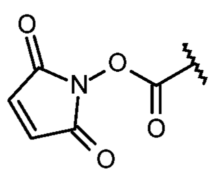 | ($G^{L8}$) | 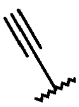 |

(continued)

| | | | |
|---|---|---|---|
| ($G^{L3-1}$) | 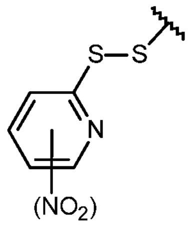 where the $NO_2$ group is optional | ($G^{L9}$) | 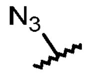 |
| ($G^{L3-2}$) | 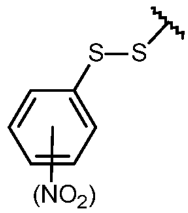 where the $NO_2$ group is optional | ($G^{L10}$) | 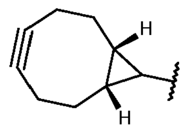 |
| ($G^{L3-3}$) | 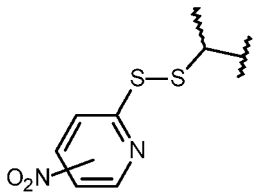 where the $NO_2$ group is optional | ($G^{L11}$) | 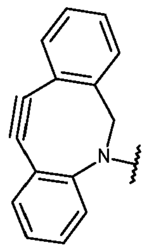 |
| ($G^{L3-4}$) | 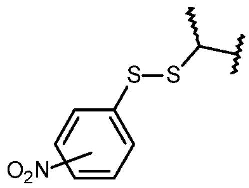 where the $NO_2$ group is optional | ($G^{L12}$) | 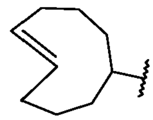 |
| ($G^{L4}$) | 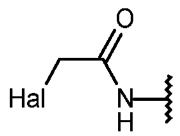 Where Hal = I, Br, Cl | ($G^{L13}$) | 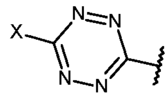 |
| ($G^{L5}$) | 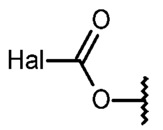 | ($G^{L14}$) | 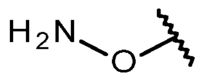 |

where Ar represents a $C_{5-6}$ arylene group, e.g., phenylene, and X represents $C_{1-4}$ alkyl.

In some embodiments, $G^L$ is selected from $G^{L1-1}$ and $G^{L1-2}$. In some of these embodiments, $G^L$ is $G^{L1-1}$. $G^{LL}$ may be selected from:

| | | | |
|---|---|---|---|
| ($G^{LL1-1}$) | 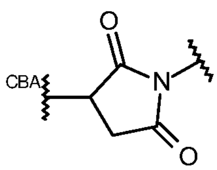 | ($G^{LL8-1}$) | 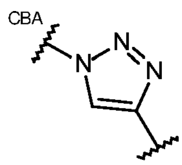 |

(continued)

| | | | |
|---|---|---|---|
| ($G^{LL1-2}$) | 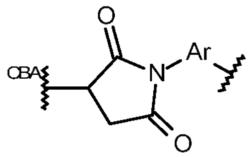 | ($G^{LL8-2}$) | 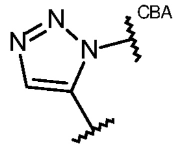 |
| ($G^{LL2}$) | 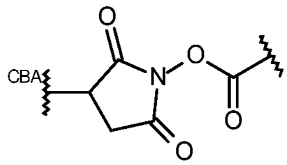 | ($G^{LL9-1}$) | 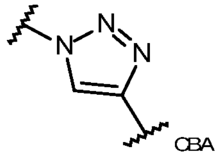 |
| ($G^{LL3-1}$) | 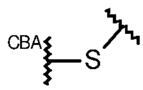 | ($G^{LL9-2}$) | 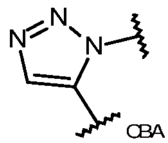 |
| ($G^{LL3-2}$) | 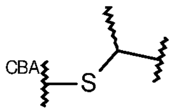 | ($G^{LL10}$) | 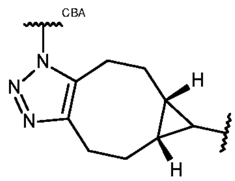 |
| ($G^{LL-4}$) | 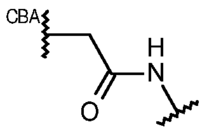 | ($G^{LL11}$) | 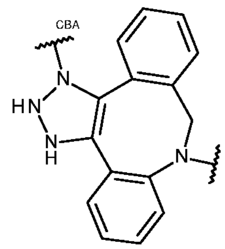 |
| ($G^{LL5}$) | 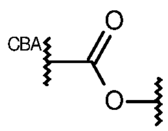 | ($G^{LL12}$) | 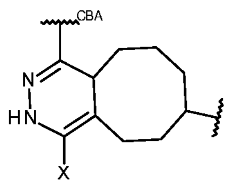 |
| ($G^{LL6}$) | 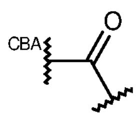 | ($G^{LL13}$) | 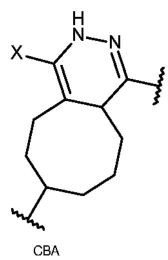 |
| ($G^{LL7}$) | 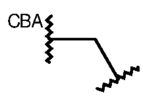 | ($G^{LL14}$) | 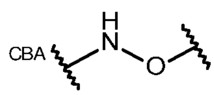 |

where Ar represents a $C_{5-6}$ arylene group, e.g. phenylene and X represents $C_{1-4}$ alkyl. In some embodiments, $G^{LL}$ is selected from $G^{LL1-1}$ and $G^{LL1-2}$. In some of these embodiments, $G^{LL}$ is $G^{LL1-1}$.

X is preferably:

[Formula 12]

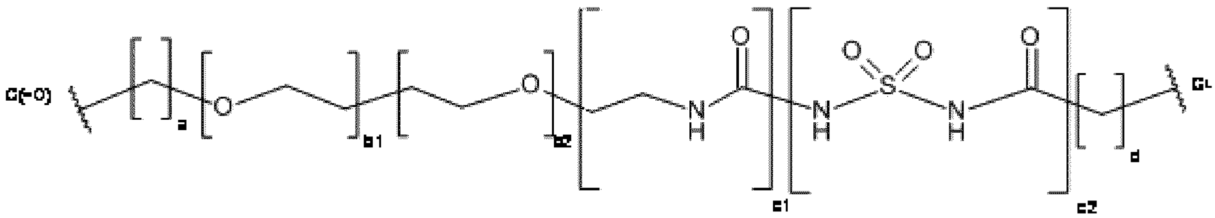

where a = 0 to 5, b1 = 0 to 16, b2 = 0 to 16, c = 0 or 1, d = 0 to 5, wherein at least b1 or b2 = 0 and at least c1 or c2 = 0.
"a" may be 0, 1, 2, 3, 4 or 5. In some embodiments, a is 0 to 3. In some of these embodiments, a is 0 or 1. In further embodiments, a is 0.
"b1" may be 0, 1, 2, 3, 4, 5, 6, 7, 8, 9, 10, 11, 12, 13, 14, 15 or 16. In some embodiments, "b1" is 0 to 12. In some of these embodiments, "b1" is 0 to 8, and may be 0, 2, 3, 4, 5 or 8.
"b2" may be 0, 1, 2, 3, 4, 5, 6, 7, 8, 9, 10, 11, 12, 13, 14, 15 or 16. In some embodiments, "b2" is 0 to 12. In some of these embodiments, "b2" is 0 to 8, and may be 0, 2, 3, 4, 5 or 8. Preferably, only one of b1 and b2 may not be 0.
"c1" may be 0 or 1. "c2" may be 0 or 1. Preferably, only one of "c1" or "c2" may not be 0.
"d" may be 0, 1, 2, 3, 4 or 5. In some embodiments, "d" is 0 to 3. In some of these embodiments, "d" is 1 or 2. In further embodiments, "d" is 2. In further embodiments, "d" is 5.

In some embodiments of X, a is 0, b1 is 0, c1 is 1, c2 is 0 and d is 2, and b2 may be from 0 to 8. In some of these embodiments, b2 is 0, 2, 3, 4, 5 or 8. In some embodiments of X, a is 1, b2 is 0, c1 is 0, c2 is 0 and d is 0, and b1 may be from 0 to 8. In some of these embodiments, b1 is 0, 2, 3, 4, 5 or 8. In some embodiments of X, a is 0, b1 is 0, c1 is 0, c2 is 0 and d is 1, and b2 may be from 0 to 8. In some of these embodiments, b2 is 0, 2, 3, 4, 5 or 8. In some embodiments of X, b1 is 0, b2 is 0, c1 is 0, c2 is 0 and one of a and d is 0. The other of a and d is from 1 to 5. In some of these embodiments, the other of a and d is 1. In other of these embodiments, the other of a and d is 5. I n some embodiments of X, a is 1, b2 is 0, c1 is 0, c2 is 1, d is 2, and b1 may be from 0 to 8. In some of these embodiments, b2 is 0, 2, 3, 4, 5 or 8.

In some embodiments, $R^L$ is Ib of Formula 6. In some embodiments, $R^{LL}$ is the group Ib' of Formula 9. $R^{L1}$ and $R^{L2}$ may be independently selected from H and methyl, or together with the carbon atom to which they are bound form a cyclopropylene or cyclobutylene group.

In some embodiments, both $R^{L1}$ and $R^{L2}$ are H. In some embodiments, $R^{L1}$ is H and $R^{L2}$ is methyl. In some embodiments, both $R^{L1}$ and $R^{L2}$ are methyl.

In some embodiments, $R^{L1}$ and $R^{L2}$ together with the carbon atom to which they are bound form a cyclopropylene group. In some embodiments, $R^{L1}$ and $R^{L2}$ together with the carbon atom to which they are bound form a cyclobutylene group.

In the group Ib of Formula 6, in some embodiments, e is 0. In other embodiments, e is 1 and the nitro group may be in any available position of the ring. In some of these embodiments, it is in the ortho position. In others of these embodiments, it is in the para position.

In some embodiments where compounds described herein are provided in a single enantiomer or in an enantiomerically enriched form, the enantiomerically enriched form has an enantiomeric ratio greater than 60:40, 70:30; 80:20 or 90:10. In further embodiments, the enantiomeric ratio is greater than 95:5, 97:3 or 99:1.

In some embodiments, $R^L$ of Formula 2 is selected from:

| | |
|---|---|
| (i) | 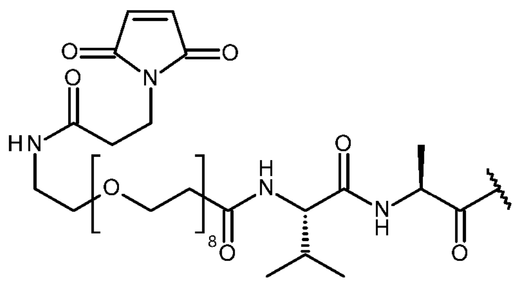 |

(continued)

| | |
|---|---|
| (ii) | 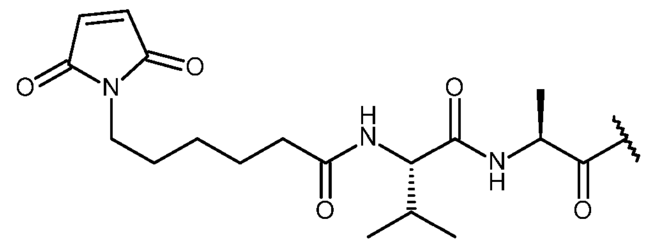 |
| (iii) | 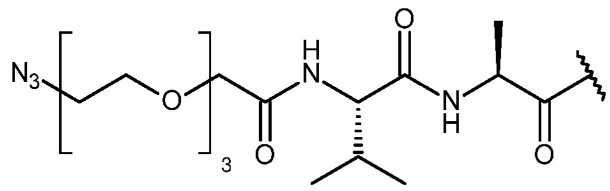 |
| (iv) | 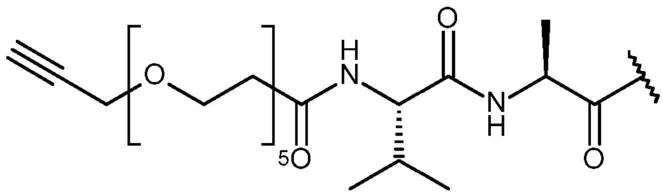 |
| (v) | 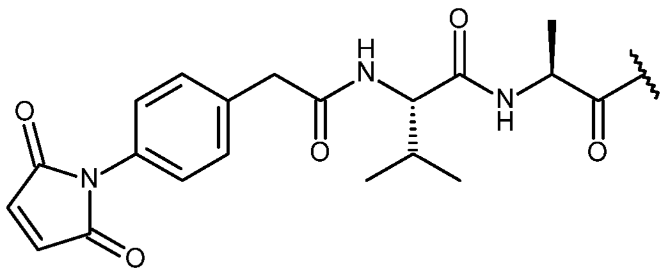 |
| (vi) | 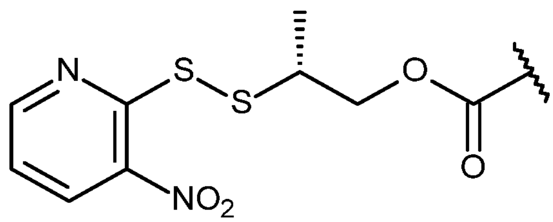 |
| (vii) | 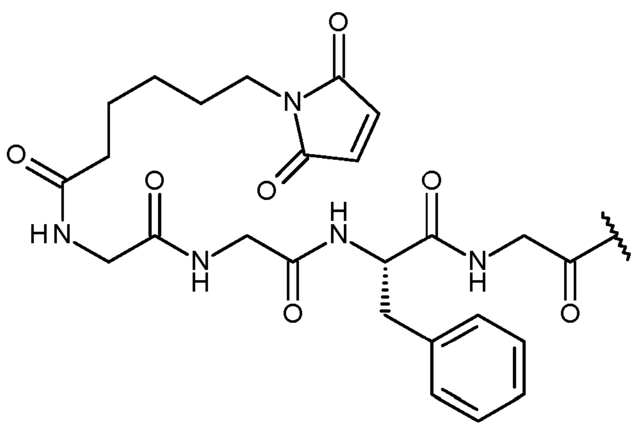 |

(continued)

| | |
|---|---|
| (viii) | 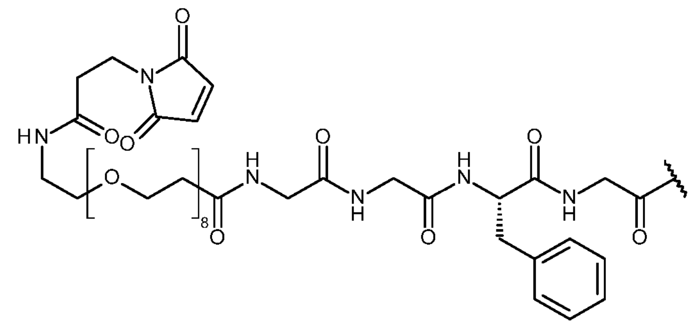 |
| (ix) | 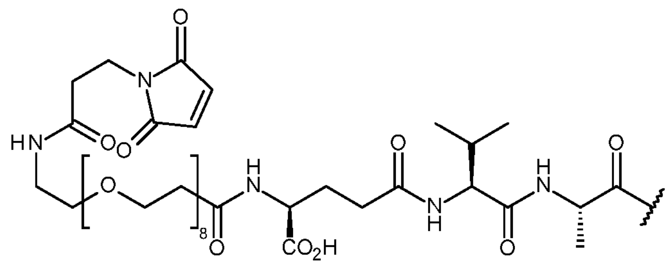 |

In some embodiments, $R^{LL}$ is a group derived from the $R^L$ groups above.

Having outlined said preferences above, certain preferred topoisomerase I linker formulas (e.g., of the Drug Linker unit) are now described. In some embodiments, the compound I of Formula 2 is the compound $I^P$:

[Formula 13]

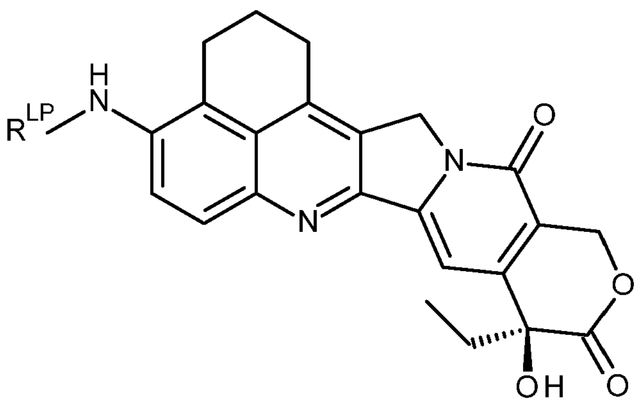

$I^P$

and salts and solvates thereof. $R^{LP}$ is a linker for connection to an antibody or antigen binding fragment thereof described herein, wherein said linker is selected from the group Ia or Ib. The group Ia is represented by the formula:

[Formula 14]

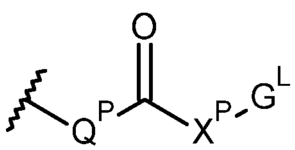

$Ia^P$

wherein $Q^P$ is:

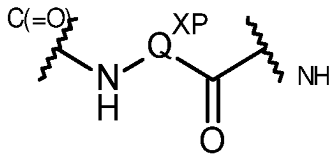

where $Q^{XP}$ is such that $Q^P$ is an amino-acid residue, a dipeptide residue or a tripeptide residue. $X^P$ in Formula 14 is:

[Formula 15]

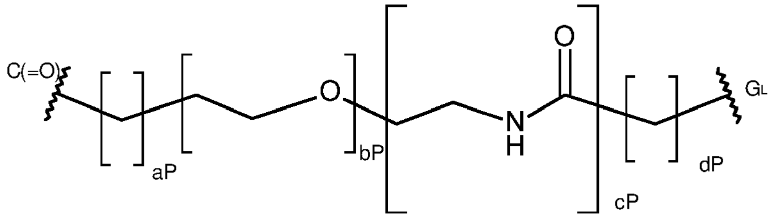

where aP = 0 to 5, bP = 0 to 16, cP = 0 or 1, and dP = 0 to 5. $G^L$ in Formula 14 is a linker for connecting to an antibody or antigen binding fragment thereof (e.g., Ligand unit). aP may be 0, 1, 2, 3, 4 or 5. In some embodiments, aP is 0 to 3. In some of these embodiments, aP is 0 or 1. In further embodiments, aP is 0. bP may be 0, 1, 2, 3, 4, 5, 6, 7, 8, 9, 10, 11, 12, 13, 14, 15 or 16. In some embodiments, b is 0 to 12. In some of these embodiments, bP is 0 to 8, and may be 0, 2, 4 or 8. cP may be 0 or 1. dP may be 0, 1, 2, 3, 4 or 5. In some embodiments, dP is 0 to 3. In some of these embodiments, dP is 1 or 2. In further embodiments, dP is 2.

The group Ib is represented by the formula:

[Formula 16]

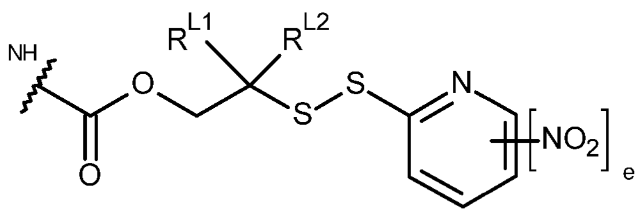

Ib

$R^{L1}$ and $R^{L2}$ are independently selected from H and methyl, or together with the carbon atom to which they are bound form a cyclopropylene or cyclobutylene group. "e" in Formula 16 is 0 or 1.

In some embodiments of $X^P$ of Formula 14, aP is 0, cP is 1 and dP is 2, and bP may be from 0 to 8. In some of these embodiments, bP is 0, 4 or 8.

The preferences for $Q^X$ of Formula 4 for compound I may apply to $Q^{XP}$ of Formula 14. The preferences for $G^L$, $R^{L1}$, $R^{L2}$ and e above for compound I of Formula 2 may apply to compound $I^P$ of Formula 13.

In some embodiments, the conjugate of the antibody-drug conjugate L-$(D^L)_p$ is the compound L-$(D^{LP})_p$ or a pharmaceutically acceptable salt or solvate thereof, wherein L is an antibody or antigen binding fragment thereof (e.g., Ligand unit), $D^{LP}$ is a topoisomerase I inhibitor (e.g., Drug Linker unit) that is the compound $III^P$:

[Formula 17]

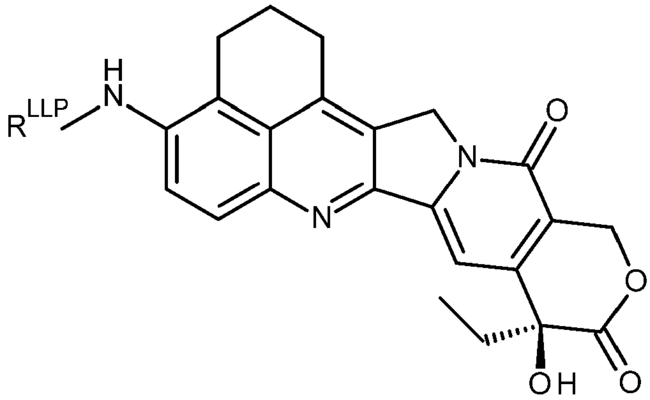

$III^P$

$R^{LLP}$ is a linker connected to the antibody or antigen binding fragment thereof (e.g., Ligand unit), wherein said linker is selected from the group Ia' or Ib'. The group Ia' is represented by the formula:

[Formula 18]

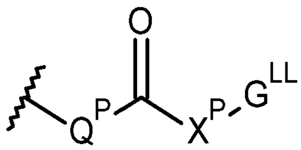

$Ia^{P'}$

where $Q^P$ and $X^P$ are as defined above, and $G^{LL}$ is a linker connected to an antibody or antigen binding fragment thereof (e.g., Ligand Unit). The group Ib' is represented by the formula:

[Formula 19]

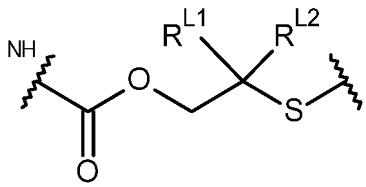

Ib'

where $R^{L1}$ and $R^{L2}$ are as defined above; and p is an integer of from 1 to 20.

In some embodiments, the compound I of Formula 2 is the compound $I^{P2}$.

[Formula 20]

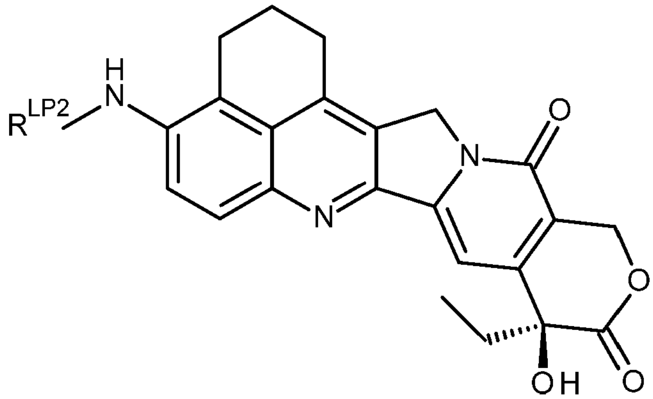

$I^{P2}$

and salts and solvates thereof. $R^{LP2}$ is a linker for connection to an antibody or antigen binding fragment thereof, wherein said linker is selected from the group Ia or Ib. The group Ia is represented by the formula:

[Formula 21]

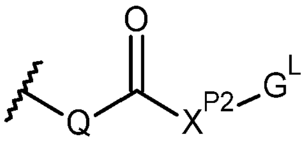

$Ia^{P2}$

Q is:

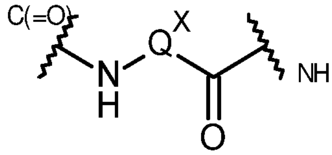

where $Q^X$ is such that Q is an amino-acid residue, a dipeptide residue, a tripeptide residue or a tetrapeptide residue. $X^{P2}$ is:

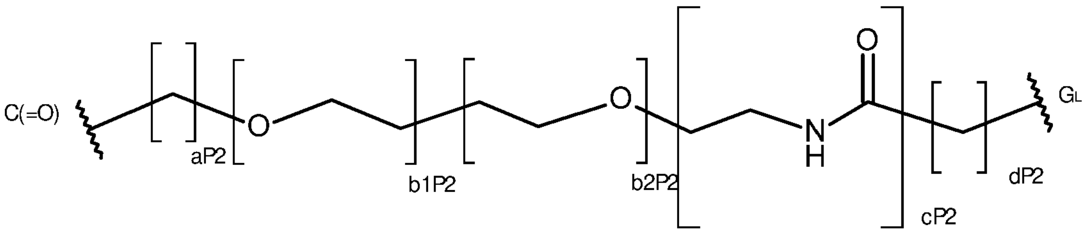

where aP2 = 0 to 5, b1P2 = 0 to 16, b2P2 = 0 to 16, cP2 = 0 or 1, dP2 = 0 to 5, wherein at least b1P2 or b2P2 = 0 (i.e., only one of b1 and b2 may not be 0). $G^L$ of Formula 21 is a linker for connecting to an antibody or antigen binding fragment thereof (e.g., Ligand Unit).

aP2 may be 0, 1, 2, 3, 4 or 5. In some embodiments, aP2 is 0 to 3. In some of these embodiments, aP2 is 0 or 1. In further embodiments, aP2 is 0. b1P2 may be 0, 1, 2, 3, 4, 5, 6, 7, 8, 9, 10, 11, 12, 13, 14, 15 or 16. In some embodiments, b1P2 is 0 to 12. In some of these embodiments, b1P2 is 0 to 8, and may be 0, 2, 3, 4, 5 or 8. b2P2 may be 0, 1, 2, 3, 4, 5, 6, 7, 8, 9, 10, 11, 12, 13, 14, 15 or 16. In some embodiments, b2P2 is 0 to 12. In some of these embodiments, b2P2 is 0 to 8, and may be 0, 2, 3, 4, 5 or 8. Preferably, only one of b1P2 and b2P2 may not be 0. cP2 may be 0 or 1. dP2 may be 0, 1, 2, 3, 4 or 5. In some embodiments, dP2 is 0 to 3. In some of these embodiments, dP2 is 1 or 2. In further embodiments, dP2 is 2. In further embodiments, dP2 is 5.

The group Ib is represented by the formula:

[Formula 22]

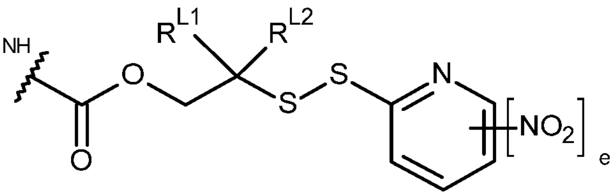

Ib

where $R^{L1}$ and $R^{L2}$ are independently selected from H and methyl, or together with the carbon atom to which they are bound form a cyclopropylene or cyclobutylene group; and e is 0 or 1.

In some embodiments of $X^{P2}$ of Formula 21, aP2 is 0, b1P2 is 0, cP2 is 1 and dP2 is 2, and b2P2 may be from 0 to 8. In some of these embodiments, b2P2 is 0, 2, 3, 4, 5 or 8. In some embodiments of $X^{P2}$, aP2 is 1, b2P2 is 0, cP2 is 0 and dP2 is 0, and b1P2 may be from 0 to 8. In some of these embodiments, b1P2 is 0, 2, 3, 4, 5 or 8. In some embodiments of $X^{P2}$, aP2 is 0, b1P2 is 0, cP2 is 0 and dP2 is 1, and b2P2 may be from 0 to 8. In some of these embodiments, b2P2 is 0, 2, 3, 4, 5 or 8. In some embodiments of $X^{P2}$, b1P2 is 0, b2P2 is 0, cP2 is 0 and one of aP2 and dP2 is 0. The other of aP2 and d is from 1 to 5. In some of these embodiments, the other of aP2 and d is 1. In other of these embodiments, the other of aP2 and dP2 is 5.

The preferences for $Q^X$ above for compound **I** of Formula 2 may apply to $Q^X$ in group $Ia^{P2}$ of Formula 21. The preferences for $G^L$, $R^{L1}$, $R^{L2}$ and e above for compound **I** of Formula 2 may apply to group $Ia^{P2}$ of Formula 21.

In some embodiments, the conjugate of the conjugate L-$(D^L)_p$ is the compound L-$(D^{LP2})_p$ or a pharmaceutically acceptable salt or solvate thereof, wherein L is an antibody or antigen binding fragment thereof (e.g., Ligand unit), and $D^{LP2}$ is a topoisomerase I inhibitor (e.g., Drug Linker unit) that is the compound $III^{P2}$:

[Formula 23]

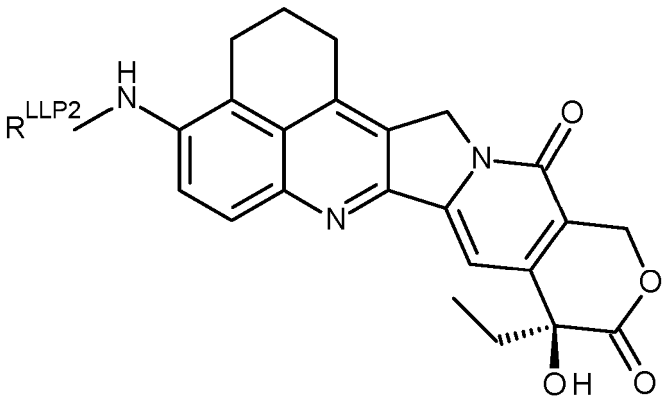

**$III^{P2}$**

$R^{LLP2}$ is a linker connected to the antibody or antigen binding fragment thereof (e.g. Ligand unit), wherein said linker is selected from the group Ia' or Ib'. The group Ia' is represented by the formula:

[Formula 24]

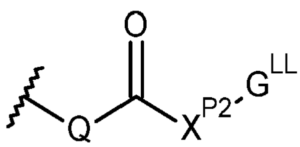

$Ia^{P2'}$

where Q and $X^{P2}$ are as defined above, and $G^{LL}$ is a linker connected to the antibody or antigen binding fragment thereof.

The group Ib' is represented by the formula:

[Formula 25]

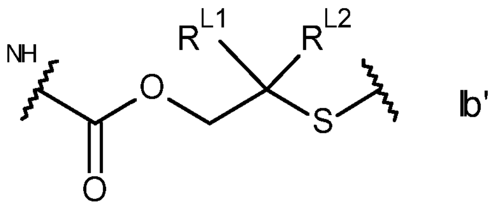

where $R^{L1}$ and $R^{L2}$ are as defined above; and p is an integer of from 1 to 20.

Particularly suitable topoisomerase I inhibitors include those having the following formulas:

[Formula 26]

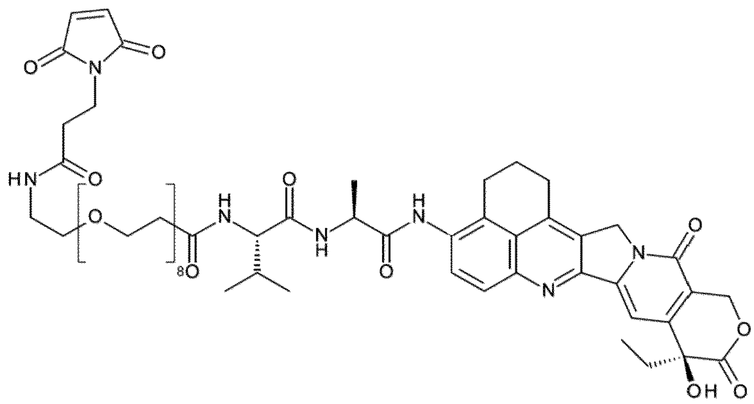

(SG3932);

[Formula 27]

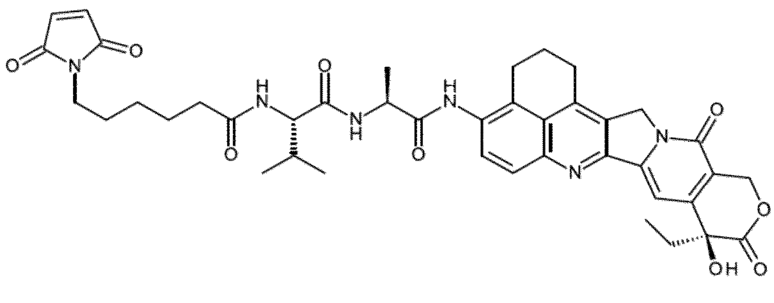

(SG4010);

[Formula 28]

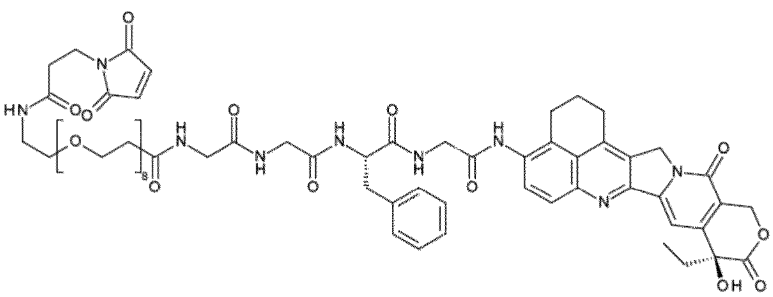

(SG4057);

[Formula 29]

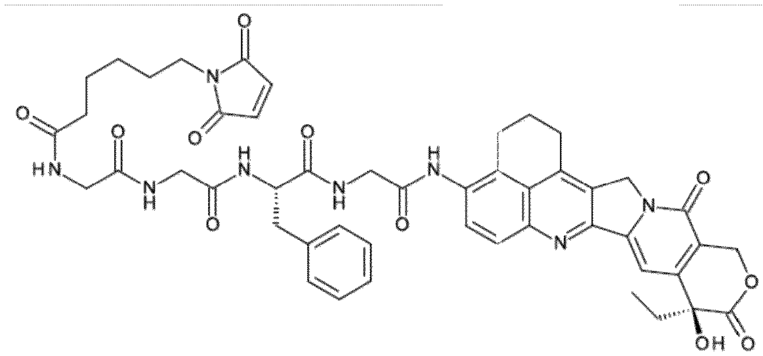

(SG4052);

and/or

[Formula 30]

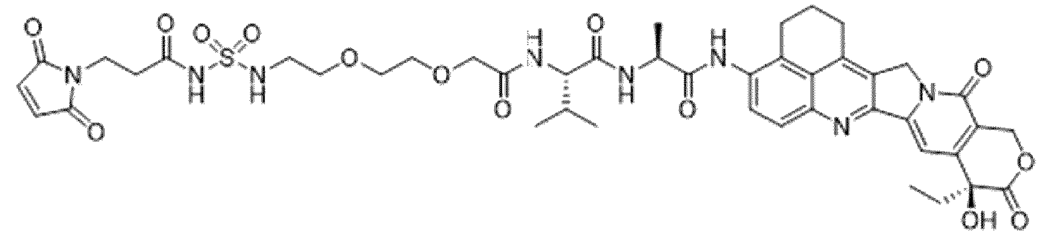

.

The inhibitor SG3932 is particularly preferred. Thus, in a preferable embodiment, an antibody or antigen binding fragment thereof is conjugated to a topoisomerase I inhibitor having the following formula of SG3932:

[Formula 26]

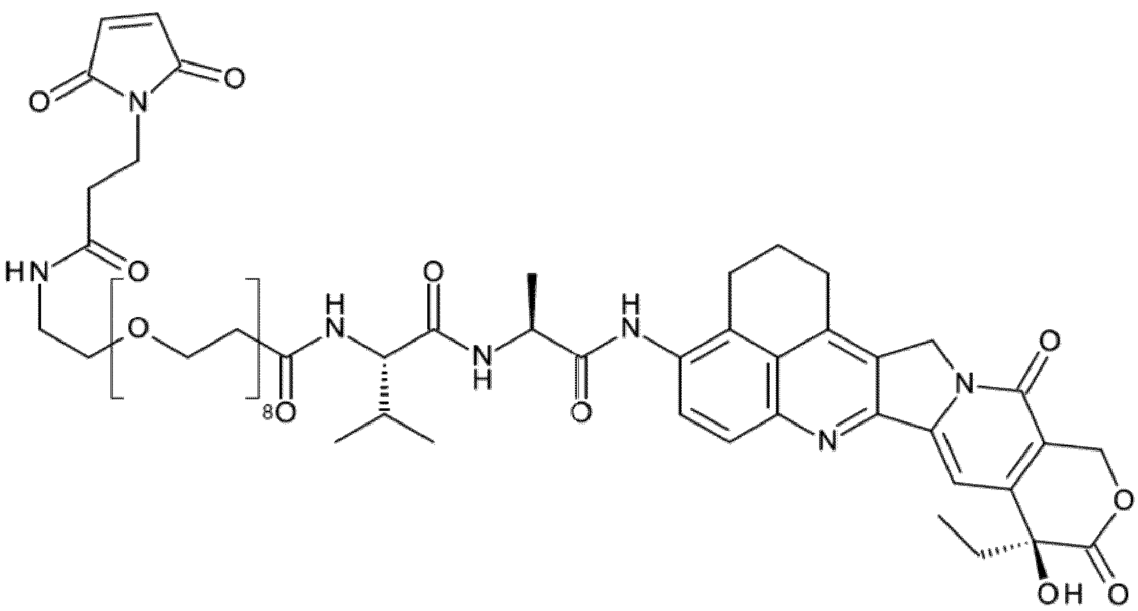

For the avoidance of doubt, the numeral "8" in Formula 26 specifies that the structure within the boxed parentheses is repeated eight times. Thus, another representation of SG3932 is:

[Formula 31]

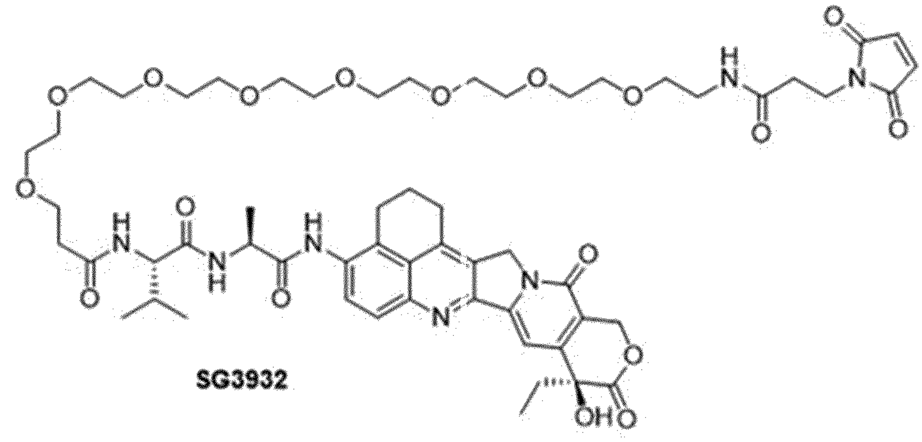

SG3932

Another representation of SG4010 is:

[Formula 32]

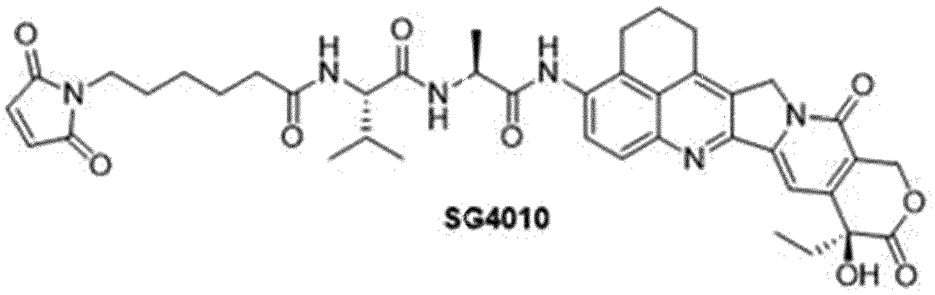

SG4010

Another representation of SG4057 is:

[Formula 33]

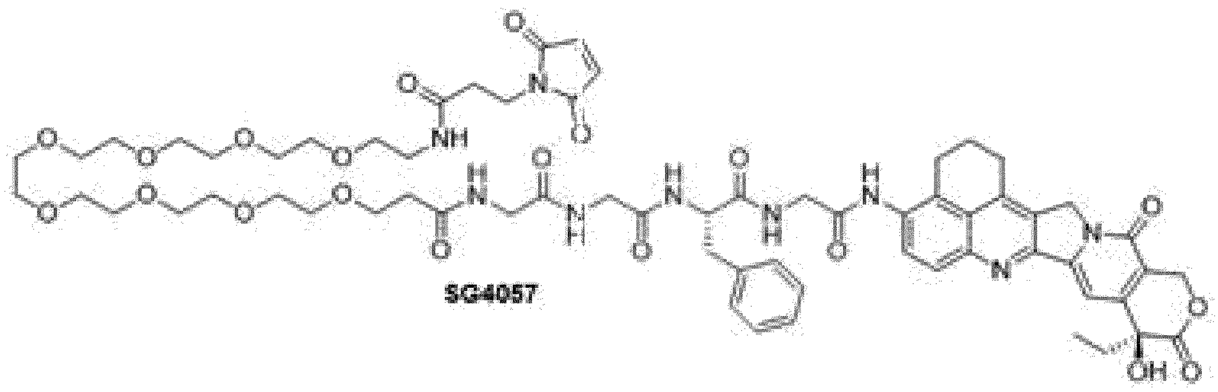

SG4057

Another representation of SG4052 is:

[Formula 34]

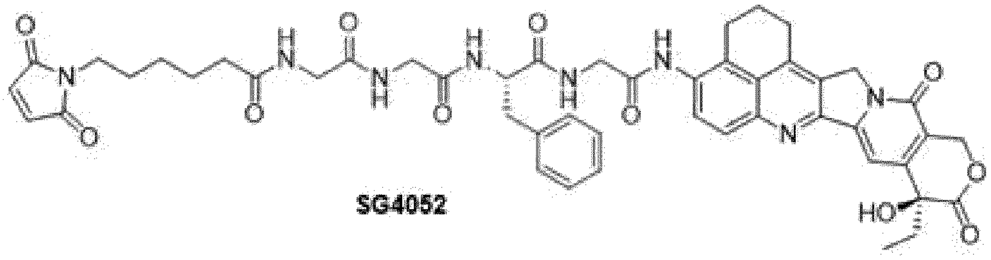

SG4052

Any antibody or antigen binding fragment thereof described herein may be conjugated to one or more of said topoisomerase I inhibitor(s).

**IV. Further Chemical Definitions.**

The following definitions pertain, in particular, to the description of topoisomerase I inhibitors above, and may even more particularly pertain to the section entitled "Further Preferences of the Preferred Embodiments".

$C_{5-6}$ arylene: The term "$C_{5-6}$ arylene", as used herein, pertains to a divalent moiety obtained by removing two hydrogen atoms from an aromatic ring atom of an aromatic compound.

In this context, the prefixes (e.g., $C_{5-6}$) denote the number of ring atoms, or range of number of ring atoms, whether carbon atoms or heteroatoms. The ring atoms may be all carbon atoms, as in "carboarylene groups", in which case the group is phenylene ($C_6$). Alternatively, the ring atoms may include one or more heteroatoms, as in "heteroarylene groups". Examples of heteroarylene groups include, but are not limited to, those derived from:

$N_1$: pyrrole (azole) ($C_5$), pyridine (azine) ($C_6$);
$O_1$: furan (oxole) ($C_5$);
$S_1$: thiophene (thiole) ($C_5$);
$N_1O_1$: oxazole ($C_5$), isoxazole ($C_5$), isoxazine ($C_6$);
$N_2O_1$: oxadiazole (furazan) ($C_5$);
$N_3O_1$: oxatriazole ($C_5$);
$N_1S_1$: thiazole ($C_5$), isothiazole ($C_5$);
$N_2$: imidazole (1,3-diazole) ($C_5$), pyrazole (1,2-diazole) ($C_5$), pyridazine (1,2-diazine) ($C_6$), pyrimidine (1,3-diazine) ($C_6$) (e.g., cytosine, thymine, uracil), pyrazine (1,4-diazine) ($C_6$); and
$N_3$: triazole ($C_5$), triazine ($C_6$).
$C_{1-4}$ alkyl: The term "$C_{1-4}$ alkyl" as used herein, pertains to a monovalent moiety obtained by removing a hydrogen atom from a carbon atom of a hydrocarbon compound having from 1 to 4 carbon atoms, which may be aliphatic or alicyclic, and which may be saturated or unsaturated (e.g. partially unsaturated, fully unsaturated). The term "$C_{1-n}$ alkyl" as used herein, pertains to a monovalent moiety obtained by removing a hydrogen atom from a carbon atom of a hydrocarbon compound having from 1 to n carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl ($C_1$), ethyl ($C_2$), propyl ($C_3$) and butyl ($C_4$). Examples of saturated linear alkyl groups include, but are not limited to, methyl ($C_1$), ethyl ($C_2$), n-propyl ($C_3$) and n-butyl ($C_4$). Examples of saturated branched alkyl groups include iso-propyl ($C_3$), iso-butyl ($C_4$), sec-butyl ($C_4$) and tert-butyl ($C_4$).

$C_{2-4}$ Alkenyl: The term "$C_{2-4}$ alkenyl" as used herein, pertains to an alkyl group having one or more carbon-carbon double bonds. Examples of unsaturated alkenyl groups include, but are not limited to, ethenyl (vinyl. $-CH=CH_2$), 1-propenyl ($-CH=CH-CH_3$), 2-propenyl (allyl, $-CH-CH=CH_2$), isopropenyl (1-methylvinyl, $-C(CH_3)=CH_2$) and butenyl ($C_4$).

$C_{2-4}$ alkynyl: The term "$C_{2-4}$ alkynyl" as used herein, pertains to an alkyl group having one or more carbon-carbon triple bonds. Examples of unsaturated alkynyl groups include, but are not limited to, ethynyl ($-C\equiv CH$) and 2-propynyl (propargyl, $-CH_2-C\equiv CH$).

$C_{3-4}$ cycloalkyl: The term "$C_{3-4}$ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms. Examples of cycloalkyl groups include, but are not limited to, those derived from: saturated monocyclic hydrocarbon compounds, such as cyclopropane ($C_3$) and cyclobutane ($C_4$); and unsaturated monocyclic hydrocarbon compounds, such as cyclopropene ($C_3$) and cyclobutene ($C_4$).

Connection labels: In Formula 35 below, the superscripted labels $^{C(=O)}$ and $^{NH}$ indicate the group to which the

atoms are bound.

[Formula 35]

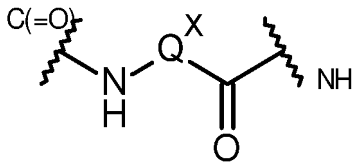

For example, the NH group is shown as being bound to a carbonyl (which is not part of the moiety illustrated), and the carbonyl is shown as being bound to a NH group (which is not part of the moiety illustrated).

V. **Synthesis of Topoisomerase I Inhibitors.**

Compounds I of Formula 2, where $R^L$ is group Ia of Formula 3 may be synthesised from the compound of Formula 36:

[Formula 36]

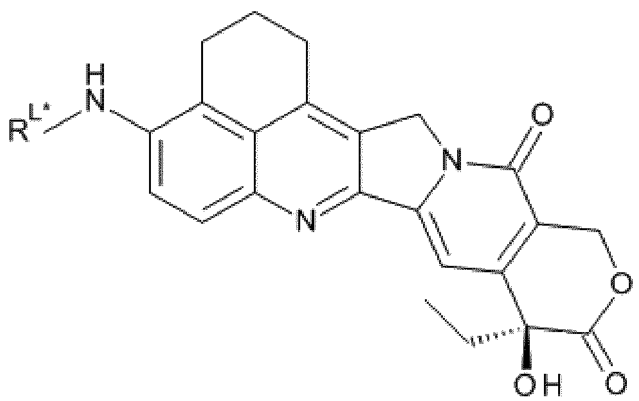

where $R^{L*}$ is -QH by linking a compound of Formula 37:

[Formula 37]

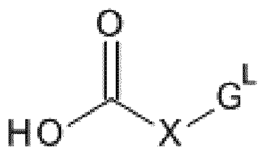

or an activated version thereof. Such a reaction may be carried out under amide coupling conditions. Compounds of Formula 36 may be synthesised by the deprotection of a compound of Formula 38:

[Formula 38]

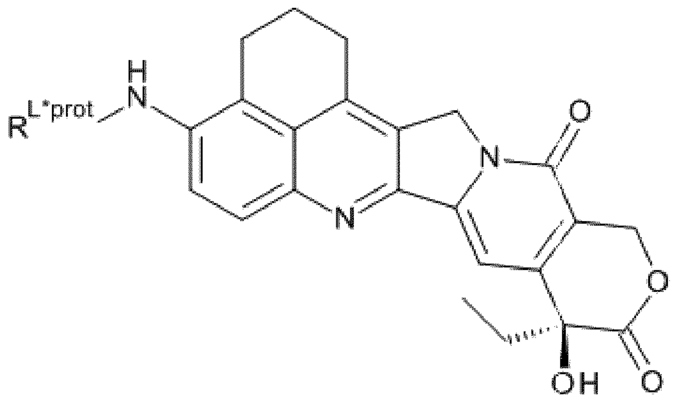

where $R^{L*prot}$ is -Q-$Prot^N$, and where $Prot^N$ is an amine protecting group. Compounds of Formula 38 may be synthesised by the coupling of a compound of Formula 39:

[Formula 39]

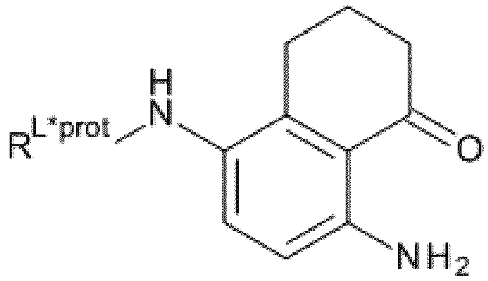

with the compound A3 using the Friedlander reaction. The compound A3 is (*S*)-4-ethyl-4-hydroxy-7,8-dihydro-1*H*-pyrano[3,4-*f*]indolizine-3,6,10(4*H*)-trione. Compounds of Formula 39 may be synthesised from compounds of Formula 40:

[Formula 40]

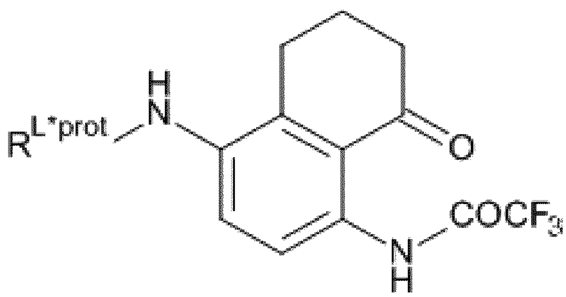

by removal of the trifluoroacetamide protecting group. Compounds of Formula 40 may be synthesised by coupling: $R^{L*prot}$-OH to the compound I7. The compound I7 is *N*-(4-amino-8-oxo-5,6,7,8-tetrahydronaphthalen-1-yl)-2,2,2-trifluoroacetamide. Compounds I of Formula 2, where $R^L$ is group Ia of Formula 3 or group Ib of Formula 6, may be synthesised from the compound I11 by coupling of the compound $R^L$-OH, or an activated form thereof. The compound I11 is (*S*)-4-amino-9-ethyl-9-hydroxy-1,2,3,9,12,15-hexahydro-10*H*,13*H*-benzo[de]pyrano[3',4':6,7]indolizino[1,2-*b*]quinoline-10,13-dione.

Amine protecting groups are well-known to those skilled in the art. Particular reference is made to the disclosure of suitable protecting groups in Greene's Protecting Groups in Organic Synthesis, Fourth Edition. John Wiley & Sons, 2007 (ISBN 978-0-471-69754-1), pages 696-871.

### VI. The anti-B7H4 antibody.

The term "antibody" covers monoclonal antibodies and fragments thereof (e.g., exhibiting the desired biological activity). In a preferable embodiment, an antibody described herein is a monoclonal antibody. In a more preferable embodiment, the antibody is a fully human monoclonal antibody. In one embodiment, methods of the invention may employ polyclonal antibodies.

In particular, an antibody is a protein including at least one or two, heavy (H) chain variable regions (abbreviated herein as VHC), and at least one or two light (L) chain variable regions (abbreviated herein as VLC). The VHC and VLC regions can be further subdivided into regions of hypervariability, termed "complementarity determining regions" ("CDR"), interspersed with regions that are more conserved, termed "framework regions" (FR). The extent of the framework region and CDRs has been precisely defined (see, Kabat, E.A., et al. Sequences of Proteins of Immunological Interest, Fifth Edition, U.S. Department of Health and Human Services, NIH Publication No. 91-3242, 1991, and Chothia, C. et al, J. Mol. Biol. 196:901-917, 1987).

Preferably, each VHC and VLC is composed of three CDRs and four FRs, arranged from amino-terminus to carboxy-terminus in the following order: FR1, CDR1, FR2, DR2, FR3, CDR3, FR4. The VHC or VLC chain of the antibody can further include all or part of a heavy or light chain constant region. In one embodiment, the antibody is a tetramer of two heavy immunoglobulin chains and two light immunoglobulin chains, wherein the heavy and light immunoglobulin chains are interconnected by, e.g., disulfide bonds. The heavy chain constant region includes three domains, CH1, CH2 and CH3. The light chain constant region is comprised of one domain, CL. The variable region of the heavy and light chains contains a binding domain that interacts with an antigen.

The term "antibody" includes intact immunoglobulins of types IgA, IgG, IgE, IgD, IgM (as well as subtypes thereof), wherein the light chains of the immunoglobulin may be of types kappa or lambda. The term antibody, as used herein, also refers to a portion of an antibody that binds to one of the above-mentioned markers, e.g., a molecule in which one or more immunoglobulin chains is not full length, but which binds to a marker. Examples of binding portions encompassed within the term antibody include (i) a Fab fragment, a monovalent fragment consisting of the VLC, VHC, CL and CH1 domains; (ii) a F(ab')2 fragment, a bivalent fragment comprising two Fab fragments linked by a disulfide bridge at the hinge region; (iii) a Fc fragment consisting of the VHC and CH1 domains; (iv) a Fv fragment consisting of the VLC and VHC domains of a single arm of an antibody, (v) a dAb fragment (Ward et al, Nature 341:544-546, 1989), which consists of a VHC domain; and (vi) an isolated complementarity determining region (CDR) having sufficient framework to bind, e.g. an antigen binding portion of a variable region. An antigen binding portion of a light chain variable region and an antigen binding portion of a heavy chain variable region, e.g., the two domains of the Fv fragment, VLC and VHC, can be joined, using recombinant methods, by a synthetic linker that enables them to be made as a single protein chain in which the VLC and VHC regions pair to form monovalent molecules (known as single chain Fv (scFv); see e.g., Bird et al. (1988) Science 1Al-ATi-Alβ; and Huston et al. (1988) Proc. Natl. Acad. ScL USA 85:5879-5883). Such single chain antibodies are also encompassed within the term antibody. These may be obtained using conventional techniques known to those skilled in the art, and the portions are screened for utility in the same manner as are intact antibodies.

In one embodiment, the antibody or antigen binding fragment is one or more selected from a murine antibody, a humanized antibody, a chimeric antibody, a monoclonal antibody, a polyclonal antibody, a recombinant antibody, a multispecific antibody, or a combination thereof.

In one embodiment, the antigen-binding fragment is one or more selected from a Fv fragment, an Fab fragment, an F(ab')2 fragment, an Fab' fragment, a dsFv fragment, an scFv fragment, an sc(Fv)2 fragment, or a combination thereof.

In a preferable embodiment, the antibody or antigen binding fragment thereof is a monoclonal antibody (mAb). In one embodiment, the antibody or antigen binding fragment thereof (e.g. mAb) of the invention is a scFV.

In one embodiment, the antibody or antigen binding fragment thereof can bind to B7-H4 molecules across species, e.g., the antibody or fragment can bind to mouse B7-H4, rat B7-H4, rabbit, human B7-H4 and/or cynomolgus monkey B7-H4. In one embodiment, the antibody or fragment can bind to human B7-H4 and cynomolgus monkey B7-H4. In one embodiment, the antibody or antigen binding fragment can also bind to mouse B7-H4. In one embodiment, the antibody or antigen binding fragment thereof can specifically bind to B7-H4, e.g., human B7-H4 and cynomolgus monkey B7-H4, but does not specifically bind to human B7-H1, B7-H2, and/or B7-H3.

In one embodiment, the antibody or antigen-binding fragment thereof can include, in addition to a VH and a VL, a heavy chain constant region or fragment thereof. In one embodiment, the heavy chain constant region is a human heavy chain constant region, e.g., a human IgG constant region, e.g., a human IgG1 constant region. In one embodiment (preferably where the antibody or antigen-binding fragment thereof is conjugated to an agent, such as a cytotoxic agent), a cysteine residue is inserted between amino acid S239 and V240 in the CH2 region of IgG1. This cysteine is referred to as "a 239 insertion" or "239i."

**VII. Preparation of the anti-B7H4 antibody.**

The antibodies described herein can be obtained using conventional techniques known to persons skilled in the art and their utility confirmed by conventional binding studies. By way of example, a simple binding assay is to incubate the cell expressing an antigen with the antibody. If the antibody is tagged with a fluorophore, the binding of the antibody to the antigen can be detected by FACS analysis.

Antibodies described herein can be raised in various animals including mice, rats, rabbits, goats, sheep, monkeys or horses. Antibodies may be raised following immunization with individual capsular polysaccharides, or with a plurality of capsular polysaccharides. Blood isolated from these animals contains polyclonal antibodies - multiple antibodies that bind to the same antigen. Antigens may also be injected into chickens for generation of polyclonal antibodies in egg yolk. To obtain a monoclonal antibody that is specific for a single epitope of an antigen, antibody-secreting lymphocytes are isolated from an animal and immortalized by fusing them with a cancer cell line. The fused cells are called hybridomas, and will continually grow and secrete antibody in culture. Single hybridoma cells are isolated by dilution cloning to generate cell clones that all produce the same antibody; these antibodies are called monoclonal antibodies. Methods for producing monoclonal antibodies are conventional techniques known to those skilled in the art (see e.g. Making and Using Antibodies: A Practical Handbook. GC Howard. CRC Books. 2006. ISBN 0849335280). Polyclonal and monoclonal antibodies are often purified using Protein A/G or antigen-affinity chromatography.

The antibody or antigen binding fragment described herein may be prepared as a monoclonal anti-B7H4 antibody, which can be prepared using hybridoma methods, such as those described by Kohler and Milstein, Nature 256:495 (1975). Using the hybridoma method, a mouse, hamster, or other appropriate host animal, is immunized as described above to elicit the production by lymphocytes of antibodies that will specifically bind to an immunizing antigen. Lymphocytes can also be immunized in vitro. Following immunization, the lymphocytes are isolated and fused with a suitable myeloma cell line using, for example, polyethylene glycol, to form hybridoma cells that can then be selected away from unfused lymphocytes and myeloma cells. Hybridomas that produce monoclonal antibodies directed specifically against a chosen antigen as determined by immunoprecipitation, immunoblotting, or an in vitro binding assay, e.g., radioimmunoassay (RIA) or enzyme-linked immunosorbent assay (ELISA), can then be propagated either in *in vitro* culture using standard methods (Goding, Monoclonal Antibodies: Principles and Practice, Academic Press, 1986) or in vivo as ascites tumors in an animal. The monoclonal antibodies can then be purified from the culture medium or ascites fluid using known methods.

Alternatively, the antibody or antigen binding fragment thereof (e.g. as monoclonal antibodies) can also be made using recombinant DNA methods as described in U.S. Patent No. 4,816,567. The polynucleotides encoding a monoclonal antibody are isolated from mature B-cells or hybridoma cell, such as by RT-PCR using oligonucleotide primers that specifically amplify the genes encoding the heavy and light chains of the antibody, and their sequence is determined using conventional procedures.

The isolated polynucleotides encoding the heavy and light chains are then cloned into suitable expression vectors, which when transfected into host cells such as E. coli cells, simian COS cells, Chinese hamster ovary (CHO) cells, or myeloma cells that do not otherwise produce immunoglobulin protein, monoclonal antibodies are generated by the host cells. Also, recombinant monoclonal antibodies or antigen-binding fragments thereof of the desired species can be isolated from phage display libraries expressing CDRs of the desired species as described in McCafferty et al., Nature 348:552-554 (1990); Clackson et al., Nature, 352:624-628 (1991); and Marks et al., J. Mol. Biol. 222:581-597 (1991).

The polynucleotide(s) encoding an antibody or an antigen-binding fragment thereof of the invention can further be modified in a number of different manners using recombinant DNA technology to generate alternative antibodies. In some embodiments, the constant domains of the light and heavy chains of, for example, a mouse monoclonal antibody can be substituted (1) for those regions of, for example, a human antibody to generate a chimeric antibody or (2) for a non-immunoglobulin polypeptide to generate a fusion antibody. In some embodiments, the constant regions are truncated or removed to generate the desired antibody fragment of a monoclonal antibody. Site-directed or high-density mutagenesis of the variable region can be used to optimize specificity, affinity, etc. of a monoclonal antibody.

In one embodiment, the antibody or antigen-binding fragment thereof is a human antibody or antigen-binding fragment thereof. Human antibodies can be directly prepared using various techniques known in the art. Immortalized human B lymphocytes immunized in vitro or isolated from an immunized individual that produce an antibody directed against a target antigen can be generated. See, e.g., Cole et al., Monoclonal Antibodies and Cancer Therapy, Alan R. Liss, p. 77 (1985); Boemer et al., J. Immunol. 147 (1):86-95 (1991); U.S. Patent 5,750,373.

In one embodiment, the antibody or antigen-binding fragment thereof can be selected from a phage library, where that phage library expresses human antibodies, as described, for example, in Vaughan et al., Nat. Biotech. 14:309-314 (1996); Sheets et al., Proc. Natl. Acad. Sci. USA, 95:6157-6162 (1998); Hoogenboom and Winter, J. Mol. Biol. 227:381 (1991); and Marks et al., J. Mol. Biol. 222:581 (1991). Techniques for the generation and use of antibody phage libraries are also described in U.S. Patent Nos. 5,969,108, 6,172,197, 5,885,793, 6,521,404; 6,544,731; 6,555,313; 6,582,915; 6,593,081; 6,300,064; 6,653,068; 6,706,484; and 7,264,963; and Rothe et al., J. Molec. Biol. 376:1182-1200 (2008),

Affinity maturation strategies and chain shuffling strategies are known in the art and can be employed to generate high affinity human antibodies or antigen-binding fragments thereof. See Marks et al., BioTechnology 10:779-783 (1992), incorporated by reference in its entirety.

In one embodiment, the antibody or antigen binding fragment thereof (e.g. an monoclonal antibody) can be a humanized antibody. Methods for engineering, humanizing or resurfacing non-human or human antibodies can also be used and are well known in the art. A humanized, resurfaced or similarly engineered antibody can have one or more amino acid residues from a source that is non-human, e.g., but not limited to, mouse, rat, rabbit, non-human primate, or other mammal. These non-human amino acid residues are replaced by residues that are often referred to as "import" residues, which are typically taken from an "import" variable, constant or other domain of a known human sequence. Such imported sequences can be used to reduce immunogenicity or reduce, enhance or modify binding, affinity, on-rate, off-rate, avidity, specificity, half-life, or any other suitable characteristic, as known in the art. Suitably, the CDR residues may be directly and most substantially involved in influencing B7-H4 binding. Accordingly, part or all of the non-human or human CDR sequences are preferably maintained while the non-human sequences of the variable and constant regions can be replaced with human or other amino acids.

Antibodies can also optionally be humanized, resurfaced, engineered or human antibodies engineered with retention of high affinity for the antigen B7-H4 and other favorable biological properties. To achieve this goal, humanized (or human) or engineered anti-B7H4 antibodies and resurfaced antibodies can be optionally prepared by a process of analysis of the parental sequences and various conceptual humanized and engineered products using three-dimensional models of the parental, engineered, and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen, such as B7-H4. In this way, FW residues can be selected and combined from the consensus and import sequences so that the desired antibody characteristic, such as increased affinity for the target antigen(s), is achieved.

Humanization, resurfacing or engineering of anti-B7H4 antibodies or antigen-binding fragments thereof of the present invention can be performed using any known method, such as but not limited to those described in, Jones et al., Nature 321:522 (1986); Riechmann et al., Nature 332:323 (1988); Verhoeyen et al., Science 239:1534 (1988); Sims et al., J. Immunol. 151: 2296 (1993); Chothia and Lesk, J. Mol. Biol. 196:901 (1987); Carter et al., Proc. Natl. Acad. Sci. USA 89:4285 (1992); Presta et al., J. Immunol. 151:2623 (1993); U.S. Pat. Nos. 5,639,641, 5,723,323; 5,976,862; 5,824,514; 5,817,483; 5,814,476; 5,763,192; 5,723,323; 5,766,886; 5,714,352; 6,204,023; 6,180,370; 5,693,762; 5,530,101; 5,585,089; 5,225,539; 4,816,567, 7,557,189; 7,538,195; and 7,342,110; International Application Nos. PCT/US98/16280; PCT/US96/18978; PCT/US91/09630; PCT/US91/05939; PCT/US94/01234; PCT/GB89/01334; PCT/GB91/01134; PCT/GB92/01755; International Patent Application Publication Nos. WO90/14443; WO90/14424; WO90/14430; and European Patent Publication No. EP 229246;

Anti-B7H4 humanized antibodies and antigen-binding fragments thereof can also be made in transgenic mice containing human immunoglobulin loci that are capable upon immunization of producing the full repertoire of human antibodies in the absence of endogenous immunoglobulin production. This approach is described in U.S. Patent Nos. 5,545,807; 5,545,806; 5,569,825; 5,625,126; 5,633,425; and 5,661,016.

In one embodiment, a fragment (e.g. antibody fragment) of the antibody (e.g., anti-B7H4 antibody) is provided. Various techniques are known for the production of antibody fragments. Traditionally, these fragments are derived via proteolytic digestion of intact antibodies, as described, for example, by Morimoto et al., J. Biochem. Biophys. Meth. 24:107-117 (1993) and Brennan et al., Science 229:81 (1985). In one embodiment, anti-B7H4 antibody fragments are produced recombinantly. Fab, Fv, and scFv antibody fragments can all be expressed in and secreted from *E. coli* or other host cells, thus allowing the production of large amounts of these fragments. Such anti-B7-H4 antibody fragments can also be isolated from the antibody phage libraries discussed above. The anti-B7-H4 antibody fragments can also be linear antibodies as described in U.S. Patent No. 5,641,870. Other techniques for the production of antibody fragments will be apparent to the skilled practitioner.

According to the present invention, techniques can be adapted for the production of single-chain antibodies specific to B7-H4. See, e.g., U.S. Pat. No. 4,946,778). In addition, methods can be adapted for the construction of Fab expression libraries to allow rapid and effective identification of monoclonal Fab fragments with the desired specificity for B7-H4, or derivatives, fragments, analogs or homologs thereof. See, e.g., Huse et al., Science 246:1275-1281 (1989). Antibody fragments can be produced by techniques known in the art including, but not limited to: F(ab')2 fragment produced by pepsin digestion of an antibody molecule; Fab fragment generated by reducing the disulfide bridges of an F(ab')2 fragment; Fab fragment generated by the treatment of the antibody molecule with papain and a reducing agent; or Fv fragments.

In one embodiment, an antibody or antigen-binding fragment thereof described herein can be modified in order to increase its serum half-life. This can be achieved, for example, by incorporation of a salvage receptor binding epitope into

the antibody or antibody fragment, by mutation of the appropriate region in the antibody or antibody fragment or by incorporating the epitope into a peptide tag that is then fused to the antibody or antibody fragment at either end or in the middle (e.g., by DNA or peptide synthesis), or by YTE mutation. Other methods to increase the serum half-life of an antibody or antigen-binding fragment thereof, e.g., conjugation to a heterologous molecule, such as PEG, are known in the art.

A modified antibody or antigen-binding fragment thereof as provided herein can comprise any type of variable region that provides for the association of the antibody or polypeptide with B7-H4. In this regard, the variable region can comprise or be derived from any type of mammal that can be induced to mount a humoral response and generate immunoglobulins against the desired antigen. As such, the variable region of an anti-B7H4 antibody or antigen-binding fragment thereof can be, for example, of human, murine, non-human primate (e.g., cynomolgus monkeys, macaques, etc.) or lupine origin. In one embodiment, both the variable and constant regions of the modified antibody or antigen-binding fragment thereof are human. In one embodiment, the variable regions of a compatible antibody (usually derived from a non-human source) can be engineered or specifically tailored to improve the binding properties or reduce the immunogenicity of the molecule. In this respect, variable regions useful in the present invention can be humanized or otherwise altered through the inclusion of imported amino acid sequences.

In one embodiment, the variable domains in both the heavy and light chains of an antibody or antigen-binding fragment thereof are altered by at least partial replacement of one or more CDRs and/or by partial framework region replacement and sequence changing. Although the CDRs can be derived from an antibody of the same class or even subclass as the antibody from which the framework regions are derived, it is envisaged that the CDRs will be derived from an antibody of different class and in certain embodiments from an antibody from a different species. It is not necessary to replace all of the CDRs with the complete CDRs from the donor variable region to transfer the antigen-binding capacity of one variable domain to another. Rather, it is only necessary to transfer those residues that are necessary to maintain the activity of the antigen-binding site. Given the explanations set forth in U.S. Pat. Nos. 5,585,089, 5,693,761 and 5,693,762, it will be well within the competence of those skilled in the art to carry out routine experimentation to obtain a functional antibody with reduced immunogenicity.

Alterations to the variable region notwithstanding, those skilled in the art will appreciate that a modified antibody or antigen-binding fragment thereof described herein will comprise an antibody (e.g., full-length antibody or antigen-binding fragment thereof) in which at least a fraction of one or more of the constant region domains has been deleted or otherwise altered so as to provide desired biochemical characteristics such as increased tumor localization or reduced serum half-life when compared with an antibody of approximately the same immunogenicity comprising a native or unaltered constant region. In one embodiment, the constant region of the modified antibody will comprise a human constant region. Modifications to the constant region compatible with this invention comprise additions, deletions or substitutions of one or more amino acids in one or more domains. That is, a modified antibody disclosed herein can comprise alterations or modifications to one or more of the three heavy chain constant domains (CH1, CH2 or CH3) and/or to the light chain constant domain (CL). In one embodiment, a modified constant region wherein one or more domains are partially or entirely deleted are contemplated. In one embodiment, a modified antibody will comprise domain deleted constructs or variants wherein the entire CH2 domain has been removed (ΔCH2 constructs). In one embodiment, the omitted constant region domain can be replaced by a short amino acid spacer (e.g., 10 residues) that provides some of the molecular flexibility typically imparted by the absent constant region.

Besides their configuration, it is known in the art that the constant region mediates several effector functions. For example, antibodies bind to cells via the Fc region, with an Fc receptor site on the antibody Fc region binding to an Fc receptor (FcR) on a cell. There are a number of Fc receptors that are specific for different classes of antibody, including IgG (gamma receptors), IgE (eta receptors), IgA (alpha receptors) and IgM (mu receptors). Binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell-mediated cytotoxicity, or ADCC), release of inflammatory mediators, placental transfer and control of immunoglobulin production.

In one embodiment, an antibody or an antigen-binding fragment thereof provides for altered effector functions that, in turn, affect the biological profile of the administered antibody or antigen-binding fragment thereof. For example, the deletion or inactivation (through point mutations or other means) of a constant region domain can reduce Fc receptor binding of the circulating modified antibody. In other cases it can be that constant region modifications, consistent with this invention, moderate complement binding and thus reduce the serum half-life and nonspecific association of a conjugated cytotoxin. Yet other modifications of the constant region can be used to eliminate disulfide linkages or oligosaccharide moieties that allow for enhanced localization due to increased antigen specificity or antibody flexibility. Similarly, modifications to the constant region in accordance with this invention can easily be made using well-known biochemical or molecular engineering techniques well within the purview of the skilled artisan.

In one embodiment, the antibody or antigen-binding fragment thereof does not have one or more effector functions. For instance, in one embodiment, the antibody or antigen-binding fragment thereof has no antibody-dependent

cellular cytoxicity (ADCC) activity and/or no complement-dependent cytoxicity (CDC) activity. In one embodiment, the antibody or antigen-binding fragment thereof does not bind to an Fc receptor and/or complement factors. In one embodiment, the antibody or antigen-binding fragment thereof has no effector function.

In one embodiment, the antibody or antigen-binding fragment thereof can be engineered to fuse the CH3 domain directly to the hinge region of the respective modified antibodies or fragments thereof. In other constructs, a peptide spacer can be inserted between the hinge region and the modified CH2 and/or CH3 domains. For example, compatible constructs can be expressed in which the CH2 domain has been deleted and the remaining CH3 domain (modified or unmodified) is joined to the hinge region with a 5-20 amino acid spacer. Such a spacer can be added, for instance, to ensure that the regulatory elements of the constant domain remain free and accessible or that the hinge region remains flexible. Amino acid spacers can, in some cases, prove to be immunogenic and elicit an unwanted immune response against the construct. In one embodiment, any spacer added to the construct can be relatively non-immunogenic, or even omitted altogether, so as to maintain the desired biochemical qualities of the modified antibodies.

Besides the deletion of whole constant region domains, an antibody or antigen-binding fragment thereof provided herein can be modified by the partial deletion or substitution of a few or even a single amino acid in a constant region. For example, the mutation of a single amino acid in selected areas of the CH2 domain can be enough to substantially reduce Fc binding and thereby increase tumor localization. Similarly one or more constant region domains that control the effector function (e.g., complement C1Q binding) can be fully or partially deleted. Such partial deletions of the constant regions can improve selected characteristics of the antibody or antigen-binding fragment thereof (e.g., serum half-life) while leaving other desirable functions associated with the subject constant region domain intact. Moreover, the constant regions of the antibody and antigen-binding fragment thereof can be modified through the mutation or substitution of one or more amino acids that enhances the profile of the resulting construct. In this respect it is possible to disrupt the activity provided by a conserved binding site (e.g., Fc binding) while substantially maintaining the configuration and immunogenic profile of the modified antibody or antigen-binding fragment thereof. In one embodiment, there may be an addition of one or more amino acids to the constant region to enhance desirable characteristics such as decreasing or increasing effector function or provide for more cytotoxin or carbohydrate attachment. In one embodiment, it can be desirable to insert or replicate specific sequences derived from selected constant region domains.

The antibodies and antibody binding fragments described herein further embrace variants and equivalents that are substantially homologous an antibody or antigen binding fragment specifically described herein (e.g. murine, chimeric, humanized or human antibody, or antigen-binding fragments thereof). These can contain, for example, conservative substitution mutations, i.e., the substitution of one or more amino acids by similar amino acids. For example, conservative substitution refers to the substitution of an amino acid with another within the same general class such as, for example, one acidic amino acid with another acidic amino acid, one basic amino acid with another basic amino acid or one neutral amino acid by another neutral amino acid. What is intended by a conservative amino acid substitution is well known in the art.

In one embodiment, the antibody or antigen-binding fragment thereof can be further modified to contain additional chemical moieties not normally part of the protein. Those derivatized moieties can improve the solubility, the biological half-life or absorption of the protein. The moieties can also reduce or eliminate any desirable side effects of the proteins and the like. An overview for those moieties can be found in Remington's Pharmaceutical Sciences, 22nd ed., Ed. Lloyd V. Allen, Jr. (2012).

## **VIII.** Uses **of the anti-B7H4 antibody-drug conjugate.**

Preferably, the conjugates can be used to treat proliferative disease. The term "proliferative disease" pertains to an unwanted or uncontrolled cellular proliferation of excessive or abnormal cells which is undesired, such as, neoplastic or hyperplastic growth, whether *in vitro* or *in vivo.* The term "proliferative disease" may alternatively be referred to as "cancer".

A suitable proliferative disease (e.g., cancer) will preferably be characterized by the presence cancerous cells that express B7-H4.

Examples of proliferative conditions include, but are not limited to, benign, pre-malignant, and malignant cellular proliferation, including but not limited to, neoplasms and tumors (e.g., histocytoma, glioma, astrocyoma, osteoma), cancers (e.g., lung cancer, small cell lung cancer, gastrointestinal cancer, bowel cancer, colon cancer, breast carcinoma, ovarian carcinoma, prostate cancer, testicular cancer, liver cancer, kidney cancer, bladder cancer, pancreatic cancer, brain cancer, sarcoma, osteosarcoma, Kaposi's sarcoma, melanoma), leukemias, psoriasis, bone diseases, fibroproliferative disorders (e.g., of connective tissues), and atherosclerosis. Other cancers of interest include, but are not limited to, haematological; malignancies such as leukemias and lymphomas, such as non-Hodgkin lymphoma, and subtypes such as DLBCL, marginal zone, mantle zone, and follicular, Hodgkin lymphoma, AML, and other cancers of B or T cell origin. Any type of cell may be treated, including but not limited to, lung, gastrointestinal (including, e.g. bowel, colon), breast (mammary), ovarian, prostate, liver (hepatic), kidney (renal), bladder, pancreas, brain, and skin.

In some embodiments, the antibody-drug conjugates described herein are used to treat a cancer selected from the list comprising: breast cancer, ovarian cancer, endometrial cancer, cholangiocarcinoma, lung cancer, pancreatic

cancer, and gastric cancer. In some embodiments, the antibody-drug conjugates described herein are used to treat a cancer selected from the list comprising: breast cancer, ovarian cancer, endometrial cancer, and cholangiocarcinoma. In some embodiments, the breast cancer is hormone receptor positive (HR+) breast cancer. In some embodiments, the breast cancer is human epidermal growth factor receptor 2 positive (HER2+) breast cancer. In other embodiments, the breast cancer is triple negative breast cancer (TNBC). In some embodiments, the lung cancer is non-small cell lung cancer (NSCLC). In some embodiments, the NSCLC is a squamous cell carcinoma. In other embodiments, the NSCLC is an adenocarcinoma.

The antibody-drug conjugate of the present invention can retard cancer cell growth, suppress its proliferation, and further disrupt cancer cells. These actions can accomplish relief from a symptom caused by cancer, improvement of quality of life (QOL) in a cancer patient, and preserves the life of a cancer patient, thus achieving a therapeutic effect. Even in failing to lead to disruption of cancer cells, suppression or control of cancer cell proliferation can result in achieving longer-term survival as well as accomplishing higher QOL in a cancer patient.

The anti-H7B4 ADC of the present invention can be expected to exert a therapeutic effect by application as systemic therapy to patients, and additionally, by local application to cancer tissues.

The anti-H7B4 ADC may be administered as a pharmaceutical composition with one or more pharmaceutically compatible components contained. The pharmaceutically compatible component can be appropriately selected from formulation additives or the like that are generally used in the art, in view of the dosage, administration concentration or the like of the anti-H7B4 ADC. For example, the anti-H7B4 ADC used in the present invention may be administered as a pharmaceutical composition containing a buffering agent such as a histidine buffering agent, an excipient such as sucrose, and a surfactant such as polysorbate 80. The pharmaceutical composition containing the anti-H7B4 ADC used in the present invention can be used as an injection, as an aqueous injection or a lyophilized injection, or even as a lyophilized injection.

If the pharmaceutical composition containing the anti-H7B4 ADC used in the present invention is an aqueous injection, it can be diluted with a suitable diluent and then given as an intravenous infusion. Examples of the diluent can include dextrose solution and physiological saline.

If the pharmaceutical composition containing the anti-H7B4 antibody-drug conjugate used in the present invention is a lyophilized injection, it can be dissolved with injection-grade water, then diluted for a requisite amount with a suitable diluent and then given as an intravenous infusion. Examples of the diluent include dextrose solution and physiological saline.

Examples of the administration route possibly used for administering the pharmaceutical composition described herein can include intravenous, intradermal, subcutaneous, intramuscular, and intraperitoneal routes.

**IX. Method of predicting a patient response to an anti-B7H4 antibody-drug conjugate.**

FIG. 14 is a flowchart of steps 1-6 of a method 7 by which an analysis system analyzes a digital image of tissue from a cancer patient and predicts how the cancer patient will likely respond to a therapy involving an anti-B7H4 antibody-drug conjugate (ADC). In one embodiment, the method predicts the response to an ADC of a patient having a cancer selected from the group consisting of breast cancer, ovarian cancer, endometrial cancer, cholangiocarcinoma, lung cancer, pancreatic cancer, and gastric cancer. In one embodiment, the method predicts the response to an ADC of a patient having a cancer selected from the group consisting of breast cancer, ovarian cancer, endometrial cancer, and cholangiocarcinoma. In one embodiment, the method predicts the response to an ADC of a patient with breast cancer. In one embodiment, the method predicts the response to an ADC of a patient with ovarian cancer. In one embodiment, the method predicts the ADC response of a patient with endometrial cancer.

In a first step 1, a high-resolution digital image is acquired of a tissue slice from the cancer patient that has been stained using one or more biomarkers or stains.

To predict the efficacy of the ADC therapy, a diagnostic biomarker with an attached dye is used that targets the same protein as that targeted by the ADC therapy. In one embodiment, the anti-B7H4 ADC therapy to which the scoring is directed is an anti-B7H4 antibody conjugated to a topoisomerase I inhibitor. In one embodiment, the anti-B7H4 ADC comprises an anti-B7H4 antibody conjugated to a topoisomerase I inhibitor selected from:

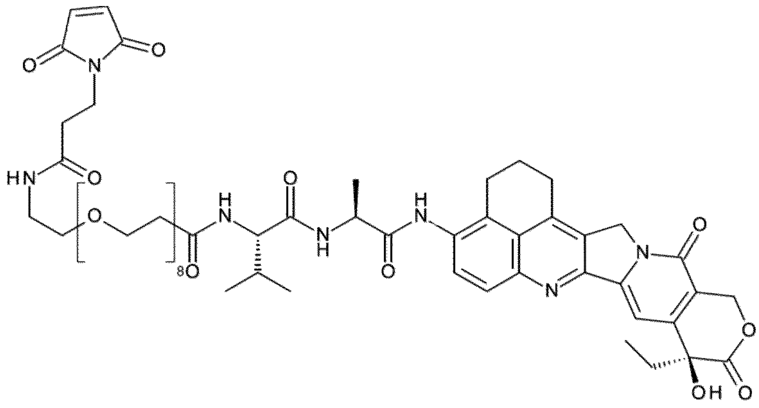
(SG3932);

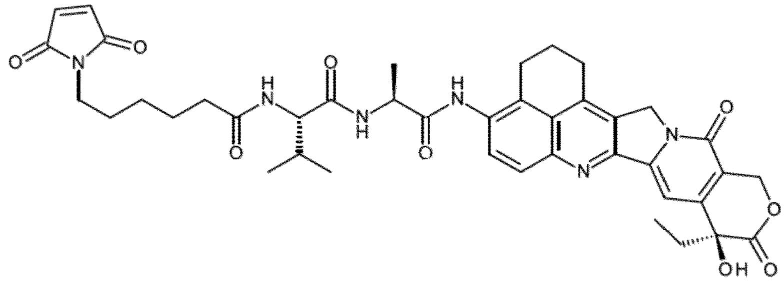
(SG4010);

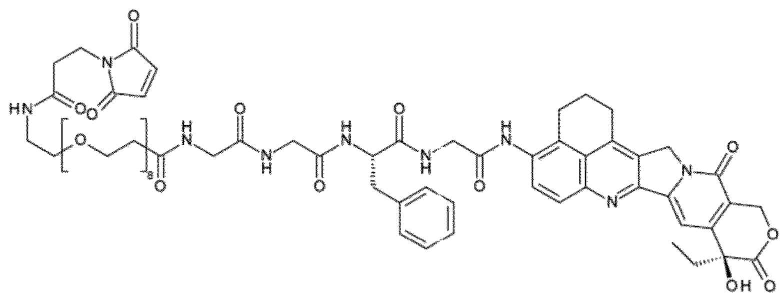
(SG4057);

and/or

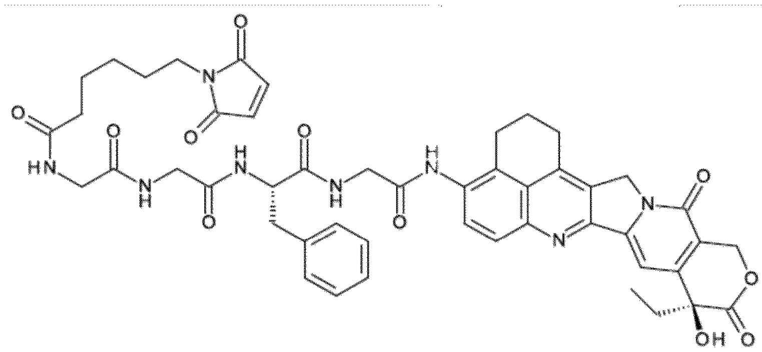
(SG4052).

In one embodiment, the anti-B7H4 ADC comprises an anti-B7H4 antibody conjugated to the topoisomerase inhibitor SG3932, which is represented by the structure below:

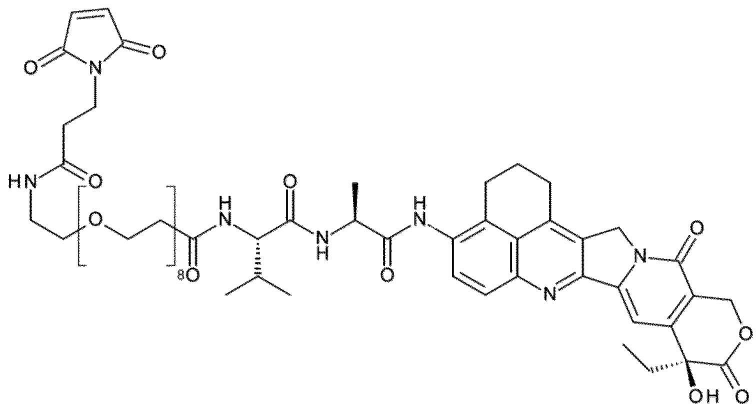 (SG3932).

Thus, in embodiments, the diagnostic biomarker also targets the B7-H4 protein.

In step 2, a pretrained convolutional neural network processes a digital image of tissue of the cancer patient that has been stained with the diagnostic antibody linked to the dye, such as 3,3'-Diaminobenzidine (DAB). The staining intensity of the dye in the membrane of a cancer cell is determined based on the mean staining intensity of the dye of all pixels associated with the corresponding segmented membrane object. Moreover, the staining intensity of the dye in a single pixel is computed based on the red, green and blue color components of the pixel. The result of the image analysis processing is two posterior image layers representing, for each pixel in the digital image, the probability that the pixel belongs to a cell nucleus and the probability that the pixel belongs to a cell membrane.

In another embodiment of step 2, two pretrained convolutional networks process the digital image of tissue. The result of the processing by the first network is a posterior image layer representing, for each pixel in the digital image, the likelihood that the pixel belongs to a cell nucleus. The result of the processing by the second network is a posterior image layer representing, for each pixel in the digital image, the likelihood that the pixel belongs to a cell membrane.

In step 3, individual cancer cells are detected based on a heuristic image analysis of the posterior layers for nuclei and membranes. Cancer cell objects are generated that include cell membrane objects and optionally also cell cytoplasm objects.

In step 4, a single-cell ADC score is determined for each cancer cell. The single-cell ADC score is based on (1) the amount of DAB in the cell membrane, and (2) on the amount of ADC payload uptake. The amount of DAB is determined by the staining intensity of each membrane based on the average optical density of the brown diaminobenzidine (DAB) signal in the pixels of the membrane. The amount of ADC payload uptake is estimated based on the amount of DAB in the cell membrane and optionally also in the cell cytoplasm, and further optionally also on the amount of DAB in the membranes and cytoplasm of neighboring cells to the cell for which the score is determined. The amount DAB in the cell cytoplasm is determined by the staining intensity of each cytoplasm, which is computed based on the average optical density of the brown DAB signal in pixels of the cytoplasm. The amount of DAB in neighboring cells is determined for those cancer cells within a predefined distance of the cell for which the score is being determined.

In step 5, a patient score is computed for the digital image of tissue based on a statistical operation on the single-cell ADC score of all cancer cells in the digital image. The patient score is indicative of how the cancer patient will respond to a therapy involving an anti-B7H4 ADC. The parameters of the single-cell ADC score in step 4 and the type of statistical operation in step 5 are optimized using a training cohort of patients with known responses to the ADC therapy. Optimization goals are low p-values in a Kaplan Meier analysis of the groups of score-positive versus score-negative patients in the training cohort.

In step 6, the therapy involving the anti-B7H4 ADC is recommended to score-positive patients if the score is larger than a predetermined threshold.

### X. Examples of prediction and scoring method.

### A. Image analysis of stained tissue.

The method of FIG. 14 is now described in relation to a particular image of stained cancer tissue.

In step 1, a tissue sample is immunohistochemically stained using a dye linked to a diagnostic antibody that binds to the associated protein on the cancer cells in the tissue sample. FIG. 15 (upper-left image) is a digital image 8 of a portion of stained tissue that was acquired in step 1. Image 8 shows tissue from a cancer patient that has been immunohistochemically stained with an anti-B7H4 diagnostic antibody linked to a dye. In this example, the diagnostic antibody is an anti-B7H4 generated clone (denoted B7H4 IHC Tool Ab) reformatted as a mouse anti-human B7H4 IgG1 clone. IgG1 indicates

the isotype of the anti-B7H4 antibody. The staining protocol was developed using the Dako Autostainer platform. The anti-B7H4 antibody binds to the membrane protein B7-H4 so that the 3,3'-Diaminobenzidine (DAB) stain indicates the location of the protein B7-H4 in the tissue sample.

In step 2, image analysis is performed on the digital image 8 to generate posterior image layers of cancer cell nuclei and membranes using a convolutional neural network. The image analysis is used to detect the cancer cells and their components, such as the nuclei, the membrane and the cytoplasm. FIG. 15 illustrates the image analysis process of step 2. The convolutional neural network generates posterior layers (gray value images) that indicate, for each pixel of digital image 8, the probability that each pixel belongs to either the nucleus (FIG. 15 upper-right image) or the membrane (FIG. 15 lower-left image) of the cell. High probabilities are shown in black, low probabilities in white.

In one embodiment, the convolutional neural network includes a series of convolution layers from the input image 8 towards a bottleneck layer with very low spatial size (1 to 16 pixels), and a series of deconvolution layers towards the posterior layers that have the same size as the input image 8. This network architecture is called a U-Net. The training of the weights of the convolutional neural networks is performed by generating manual annotation layers for nuclei and membranes in multiple training images, and then adjusting by an optimization algorithm the network weights so that the generated posterior layers are most similar to the manually generated annotation layers.

In another embodiment, the annotation layers for nuclei and membranes are generated automatically and corrected manually in multiple training images. Epithelium regions and nuclei centers are manually annotated as regions and points, respectively. For each training image, the membrane segmentation is automatically generated by applying a region growing-like algorithm (e.g., watershed segmentation) seeded by the annotated nuclei centers and constrained by the extent of the annotated epithelium region. Given a training image, the nuclei segmentation is automatically generated by applying a blob detection algorithm (e.g., by the maximally-stable-extremal-regions MSER algorithm) and by selecting as nuclei only the detected blobs that contain an annotated nucleus center. The automatically generated membrane and nuclei segmentations are visually reviewed and manually corrected if necessary. The correction steps involve one of the following methods: rejecting incorrectly segmented membranes or nuclei, explicitly accepting correctly annotated membranes or nuclei, or refining the shapes of the membranes or nuclei. For each image with annotated membranes or nuclei, an annotation layer is created. In one embodiment, each pixel of the annotation layer is assigned a "1" if it belongs to the annotated object (membrane or nucleus); otherwise it is assigned a "0". In another embodiment, the pixels of the annotation layer represent the distance to the nearest annotated object. The network weights are adjusted by an optimization algorithm so that the generated posterior layers are most similar to the automatically generated membrane and nuclei annotation layers.

FIG. 16 illustrates step 3 in which individual cancer cell objects are detected that each include a cell membrane and a cell cytoplasm. A heuristic image analysis process uses watershed segmentation to segment the cell nuclei using the nucleus posterior layer generated by the convolutional neural network. The segmentation generates nucleus objects. Each nucleus object is assigned a unique identifier (UID). The individually identified nuclei are shown as dark objects in FIG. 16 (lower-right image). The detected nuclei are also displayed as overlays in the input image 8 (upper-left image) and in the posterior layers for nuclei (upper-right image) and membranes (lower-left image).

In one embodiment, the watershed segmentation involves a thresholding of the nucleus posterior layer with a predefined first size threshold. All single connected pixels that are above a first size threshold are considered to belong to a nucleus object. Nucleus objects with an area smaller than 16 um^2 are discarded. A UID is assigned to each nucleus object. In a subsequent step, the nucleus objects are grown towards smaller nucleus posteriors in which the added nucleus posterior pixels must be greater than a second predefined threshold.

FIG. 17 illustrates further segmentation steps in which nucleus objects are used to detect and improve the segmentation of the membranes. A region grow algorithm uses the detected nucleus objects as seeds to grow to the ridge of the membrane (approximate cell border) in the membrane posterior layer. Detected membranes (lower-right image) are shown as overlays in the input image 8 (upper-left image) and in the posterior layers for nuclei (upper-right image) and membranes (lower-left image).

FIG. 18 illustrates the detection and segmentation of membrane objects which are segmented by growing the region of the border pixels of detected cells outwards to the membrane probability layer and to a predefined membrane layer posterior threshold value. The thicker border regions become the membrane objects. Each membrane object is assigned the same UID as that of the associated the nucleus object.

The space between the membrane and the nucleus is assigned to the cytoplasm using the UID of the nucleus. For each membrane (FIG. 18 see top-left image) and cytoplasm (FIG. 18 see top-left image), the average optical densities of the DAB staining is exported to a file on a hard drive together with the UIDs. For each cell (defined as in including a nucleus, cytoplasm and membrane), the position of the center of gravity (x,y) of the cell within the slide is also exported. The file may reside on a hard disk, a solid state disk or a portion of dedicated RAM in a computer system.

FIG. 19 illustrates the results of the image analysis in an image analysis software environment. FIG. 19 (upper-left image) shows the segmentation of nucleus objects and membrane objects as an overlay on a digital image of stained tissue. FIG. 19 (lower-left image) shows the segmentation of a nucleus object as an overlay on an optical density

representation of the digital image. Dark optical density pixels are associated with a high amount of DAB, and bright optical density pixels are associated with a low amount of DAB. The DAB optical density of each image pixel is computed from the red-green-blue representation of the image pixel by transformation of the red-green-blue color space so that the brown DAB component becomes an independent color, and by taking the logarithm of that brown color component. FIG. 19 (upper-right image) and FIG. 20 (top) show the image analysis script used to generate the segmented image within a Definiens Developer XD platform. FIG. 19 (lower-right image) and FIG. 20 (bottom) shows the exported measurements for all cell membrane objects and cytoplasm objects in image 8.

**B. Calculation of predictive ADC score.**

Based on the optical density of the DAB staining within the membrane objects and optionally the cytoplasm objects, a single-cell ADC score is computed for each cancer cell in the digital image 8. The single-cell ADC score is also optionally based on the staining intensities of the DAB dye in the membrane objects and cytoplasm objects of neighboring cancer cells that are closer than a predefined distance to the cancer cell for which the single-cell ADC score is being computed. The aggregate of the single-cell ADC scores is predictive of a response of the cancer patient to an anti-B7H4 ADC therapy.

FIG. 21 illustrates exemplary quantitative results of the optical density of staining from the image analysis of steps 2-3 in a schematic drawing using gray values of membrane and cytoplasm pixels. The steps of heuristic image analysis illustrated in FIGS. 15-20 are used to obtain the example segmentation of FIG. 21 into cell nuclei, cell membranes and cell cytoplasm. Bright gray values in FIG. 21 are associated with high DAB optical density, and therefore with a high amount of proteins targeted by the diagnostic antibody. Dark gray values are associated with a low DAB optical density. Brighter pixels correspond to a higher DAB optical density.

FIG. 22 lists the exemplary quantitative amounts of staining on the membranes and in the cytoplasms of the image of FIG. 21, which is reproduced in part in FIG. 22. The optical density of the brown DAB signal from the membranes of the first, second and third cells is 0.949, 0.369 and 0.498, respectively. The optical density of the brown DAB signal from the cytoplasms of the first, second and third cells is 0.796, 0.533 and 0.369, respectively. In the schematic image of FIG. 22, the first cancer cell 9 expresses a high amount of the target protein B7-H4 and would be very likely to be killed by the ADC payload entering the cell linked to the ADC antibody (effect 1 in FIG. 23). The second cancer cell 10 and third cancer cell 11 do not express sufficient amounts of the target protein B7-H4 to be killed directly by the anti-B7H4 ADC. However, due to the vicinity of the second cancer cell 10 to the first cancer cell 9, the toxic payload released from the first cancer cell would also kill the second cancer cell 10 (effect 3 in FIG. 23). The third cancer cell 11 would remain active and could be the origin of a drug resistance mechanism, which could eventually cause the death of the patient.

FIG. 23 illustrates the mechanism by which an anti-B7H4 ADC therapy kills cancer cells. In a first step, the ADC antibody binds to the target protein B7-H4 and inhibits the natural function of the target protein, which may lead to cell death. In a second step, the payload (e.g., a type I topoisomerase inhibitor) is internalized into the cell and kills the cell by the toxicity of the payload. This uptake of the payload depends on the amount of target protein on the membrane, and on the difference in the amount of target protein on the membrane and in the cytoplasm. After uptake, the payload can be released from the cell into the surrounding tissue. In a third step, the payload may enter nearby cells and may kill them as well. The spatial distribution of the payload in the tissue is spread by passive diffusion.

Traditional IHC scoring reflects both the effect of inhibition of the target protein due to ADC binding, as well as the effect of the cytotoxic payload entering a cancer cell together with the ADC antibody. Thus, the traditional scoring for ADC therapies does not reflect the importance of the presence of the target protein in the cytoplasm and the effect of the cytotoxic payload that diffuses into the tissue after being released from the first killed cancer cell. In comparison, the novel predictive ADC Score measures the effect of the release of the cytotoxic payload on neighboring cancer cells.

In step 4 of the method of FIG. 14, a single-cell ADC score is determined for each cancer cell. FIG. 24 illustrates the calculation of the single-cell ADC score for each of the three cells shown in FIG. 22 and incorporates an exponential weighting factor based on cell separation to reflect the uptake of the ADC payload into neighboring cells. The single-cell score can be calculated based on the formula shown in FIG. 25 or the formula recited in claim 4. The optical densities listed in FIG. 22 for the DAB signal from the cell membranes (0.949, 0.369 and 0.498) and from the cytoplasms (0.796, 0.533 and 0.369) are inputs into the calculation illustrated in FIG. 24. The first, second and third cells 9-11 have single-cell scores of 0.145, 0.012 and 0.064, respectively.

Thus, the single-cell ADC score incorporates the measurement of the amount of target protein on the cell membrane using the DAB optical density and optionally an estimation of the amount of ADC payload uptake. As shown in FIG. 23, the uptake of the ADC payload for a first cell depends on both the amount of dye in its membrane and in its cytoplasm, as well as on the amount of dye in the membrane and the cytoplasm of a second cell in the vicinity of the first cell. More specifically, the vicinity may be a circular disk with a predefined radius around the first cancer cell. In one embodiment, the single-cell ADC score for the first cancer cell is determined by a distance-weighted sum of the several powers of DAB optical densities of membrane and cytoplasm objects whose associated cancer cell centers are closer to

the first cancer cell center than a predefined distance. In one embodiment, the predefined distance is 50um, as used in the calculation of FIG. 24. In another embodiment, the distance is 20um. In yet another embodiment, the distance weighting involves computing the exponential of the scaled negative Euclidean distance from the first cancer cell center to the other cancer cell centers in the sum. In another embodiment, the powers in the sum are restricted to 0, 1, and 2 (constants, linear terms, squares).

FIG. 25 shows one embodiment of a formula for calculating the single-cell ADC score. The functions $a_{kl}$ in the formula depend on the distance $|r_j - r_i|$ from the cell j to the cell i. $ODM_j$ is the DAB optical density of the membrane of cell j, and $ODC_j$ is the DAB optical density of the cytoplasm of cell j. The constants A_ij , r_norm and d are the same for all types of cancer. However, the threshold for the score to determine whether the patient is eligible for the ADC therapy is not the same for different types of cancer.

In step 5 of the method for indicating how a particular cancer patient will respond to an ADC therapy, a response score is computed for the digital image 8 of tissue from the cancer patient based on the staining of the target protein B7-H4 such that the score indicates how the cancer patient will respond to the anti-B7H4 ADC therapy. The response score is generated by aggregating all single-cell ADC scores of the tissue sample using a statistical operation. Thus, the score is computed based on a statistic of the single-cell ADC scores for all cancer cells detected in the digital image. In one embodiment, the statistic is a predefined quantile of the ADC payload uptake estimates of all cancer cells the image 8.

In another embodiment, the formula for calculating the single-cell ADC score takes into account only the optical density of the stained membrane of each cancer cell and does not consider either the staining of the cytoplasm or whether other cancer cells are located within a predetermined distance of the cancer cell for which the single-cell ADC score is being calculated.

In step 6 of the method, the anti-B7H4 ADC therapy is recommended to the cancer patient if the response score is larger than a predetermined threshold. The predetermined threshold in step 6 and the quantile in step 5 are determined by optimizing the positive predictive value, the negative predictive value, and the prevalence of a positive recommendation using a cohort of patients with known single-cell ADC scores and therapy response parameters.

In another example of the computation of the response score in step 5, a digital images of a tissue sample of a cancer patient is acquired and stained with a diagnostic antibody (dAB). Image analysis is performed on the digital images to detect N cancer cells $(c_i)_{0 \le i < N}$. The optical density in the segmented membrane of cell $c_i$ is denoted as $OD_M^i$ , and the optical density in the segmented cytoplasm is denoted as $OD_C^i$ . The response score $S^S$ associated with digital images indicates the likelihood that the cancer patient will respond to the anti-B7H4 ADC therapy. The ADC binds specifically to the same B7-H4 protein as the diagnostic antibody. The response score $S^S$ can be expressed as:

[Formula 41]

$$S^s = \Lambda_{0 \le i < N}[\, p(c_i)\, ]$$

where $\Lambda$ is a aggregation operator over a set of cells detected in the digital images. The aggregation operation is a single number representative of the positivity values $p(c_i)$ associated with the detected cells. In one example, the aggregation operation is the arithmetic mean. In another example, the aggregation operation is one of several kinds of averages, such as a harmonic mean or a geometric mean. In another example, the aggregation operation is the frequency of a sub-group of detected cells. In another example, the aggregation operation is a sum. In one embodiment, the aggregation operation is one of several kinds of averages of a subset of cells. In yet another example, the aggregation operation is a quantile.

The term p() denotes a positivity function that represents for each cell its probability of responding to the anti-B7H4 ADC therapy. The positivity function for a cell is positive for an aggregation of dAB positivity values associated with the cell's neighboring cells. The positivity value $p(c_i)$ associated with a cell $c_i$ is expressed as:

[Formula 42]

$$p(c_i) = \mathrm{V}_{(c_j)_{j \in \pi_i}}\left[\, p^{dAB}(c_j)\right]$$

where $\pi_i$ is the set of neighboring cancer cells in the proximity of the cell $c_i$, which includes cell $c_i$, defined as the cancer cells whose distance to the cell $c_i$ is smaller than a predefined constant. In one example, the distance between $c_i$ and $c_j$ is the Euclidean distance between the two cell centers. In another example, the distance is the minimal distance between the

membranes of $c_i$ and $c_j$. In Formula 42, $\boldsymbol{p^{dAB}(c_j)}$ is a positivity value associated with cell $c_j$ based on its optical density in the membrane and its optical density in its cytoplasm. In one example, $p^{dAB}(c_j)$ is defined as the function that associates 1 to cells for which

[Formula 43]

$$\alpha_1\left(OD_M^j\right)^{\gamma_1} + \alpha_2\left(OD_C^j\right)^{\gamma_2} + \alpha_3\left(h\left(OD_M^j, OD_C^j\right)\right)^{\gamma_3} > T_p$$

and 0 otherwise. In Formula 43, $\alpha_1, \alpha_2, \alpha_3, \gamma_1, \gamma_2, \gamma_3$ and $T_p$ are predefined constants, and $h(\ )$ is a function measuring the difference between $OD_M^j$ and $OD_C^j$. The predefined constants are determined by statistical analysis of a cohort of cancer patients with known treatment response to the anti-B7H4 ADC therapy. In another example, $p^{dAB}(c_j)$ is expressed as:

[Formula 44]

$$p^{dAB}(c_j) = \alpha_1\left(OD_M^j\right)^{\gamma_1} + \alpha_2\left(OD_C^j\right)^{\gamma_2} + \alpha_3\left(h\left(OD_M^j, OD_C^j\right)\right)^{\gamma_3}$$

In one example, the function $h(\ )$ is equal to the difference between $OD_M^j$ and $OD_C^j$, expressed as $h\left(OD_M^j, OD_C^j\right) = OD_M^j \times \left(OD_M^j - OD_C^j\right)$. In another example, the function $h(\ )$ considers only the positive difference between $OD_M^j$ and $OD_C^j$, expressed as $h\left(OD_M^j, OD_C^j\right) = OD_M^j \times \max\left(0, OD_M^j - OD_C^j\right)$. In yet another example, the function $h(\ )$ is equal to the contrast between $OD_C^j$ and $OD_M^j$, expressed as $h\left(OD_M^j, OD_C^j\right) = OD_M^j \times \max\left(0, \frac{OD_M^j - OD_C^j}{OD_M^j + OD_C^j}\right)$.

In Formula 42, V is a aggregation operator over the set of neighboring cancer cells. The aggregation operation is a single number representative of the dAB positivity values associated with the neighboring cells. In one example, the aggregation operation is the weighted arithmetic mean of the dAB positivity, expressed as:

[Formula 45]

$$\mathrm{V}_{(c_j)_{j\in\pi_i}}\left[p^{dAB}(c_j)\right] = \frac{\sum_{(c_j)_{j\in\pi_i}} w_{c_i}(c_j)\left[p^{dAB}(c_j)\right]}{\sum_{(c_j)_{j\in\pi_i}} w_{c_i}(c_j)}$$

where $w_{c_i}(c_j)$ denotes a weighting function that computes the likelihood of the cell $c_i$ being influenced by the neighboring cell $c_j$. In another example, the aggregation operation associates 1 to a cell $c_i$ if the sum of the dAB positivity values of its neighboring cells is above an arbitrary real constant $T_n$, and otherwise 0. In another example, the aggregation operation is a quantile, a frequency measure, or one of several kinds of averages, such as a harmonic mean or a geometric mean. In one example, the weighting function $w_{c_i}(c_j)$ is a Gaussian function of a spatial distance $d(c_i, c_j)$ between the cell $c_i$ and the cell $c_j$, expressed as:

[Formula 46]

$$w_{c_i}(c_j) = exp\left[-\frac{d(c_i,c_j)^{\alpha}}{\sigma^{\alpha}}\right]$$

where $\sigma$ and $\alpha$ are predefined constants. In another example, the weighting function is a sigmoid of the spatial distance:

[Formula 47]

$$w_{c_i}(c_j) = 1 - \frac{1}{1+exp\left[-\frac{(d(c_i,c_j)-\beta)^{\alpha}}{\sigma^{\alpha}}\right]}$$

where $\sigma$, $\alpha$ and $\beta$ are predefined constants. In another example, the weighting function is a decreasing function of the Euclidean distance between the cell $c_i$ and the cell $c_j$. In yet another example, the weighting function is a Heaviside function of the distance between the cell $c_i$ and the cell $c_j$ that associates 1 if the distance is smaller than an predefined constant $T_d$, and otherwise 0.

One particular example of the positivity function $p(c_i)$ associates 1 to the cell $c_i$ for which

[Formula 48]

$$\left(\sum_{(c_j)_{j\in\pi_i}}\left[OD_M^j > T_p\right]\right) > T_n$$

and otherwise 0.

C. Validation of method of prediction.

The accuracy of the novel predictive ADC score generated according to the method of FIG. 14 was validated based on a patient-derived xenograft (PDX) study of 15 patients with varying B7-H4 expression levels as indicated by a manual "H Score". Briefly, tumor tissue fragments were implanted subcutaneously into female athymic nude mice between 6 to 8 weeks of age. When tumors reached the appropriate tumor volume range (typically 150-300 mm), animals were randomized into treatment and control groups and dosing with the B7H4-SG3932 ADC was initiated. Tumor-bearing mice were administered a single dose of B7H4-SG3932 ADC via intravenous injection. Animals were observed daily, and tumor dimensions and body weight were measured and recorded twice weekly. Tumor volumes were measured by digital caliper and the volumes of tumors were calculated using the following formula: tumor volume = [length (mm) x width (mm)2 x 0.52, where the length and width are the longest and shortest diameters of the tumor, respectively.

FIG. 26 shows the correlation between actual outcome of the 15 patients in the PDX Breast study and the manual H Score listed by response category. The response categories are PD for progressive disease, SD for stable disease and R for response. The tissue analyzed in FIG. 26 was that of mice bearing tumor cells from 15 human cancer patients.

FIGS. 27-28 show a correlation of the results of a proportion score between a manually assessed H Score and the QCS Score. FIG. 27 compares a manual proportion score to the mean H Score. FIG. 28 compares a manual proportion score to the median QCS score (q50) for optical density of B7H4 membrane staining. For the proportion score, there is a strong correlation between the QCS score (q50) and the manually assessed H Score.

FIGS. 29-30 show a correlation of the results of an intensity score between a manually assessed H Score and the QCS Score. FIG. 29 compares a manual intensity score to the mean H Score. FIG. 30 compares a manual intensity score to the median QCS score for optical density of B7H4 membrane staining. For the intensity score, there is a weaker correlation between the QCS score and the manually assessed H Score.

FIGS. 31-32 illustrate the correlation of the actual response category of each patient as a function of the mean H Score compared to the actual response category of the patient as a function of the median QCS Score (q50). FIG. 31 shows the mean H Score of patients in each of the three response categories PD (progressive disease), R (response) and SD (stable disease). FIG. 32 shows the median QCS score (q50) of patients in each of the three response categories PD, R and SD. While the mean H Score was a less optimal differentiator of patients in the PD and R response categories, lower

QCS Scores were associated with progressive disease, and higher QCS Scores tended to be associated with a response to the anti-H7B4 ADC.

FIGS. 33-34 show that the differences in levels of B7-H4 at baseline between the PD and R response categories are significant using both the QCS Score and the H Score. FIG. 33 shows that the p-value of the T-test between PD (progressive disease) and R (response) groups of patient samples was 0.019 using the H Score. FIG. 34 shows that the p-value of the T-test between PD and R groups of patient samples was 0.011 using the QCS Score.

FIG. 35 shows the relationship between tumor growth in percent and QCS score (median/50% quantile of the membrane optical density of all tumor cells in the sample). X-axis values greater than 0 denotes that the tumor increased in size during observation, and values less than 0 means that the tumor size shrank during observation.

The QCS q50 score for each patient sample is computed by determining the median membrane optical density of all tumor cells in the B7H4-stained digital tissue image acquired from the patient sample.

FIG. 36 shows the correlation between the QCS Score and tumor growth. There was a negative correlation between the baseline B7-H4 optical density staining levels assessed using the QCS Score and the change in tumor size post treatment. In the graph of FIG. 36, Spearman's rho is -0.65, and p equals 0.011. Thus, there was a larger decrease in tumor size associated with a higher optical density of B7-H4 staining.

**D. Example showing higher QCS scores from diagnostic antibody staining are correlated to decreasing tumor cell density after treatment with ADC.**

An example showing the application of the Quantitative Continuous Score (QCS) involved using the score and associated image analysis to evaluate the pharmacokinetics (PK) and pharmacodynamics (PD) of the anti-H7B4 ADC E02-GL-SG3932. FIG. 37 illustrates how the PK and PD studies were performed. A xenograft mouse model was used to study how the ADC reduced tumor growth of human breast cancer tissue. Human tumor cells from 28 breast cancer patients were implanted into mice and cloned. Image analysis and dyed diagnostic antibodies associated with the antibody of the ADC E02-GL-SG3932 were used to track the growth of the implanted tumor cells over time.

FIGS. 38A-D are graphs that summarize the PK and PD results. Tumor samples were harvested at different times after treatment with a single dose of the ADC and then stained by immunohistochemistry. The deep learning-based QCS algorithm was used to quantify the ADC, and a customized HALO solution determined the prevalence of gH2AX and cleaved caspase 3 in the tumor cells.

FIG. 38A shows the binding and uptake of the ADC in epithelial cells based on the cell ratio of epithelial cells with membrane staining for ADC-IgG (human immunoglobulin G1) over all epithelial cells. Cells were counted as stained only if they exhibited staining above the maximum value observed in the control. FIG. 38A shows that ADC uptake increased over time up to 96 hours after ADC treatment and increased more with higher dosages of the ADC.

FIG. 38B illustrates the increasing DNA damage (gH2AX) over time after ADC treatment. DNA damage is represented by the fraction of epithelial cells found positive for foci in the gH2AX assay. The slides were analyzed with a commercially available image analysis system Halo (Indica Labs, Albuquerque, USA) to determine the prevalence of yH2AX. FIG. 38B shows that DNA damage peaked at about 168 hours after ADC treatment.

FIG. 38C illustrates the increasing DNA damage over time after ADC treatment based on the percent of cleaved caspase-3 positive tumor cells. The slides were analyzed using the HALO image analysis system.

FIG. 38D shows increasing tumor cell death over time after ADC treatment based on decreasing cell density of all epithelial cells. Treatment with E02-GL-SG3932 results in a decrease in cell number, indicating cell death, compared to treatment with an isotype ADC.

Before image analysis was performed on stained tissue, a diagnostic antibody closely associated with the antibody of the ADC E02-GL-SG3932 was first selected. The diagnostic antibody was then used for staining. First, the xenograft model was used to confirm that membrane staining of tumor cells by a dye linked to three diagnostic antibodies, D11, A57.1 and Cal63, was highly correlated among the three. FIG. 39A shows the median optical density of membrane staining of tumor tissue from the 28 breast cancer patients that were cloned by implanting into mice. The highly correlated degree of staining of the 28 distinct tumor samples resulting from the three diagnostic antibodies demonstrates that the degree of tumor progression was consistently measured by the diagnostic antibodies. In only two of the 28 tumor samples did one of the diagnostic antibodies produce a staining level that varied significantly from that of the other two diagnostic antibodies.

FIG. 39B shows that for tumor clone sample #21, membrane staining by the diagnostic antibody D11 was far less than the staining by the diagnostic antibodies A57.1 and Cal63. FIG. 39C shows that for tumor sample #28, membrane staining by the diagnostic antibody A57.1 was significantly greater than the staining by the diagnostic antibodies D11 and Cal63. Thus, the diagnostic antibody Cal63 provided the most consistent membrane staining of the tumor samples and was used for the PK and PD studies.

FIGS. 40A-B show digital images of clone samples #26 and #4 stained using the diagnostic antibody Cal63 linked to a dye. Using image analysis and the QCS method, a single-cell score based on membrane staining was determined for

each tumor cell in the digital image. Then the distribution of similarly stained cells was determined. The QCS score was used to study the pharmacokinetics (PK) and pharmacodynamics (PD) of the ADC E02-GL-SG3932.

FIGS. 41A-B show the distribution of membrane optical density of samples stained using the anti-B7H4 diagnostic antibody Cal63 after treatment with various dosages of E02-GL-SG3932 or the ADC without a cytotoxic payload. The graphs of FIGS. 41A-B show that ADC treatment increased the target expression level and was independent of ADC concentration at the tested levels.

FIG. 41A shows the distribution of per-cell membrane staining (membrane optical density) for clone sample #26 before and after treatment with various concentrations of the ADC without payload. The upper graph is at time zero, and the lower graph is at 168 hours after treatment. The isotype control has roughly the same staining distribution as the untreated sample at time zero. At 168 hours after treatment, the number of cells with a higher level of membrane staining increased. The increased staining was relatively independent of ADC concentration. The increased staining after ADC treatment demonstrates that the ADC triggered an elevated target level of the B7H4-antibody in epithelial cell membranes.

FIG. 41B shows the distribution of per-cell membrane staining for clone sample #4 before and after treatment with the ADC E02-GL-SG3932. The upper graph is at time zero, and the lower graph is at 168 hours after treatment. The upper graph shows that the median optical density of membrane staining of clone sample #4 at time zero is about 60% that of clone sample #26 in FIG. 41A. At 168 hours after treatment, the number of cells with a higher level of membrane staining increased. Even starting from this lower prevalence of membrane staining of tumor cells, ADC treatment triggered an elevated target level.

FIGS. 42A-B show the per-cell distribution of bound IgG before and after ADC treatment for clone samples #26 and #4. The upper graphs of FIGS. 42A-B show that sample #26 has a higher baseline distribution of bound IgG than does sample #4. The lower graphs of FIGS. 42A-B show the distribution of bound IgG after ADC treatment and demonstrate that the higher baseline target level of sample #26 resulted in higher ADC binding compared to sample #4. Thus, ADC binding to target cells depends on target expression.

FIG. 42C is a graph of the maximum membrane optical density of bound IgG at various dosage levels of E02-GL-SG3932. FIG. 42C shows that with increasing dosage levels of the ADC without payload, the mean value of the distribution of membrane optical density of bound IgG increased.

FIG. 42D shows the percent of cleaved Caspase-3 positive tumor for clone sample #26 at dosage levels of the ADC E02-GL-SG3932 from 1 to 7 mg/kg. FIG. 42D shows that at a dosage level of 7 mg/kg, the percent positive tumor value is higher for clone #26 than for clone #4.

FIGS. 43A-B are graphs showing the effects of target expression on pharmacokinetics (PK) and pharmacodynamics (PD) of E02-GL-SG3932 at various dosages.

FIG. 43A illustrates the membrane binding kinetics of E02-GL-SG3932. FIG. 43A charts the median membrane optical density of IgG for clones #26 and #4 at various times after treatment with various dosages of the ADC without payload. The median membrane optical density increased with time elapsed since treatment and reached a maximum after about 96 hours after treatment.

FIG. 43B illustrates the kinetics of ADC presence in the membrane versus the cytosol. The graph of FIG. 43B shows the median optical density of clone #26 at various times after treatment with 7 mg/kg of E02-GL-SG3932. The optical density is an indication of target expression. The graph of FIG. 43B shows the optical density in the membrane and in the cytosol. After about 24 hours from treatment, the target expression in the membrane increases at a faster rate than does the target expression in the cytosol. Thus, the ADC binding depended on both the dosage and the target expression.

FIGS. 44A-B are graphs showing the effects of target expression on PK and PD of E02-GL-SG3932 at various dosages. FIGS. 44A-B show that the ADC is also effective on lower target expressing cell lines. FIG. 44A relates to clone #26, and FIG. 44B relates to clone #4.

The upper graphs of FIGS. 44A-B show the density of stained epithelial cells at various times after treatment with the ADC without payload. The upper graph of FIG. 44A shows that the epithelial cell density decreased by 51.2% by 288 hours after treatment of clone #26 with 7 mg/kg of the ADC without payload compared to treatment with the isotype control. The upper graph of FIG. 44B shows that the epithelial cell density decreased by 44.7% by 288 hours after treatment of clone #4 with 7 mg/kg of the ADC without payload compared to treatment with the isotype control.

The lower graphs of FIGS. 44A-B illustrate the increasing DNA damage over time after ADC treatment based on the percent of cleaved caspase-3 (CC-3) positive tumor cells. Image analysis of the slides was performed using a customized HALO solution. The lower graph of FIG. 44A shows that the CC-3 positive area of damaged tumor cells of clone #26 increased significantly after 96 hours from treatment. The area of CC-3 positive tissue was higher for higher dosages of the ADC without payload. The lower graph of FIG. 44B shows that the CC-3 positive area of damaged tumor cells of clone #4 increased only moderately starting about after 48 hours from treatment, even at the higher dosage of 7 mg/kg of the ADC without payload. And the increase in CC-3 positive tissue was about the same as the increase for the isotype ADC.

FIGS. 45A-B, 46A-B and 47A-B illustrate the effects of spatial proximity of stained epithelial cells on the pharmacokinetics (PK) of E02-GL-SG3932. With closer spatial proximity of the stained tumor cells, the toxic payload of an ADC that binds to a cancer cell with target protein expression can also kill cells in the proximity that do not express the

target protein. Thus, there is a higher rate of cell death in regions with closer proximity of stained epithelial cells and therefore a higher expression of the target protein.

FIGS. 45A-B show the relationship of binary spatial proximity (SPS at 25um) to membrane optical density at various times after treatment with the ADC up to 312 hours. For clone sample #26, FIG. 45A shows that there is a large linear correlation in the relationship between binary SPS and median membrane optical density as that relationship changes over time. The correlation coefficient r equals 0.914 for the scatter plot of FIG. 45A. FIG. 45B shows that there is an even larger linear correlation for clone #4 in the relationship between binary SPS and membrane optical density as that relationship changes over time. The correlation coefficient r equals 0.925 for the scatter plot of FIG. 45B.

FIGS. 46A-B show the relationship of continuous spatial proximity (cont. SPS at 25um) to membrane optical density at various times after treatment with the ADC. For clone sample #26, FIG. 46A shows that there is a poor linear correlation in the relationship between continuous SPS and median membrane optical density as that relationship changes over time. FIG. 46A actually shows that, for clone #26 with a high membrane optical density of staining, after about 168 hours after treatment the optical density increases as the continuous spatial proximity decreases. This divides the scatter plot into two distinct groups. Thus, for clone #26 with a higher level of target expression, there was an increase in target expression over time after treatment in tumor regions of lower continuous spatial proximity.

However, for clone #4 with a lower level of target expression, FIG. 46B does not show that a second distinct group in the scatter plot forms after a longer period of time has elapsed after treatment. Instead, there remains a moderate linear correlation in the relationship between continuous SPS and membrane optical density as that relationship changes over time. The correlation coefficient r equals 0.617 for the scatter plot of FIG. 46B.

FIGS. 47A-B show the relationship of continuous spatial proximity (cont. SPS at 25um) to epithelial cell density at various times after treatment with the ADC up to 312 hours. For clone sample #26, FIG. 47A shows that there is a moderate linear correlation in the relationship between continuous SPS and epithelial density as that relationship changes over time. The correlation coefficient r equals 0.798 for the scatter plot of FIG. 47A. FIG. 47B shows that there is a poorer linear correlation for clone #4 in the relationship between continuous SPS and epithelial cell density as that relationship changes over time. The correlation coefficient r equals 0.686 for the scatter plot of FIG. 47B. FIGS. 47A-B show that with the elapse of time after ADC treatment, there is a higher rate of cell death (and therefore lower cell density) in regions of closer continuous spatial proximity between the stained tumor cells. The observed higher rate of cell death in regions with closer proximity of stained epithelial cells and therefore a higher expression of the target protein supports the proposed third effect illustrated in FIG. 23 by which non-expressing cancer cells in the vicinity of cells targeted by the ADC are also killed by the toxic payload of the ADC. Moreover, this supports the inclusion of the exponential weighting factor in the formula for the single-cell ADC score shown in FIGS. 24-25 that accounts for cell separation to reflect the uptake of the ADC payload into neighboring cells.

## Claims

**1.** A method of generating a response score to predict a response of a cancer patient to an antibody drug conjugate (ADC) that includes an ADC payload and an ADC antibody that targets a protein on cancer cells, wherein the protein is B7-H4, comprising:

staining a tissue sample immunohistochemically using a dye linked to a diagnostic antibody, wherein the diagnostic antibody is an antibody that binds to B7-H4 on the cancer cells in the tissue sample;
acquiring a digital image of the tissue sample;
detecting cancer cells in the digital image;
computing for each cancer cell a single-cell ADC score, wherein a single-cell ADC score is a score calculated for a cancer cell based on: the staining intensity of the dye in the membrane of the cancer cell, the staining intensity of the dye in the cytoplasm of the cancer cell, the staining intensities of the dye in the membranes of other cancer cells, and the staining intensities of the dye in the cytoplasms of the other cancer cells, wherein the other cancer cells are cancer cells that are closer than a predefined distance to the cancer cell; and
generating the response score by aggregating all single-cell ADC scores of the tissue sample using a statistical operation, wherein the response score is indicative of the likelihood that the cancer patient will respond to the ADC.

**2.** The method of claim 1, wherein the detecting of cancer cells involves detecting for each cancer cell the pixels that belong to the membrane and the pixels that belong to the cytoplasm, and/or wherein the dye is 3,3'-Diaminobenzidine (DAB) and/or wherein the staining intensity of each membrane is computed based on an average optical density of a brown diaminobenzidine (DAB) signal in pixels of the membrane, and wherein the staining intensity of each cytoplasm is computed based on the average optical density of the brown DAB signal in pixels of the cytoplasm.

**3.** The method of claim 1 or claim 2, wherein the single-cell ADC score for a first cancer cell is calculated as a distance-weighted sum of a plurality of powers of the staining intensities of the dye in the membranes of other cancer cells, and the staining intensities of the dye in the cytoplasms of the other cancer cells.

**4.** The method of any one of claims 1-3, wherein the single cell ADC score for a cell i is calculated as:

a sum of all cells j with $|r_j - r_i| < d \{a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|)\}$, wherein $|r_j - r_i|$ is the distance between a cell j and a cell i, $a_{kl}(|r_j - r_i|)$ are functions that depend on the distance $|r_j - r_i|$ of each cell j to each cell i, $ODM_j$ is an optical density of the dye signal in the membrane of cell j, and $ODC_j$ is an optical density of the dye signal in the cytoplasm of cell j.

**5.** The method of claim 4, wherein the functions $a_{kl}$ depend on the distance $|r_j - r_i|$ of the cell j to the cell i in the relation: $a_{kl}(|r_j - r_i|) = A_{kl} \times \exp(-|r_j - r_i| / r_{norm})$ with predefined constant coefficients $A_{oo}$, $A_{1o}$, $A_{o1}$, $A_{11}$, $A_{20}$, $A_{o2}$, and/or wherein $|r_j - r_i|$ is the distance between the center of cell j and the center of cell i.

**6.** The method of claim 5, wherein the coefficients $A_{oo}$, $A_{1o}$, $A_{o1}$, $A_{11}$, $A_{20}$, $A_{o2}$, d and $r_{norm}$ are determined by optimizing the correlation of the response score with a therapy response of a cohort of training patients.

**7.** The method of any one of claims 1-6, wherein the aggregating of all single-cell ADC scores is taken from the group consisting of: determining a mean, determining a median, and determining a quantile with a predefined percentage.

**8.** The method of any one of claims 1-7, wherein the ADC antibody comprises: a HCDR1, a HCDR2, a HCDR3, a LCDR1, a LCDR2, and a LCDR3 comprising the amino acid sequence of SEQ ID NO: 4, SEQ ID NO: 5, SEQ ID NO: 6, SEQ ID NO: 7, SEQ ID NO: 8, and SEQ ID NO: 9, respectively.

**9.** The method of claim 8, wherein the ADC antibody comprises:
a VH chain and a VL chain comprising the amino acid sequence of SEQ ID NO: 10 and SEQ ID NO: 11, respectively.

**10.** The method of claim 9, wherein the ADC antibody comprises:

a heavy chain comprising the amino acid sequence of SEQ ID NO: 12; and
a light chain comprising the amino acid sequence of SEQ ID NO: 13.

**11.** The method of any of claims 1-10, wherein patients having a response score higher than a predetermined threshold are recommended for a therapy involving the ADC.

**12.** The method of any one of claims 1-11, wherein the cancer patient has a cancer selected from the group consisting of: breast cancer, ovarian cancer, endometrial cancer, cholangiocarcinoma, lung cancer, pancreatic cancer, and gastric cancer.

**13.** The method of any one of claims 1-12, wherein the ADC is an anti-B7H4 antibody conjugated to a drug-linker, and wherein the drug-linker is represented by the following formula:

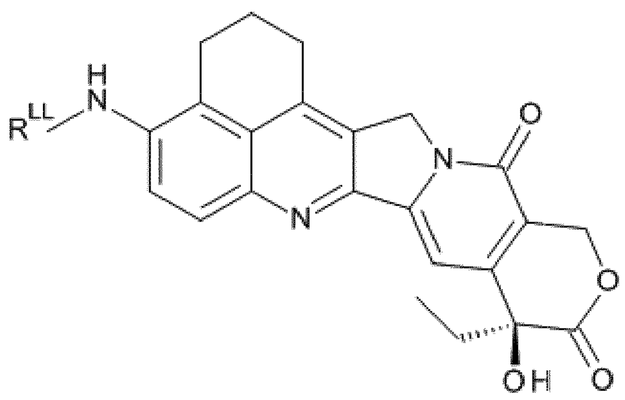

,

and wherein $R^{LL}$ represents a linker connected to the anti-B7H4 antibody.

**14.** The method of any one of claims 1-13, wherein the payload is a cytotoxin and/or wherein the ADC is an anti-B7H4 antibody conjugated to a topoisomerase inhibitor, wherein the topoisomerase inhibitor is selected from:

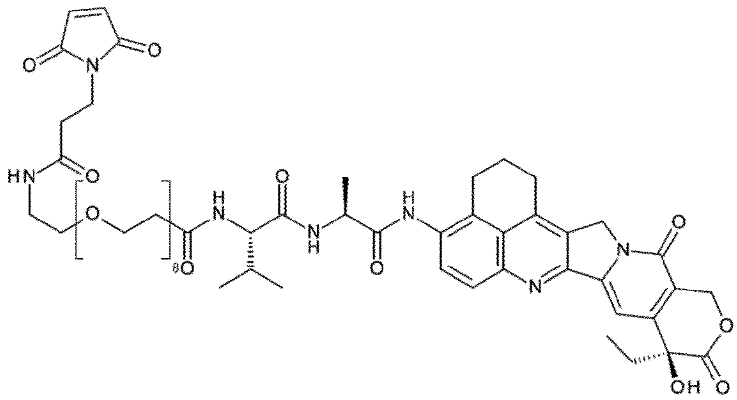
(SG3932);

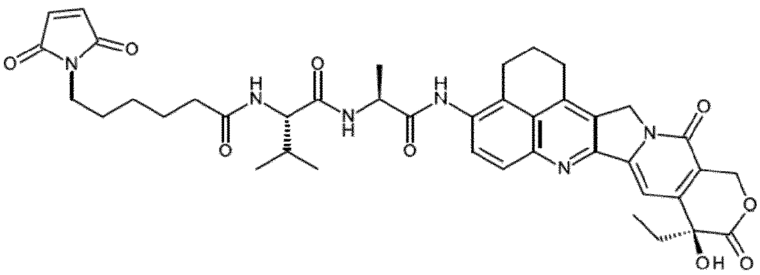
(SG4010);

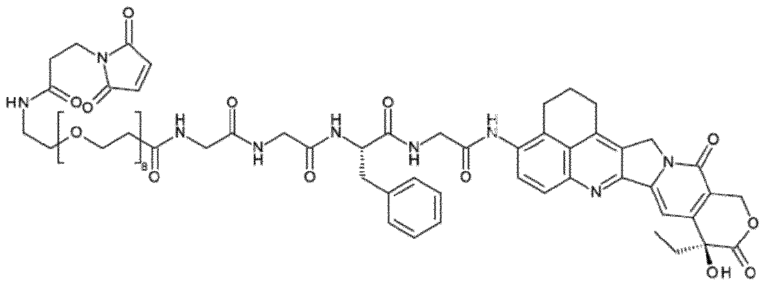
(SG4057);

and/or

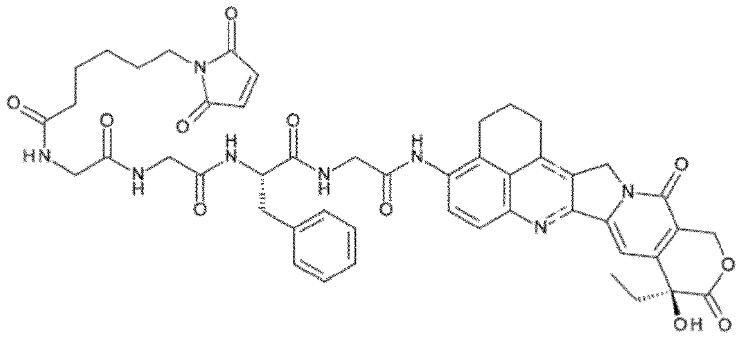
(SG4052).

**15.** The method any one of claims 1-14, wherein the ADC is an anti-B7H4 antibody conjugated to a topoisomerase inhibitor, wherein the topoisomerase inhibitor is

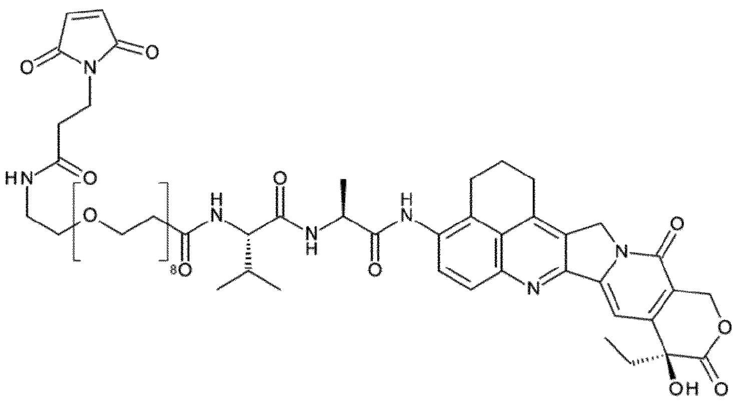
(SG3932).

## Patentansprüche

1. Verfahren zum Generieren eines Ansprechwerts zur Vorhersage des Ansprechens eines Krebspatienten auf ein Antikörper-Arzneimittel-Konjugat (ADC), das eine ADC-Payload und einen ADC-Antikörper umfasst, der auf ein Protein auf Krebszellen abzielt, wobei das Protein B7-H4 ist, umfassend:

   immunhistochemisches Färben einer Gewebeprobe unter Verwendung eines Farbstoffs, der an einen diagnostischen Antikörper gebunden ist, wobei der diagnostische Antikörper ein Antikörper ist, der an B7-H4 auf den Krebszellen in der Gewebeprobe bindet;
   Erfassen eines digitalen Bildes der Gewebeprobe;
   Detektieren von Krebszellen im digitalen Bild;
   Berechnen eines Einzelzellen-ADC-Werts für jede Krebszelle, wobei ein Einzelzellen-ADC-Wert ein Wert ist, der für eine Krebszelle basierend auf Folgendem berechnet wird: der Färbungsintensität des Farbstoffs in der Membran der Krebszelle, der Färbungsintensität des Farbstoffs im Zytoplasma der Krebszelle, der Färbungsintensität des Farbstoffs in den Membranen anderer Krebszellen und der Färbungsintensität des Farbstoffs in den Zytoplasmen der anderen Krebszellen, wobei die anderen Krebszellen Krebszellen sind, die näher an der Krebszelle sind als ein vordefinierter Abstand; und
   Generieren des Ansprechwerts durch Aggregieren aller Einzelzellen-ADC-Werte der Gewebeprobe unter Verwendung einer statistischen Operation, wobei der Ansprechwert die Wahrscheinlichkeit angibt, mit der ein Krebspatient auf das ADC anspricht.

2. Verfahren nach Anspruch 1, wobei das Detektieren von Krebszellen das Detektieren der Pixel, die zur Membran gehören, und der Pixel, die zum Zytoplasma gehören, für jede Krebszelle umfasst und/oder wobei der Farbstoff 3,3'-Diaminobenzidin (DAB) ist und/oder wobei die Färbungsintensität jeder Membran basierend auf der durchschnittlichen optischen Dichte eines Signals von braunem Diaminobenzidin (DAB) in Pixeln der Membran berechnet wird und wobei die Färbungsintensität jedes Zytoplasmas basierend auf der durchschnittlichen optischen Dichte eines Signals von braunem DAB in Pixeln des Zytoplasmas berechnet wird.

3. Verfahren nach Anspruch 1 oder Anspruch 2, wobei der Einzelzellen-ADC-Wert für eine erste Krebszelle als abstandgewichtete Summe einer Vielzahl von Stärken der Färbungsintensitäten des Farbstoffs in den Membranen anderer Krebszellen und der Färbungsintensitäten des Farbstoffs in den Zytoplasmen der anderen Krebszellen berechnet wird.

4. Verfahren nach einem der Ansprüche 1 bis 3, wobei der Einzelzellen-ADC-Wert für eine Zelle i wie folgt berechnet wird:

   Summe aller Zellen j mit $|r_j - r_i| < d$ $\{a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|)\}$,
   wobei $|r_j - r_i|$ der Abstand zwischen einer Zelle j und einer Zelle i ist, $a_{kl}(|r_j - r_i|)$ Funktionen sind, die vom Abstand $|r_j - r_i|$ jeder Zelle j von jeder Zelle i abhängen, $ODM_j$ die optische Dichte des Farbstoffsignals in der Membran von Zelle j ist und $ODC_j$ die optische Dichte des Farbstoffsignals im Zytoplasma von Zelle j ist.

**5.** Verfahren nach Anspruch 4, wobei die Funktionen $a_{kl}$ vom Abstand $|r_j - r_i|$ der Zelle j von der Zelle i in der folgenden Beziehung abhängen: $a_{kl}(|r_j - r_i|) = A_{kl} \times \exp(-|r_j - r_i|/r_{norm})$, mit vordefinierten konstanten Koeffizienten $A_{oo}$, $A_{1o}$, $A_{o1}$, $A_{11}$, $A_{20}$, $A_{o2}$, und/oder wobei $|r_j - r_i|$ der Abstand zwischen dem Zentrum von Zelle j und dem Zentrum von Zelle i ist.

**6.** Verfahren nach Anspruch 5, wobei die Koeffizienten $A_{oo}$, $A_{1o}$, $A_{o1}$, $A_{11}$, $A_{20}$, $A_{o2}$, d $r_{norm}$ durch Optimieren der Korrelation des Ansprechwerts mit dem Therapieansprechen einer Kohorte von Trainingspatienten bestimmt werden.

**7.** Verfahren nach einem der Ansprüche 1 bis 6, wobei das Aggregieren aller Einzelzellen-ADC-Werte aus folgender Gruppe genommen wird: Bestimmen eines Mittels, Bestimmen eines Medians und Bestimmen eines Quantils mit einem vordefinierten Prozentsatz.

**8.** Verfahren nach einem der Ansprüche 1 bis 7, wobei der ADC-Antikörper Folgendes umfasst:
eine HCDR1, eine HCDR2, eine HCDR3, eine LCDR1, eine LCDR2 und eine LCDR3, welche die Aminosäuresequenz der SEQ ID NO: 4, SEQ ID NO: 5, SEQ ID NO: 6, SEQ ID NO: 7, SEQ ID NO: 8 bzw. SEQ ID NO: 9 umfassen.

**9.** Verfahren nach Anspruch 8, wobei der ADC-Antikörper Folgendes umfasst:
eine VH-Kette und eine VL-Kette, welche die Aminosäuresequenz der SEQ ID NO: 10 bzw. SEQ ID NO: 11 umfassen.

**10.** Verfahren nach Anspruch 9, wobei der ADC-Antikörper Folgendes umfasst:

eine schwere Kette, welche die Aminosäuresequenz der SEQ ID NO: 12 umfasst, und
eine leichte Kette, welche die Aminosäuresequenz der SEQ ID NO: 13 umfasst.

**11.** Verfahren nach einem der Ansprüche 1 bis 10, wobei Patienten mit einem Ansprechwert höher als eine vorbestimmte Schwelle für eine ADC umfassende Therapie empfohlen werden.

**12.** Verfahren nach einem der Ansprüche 1 bis 11, wobei der Krebspatient einen Krebs aufweist, der aus folgender Gruppe ausgewählt ist: Brustkrebs, Ovarialkrebs, Endometriumkrebs, Cholangiokarzinom, Lungenkrebs, Pankreaskrebs und Magenkrebs.

**13.** Verfahren nach einem der Ansprüche 1 bis 12, wobei der ADC ein Anti-B7H4-Antikörper ist, der an einen Arzneimittel-Linker konjugiert ist, und wobei der Arzneimittel-Linker durch die folgende Formel dargestellt ist:

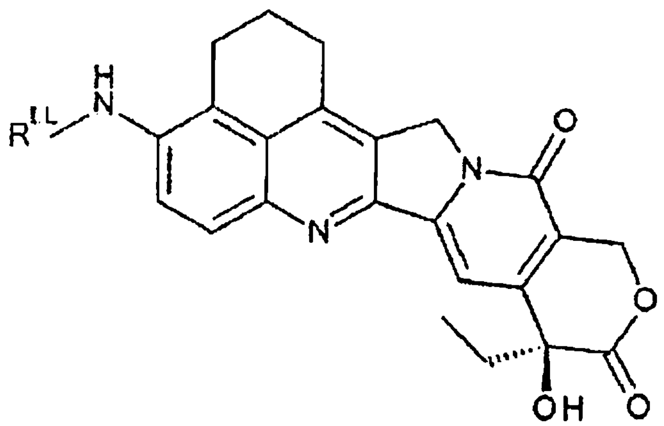

,

und wobei $R^{LL}$ für einen Linker steht, der mit dem Anti-B7H4-Antikörper verbunden ist.

**14.** Verfahren nach einem der Ansprüche 1 bis 13, wobei die Payload ein Zytotoxin ist und/oder wobei der ADC ein Anti-B7H4-Antikörper ist, der an einen Topoisomerase-Inhibitor konjugiert ist, wobei der Topoisomerase-Inhibitor aus folgenden ausgewählt ist:

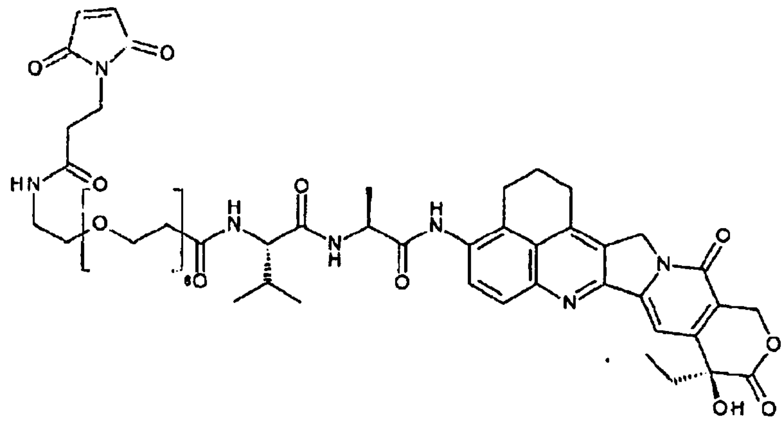

(SG3932);

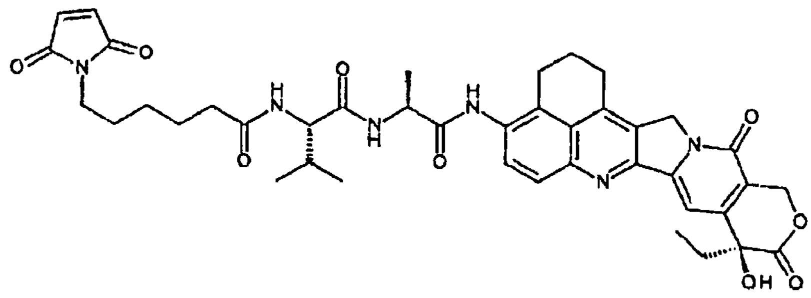

(SG4010);

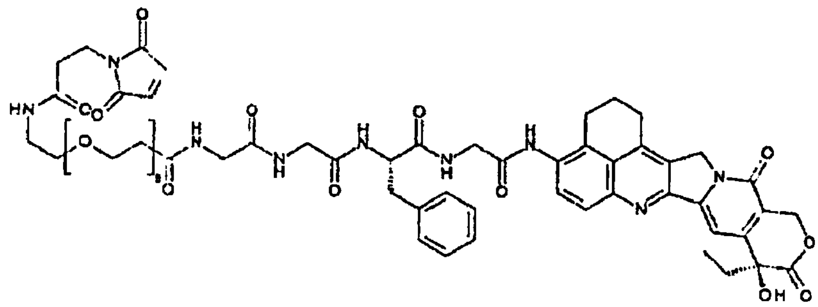

(SG4057)

und/oder

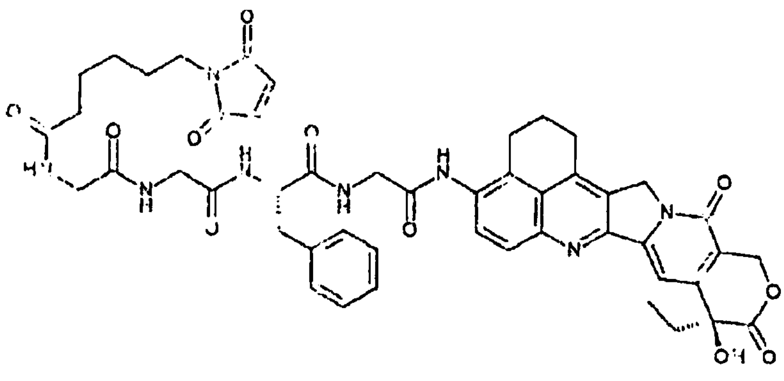

(SG4052).

**15.** Verfahren nach einem der Ansprüche 1 bis 14, wobei der ADC ein Anti-B7H4-Antikörper ist, der an einen Topoisomerase-Inhibitor konjugiert ist, wobei der Topoisomerase-Inhibitor

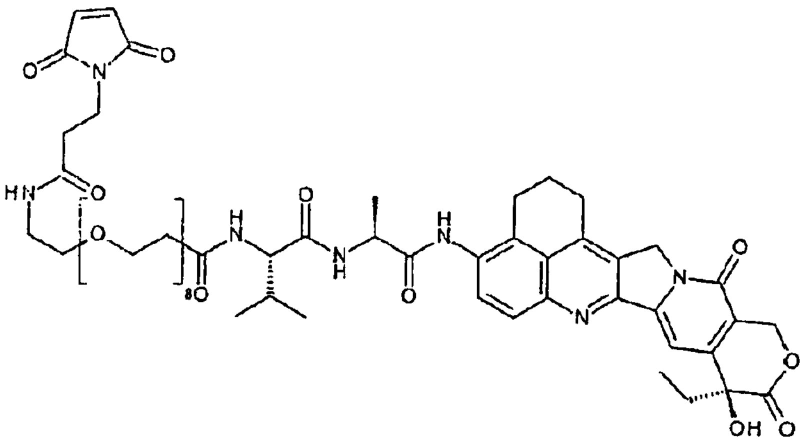

(SG3932)

ist.

## Revendications

1. Procédé pour générer un score de réponse pour prédire une réponse d'un patient atteint de cancer à un conjugué anticorps-médicament (ADC) qui comporte une charge utile ADC et un anticorps ADC qui cible une protéine sur des cellules cancéreuses, dans lequel la protéine est B7-H4, comprenant :

   la coloration immunohistochimique d'un échantillon de tissu à l'aide d'un colorant lié à un anticorps diagnostique, dans lequel l'anticorps diagnostique est un anticorps qui se lie à B7-H4 sur les cellules cancéreuses de l'échantillon de tissu ;
   l'acquisition d'une image numérique de l'échantillon de tissu ;
   la détection de cellules cancéreuses dans l'image numérique ;
   le calcul pour chaque cellule cancéreuse d'un score ADC de cellule unique, dans lequel un score ADC de cellule unique est un score calculé pour une cellule cancéreuse sur la base : de l'intensité de coloration du colorant dans la membrane de la cellule cancéreuse, de l'intensité de coloration du colorant dans le cytoplasme de la cellule cancéreuse, des intensités de coloration du colorant dans les membranes d'autres cellules cancéreuses, et des intensités de coloration du colorant dans les cytoplasmes des autres cellules cancéreuses, dans lequel les autres cellules cancéreuses sont des cellules cancéreuses qui sont plus proches qu'une distance prédéfinie de la cellule cancéreuse ; et
   la génération du score de réponse en agrégeant tous les scores ADC de cellule unique de l'échantillon de tissu à l'aide d'une opération statistique, dans lequel le score de réponse est représentatif de la probabilité que le patient atteint de cancer va répondre à l'ADC.

2. Procédé selon la revendication 1, dans lequel la détection de cellules cancéreuses implique de détecter pour chaque cellule cancéreuse les pixels appartenant à la membrane et les pixels appartenant au cytoplasme, et/ou dans lequel le colorant est une 3,3'-diaminobenzidine (DAB) et/ou dans lequel l'intensité de coloration de chaque membrane est calculée sur la base d'une densité optique moyenne d'un signal de diaminobenzidine brune (DAB) dans des pixels de la membrane, et dans lequel l'intensité de coloration de chaque cytoplasme est calculée sur la base de la densité optique moyenne du signal de DAB brune dans des pixels du cytoplasme.

3. Procédé selon la revendication 1 ou 2, dans lequel le score ADC de cellule unique pour une première cellule cancéreuse est calculé comme une somme pondérée par distance d'une pluralité de puissances des intensités de coloration du colorant dans les membranes d'autres cellules cancéreuses, et des intensités de coloration du colorant dans les cytoplasmes des autres cellules cancéreuses.

4. Procédé selon l'une quelconque des revendications 1 à 3, dans lequel le score ADC de cellule unique pour une cellule i est calculé de la manière suivante :

   une somme de toutes les cellules j avec $|r_j - r_i| < d$ $\{a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|)\}$,

où $|r_j - r_i|$ est la distance entre une cellule j et une cellule i, $a_{kl}(|r_j - r_i|)$ sont des fonctions qui dépendent de la distance $|r_j - r_i|$ entre chaque cellule j et chaque cellule i, $ODM_J$ est une densité optique du signal de colorant dans la membrane de la cellule j, et $ODC_j$ est une densité optique du signal de colorant dans le cytoplasme de la cellule j.

**5.** Procédé selon la revendication 4, dans laquelle les fonctions $a_{kl}$ dépendent de la distance $|r_j - r_i|$ entre la cellule j et la cellule i dans la relation : $a_{kl}(|r_j - r_i|) = A_{kl} \times \exp(-|r_j - r_i|/r_{norm})$ avec des coefficients constants prédéfinis $A_{oo}$ , $A_{1o}$, $A_{o1}$, $A_{11}$, $A_{20}$, $A_{o2}$, et/ou dans lequel $|r_j - r_i|$ est la distance entre le centre de la cellule j et le centre de la cellule i.

**6.** Procédé selon la revendication 5, dans lequel les coefficients $A_{oo}$ , $A_{1o}$, $A_{o1}$, $A_{11}$, $A_{20}$, $A_{o2}$, d et $r_{norm}$ sont déterminés en optimisant la corrélation du score de réponse avec une réponse thérapeutique d'une cohorte de patients en formation.

**7.** Procédé selon l'une quelconque des revendications 1 à 6, dans lequel l'agrégation de tous les scores ADC de cellule unique est choisie parmi le groupe constitué de : la détermination d'une moyenne, la détermination d'une médiane, et la détermination d'un quantile avec un pourcentage prédéfini.

**8.** Procédé selon l'une quelconque des revendications 1 à 7, dans lequel l'anticorps ADC comprend :
un HCDR1, un HCDR2, un HCDR3, un LCDR1, un LCDR2 et un LCDR3 comprenant la séquence d'acides aminés de SEQ ID NO : 4, SEQ ID NO : 5, SEQ ID NO : 6, SEQ ID NO : 7, SEQ ID NO : 8 et SEQ ID NO : 9, respectivement.

**9.** Procédé selon la revendication 8, dans lequel l'anticorps ADC comprend :
une chaîne VH et une chaîne VL comprenant la séquence d'acides aminés de SEQ ID NO :10 et SEQ ID NO : 11, respectivement.

**10.** Procédé selon la revendication 9, dans lequel l'anticorps ADC comprend :

une chaîne lourde comprenant la séquence d'acides aminés de SEQ ID NO :12 ; et
une chaîne légère comprenant la séquence d'acides aminés de SEQ ID NO :13.

**11.** Procédé selon l'une quelconque des revendications 1 à 10, dans lequel des patients ayant un score de réponse supérieur à un seuil prédéterminé sont recommandés pour une thérapie impliquant l'ADC.

**12.** Procédé selon l'une quelconque des revendications 1 à 11, dans lequel le patient atteint de cancer a un cancer sélectionné parmi le groupe consistant en : cancer du sein, cancer de l'ovaire, cancer de l'endomètre, cholangiocarcinome, cancer du poumon, cancer du pancréas et cancer gastrique.

**13.** Procédé selon l'une quelconque des revendications 1 à 12, dans lequel l'ADC est un anticorps anti-B7H4 conjugué à un lieur de médicament, et dans lequel le lieur de médicament est représenté par la formule suivante :

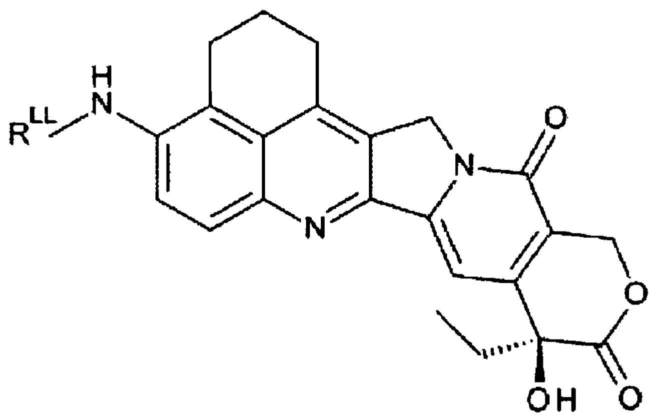

et où $R^{LL}$ représente un lieur connecté à l'anticorps anti-B7H4.

**14.** Procédé selon l'une quelconque des revendications 1 à 13, dans lequel la charge utile est une cytotoxine et/ou dans lequel l'ADC est un anticorps anti-B7H4 conjugué à un inhibiteur de topoisomérase, dans lequel l'inhibiteur de topoisomérase est sélectionné parmi :

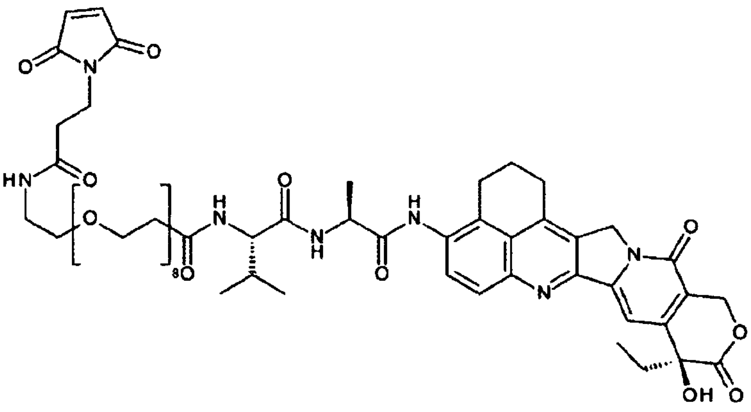

(SG3932);

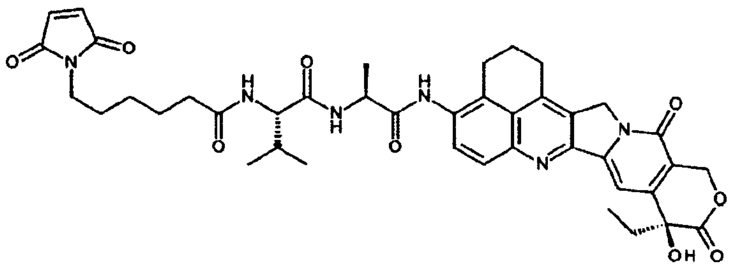

(SG4010);

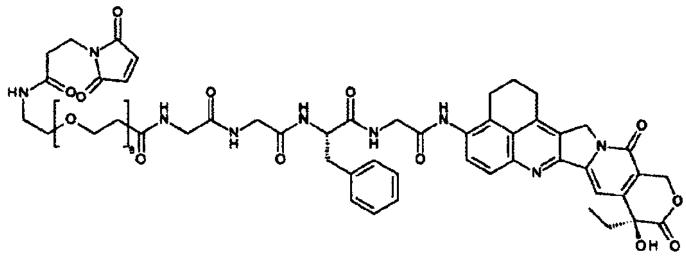

(SG4057);

et/ou

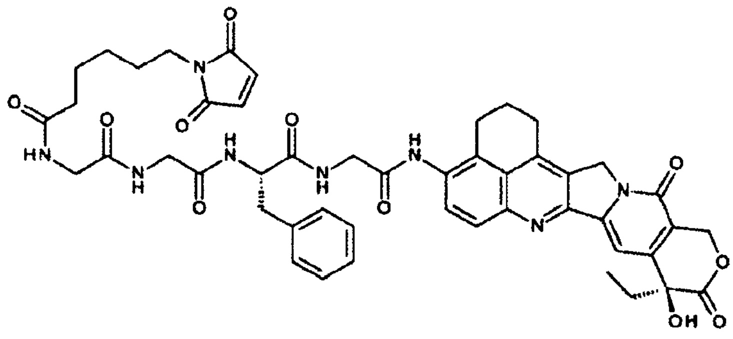

(SG4052).

**15.** Procédé selon l'une quelconque des revendications 1 à 14, dans lequel l'ADC est un anticorps anti-B7H4 conjugué à un inhibiteur de topoisomérase, dans lequel l'inhibiteur de topoisomérase est

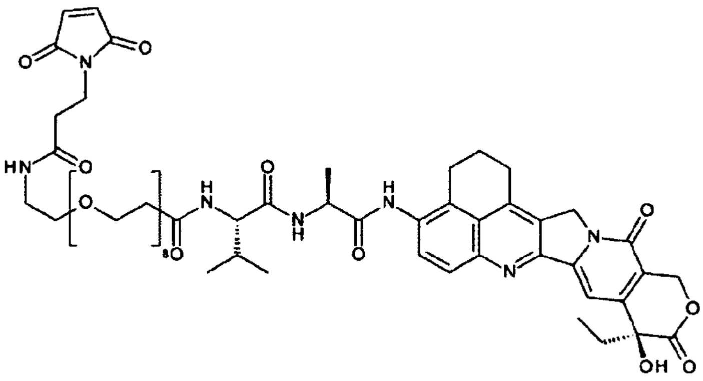
(SG3932).

gccaccatggcttccctggggcagatcctcttctggagcataattagcatcat
cattattctggctggagcaattgcactcatcattggctttggtatttcagggaga
cactccatcacagtcactactgtcgcctcagctgggaacattggggaggat
ggaatcctgagctgcacttttgaacctgacatcaaactttctgatatcgtgata
caatggctgaaggaaggtgttttaggcttggtccatgagttcaaagaaggca
aagatgagctgtcggagcaggatgaaatgttcagaggccggacagcagt
gtttgctgatcaagtgatagttggcaatgcctctttgcggctgaaaaacgtgc
aactcacagatgctggcacctacaaatgttatatcatcacttctaaaggcaa
ggggaatgctaaccttgagtataaaactggagccttcagcatgccggaagt
gaatgtggactataatgccagctcagagaccttgcggtgtgaggctccccg
atggttcccccagcccacagtggtctgggcatcccaagttgaccagggagc
caacttctcggaagtctccaataccagctttgagctgaactctgagaatgtga
ccatgaaggttgtgtctgtgctctacaatgttacgatcaacaacacatactcct
gtatgattgaaaatgacattgccaaagcaacaggggatatcaaagtgaca
gaatcggagatcaaaaggcggagtcacctacagctgctaaactcaaagg
cttctctgtgtgtctcttctttctttgccatcagctgggcacttctgcctctcagccct
tacctgatgctaaaataataa

(SEQ ID No. 1: Human B7-H4 nucleic acid sequence, including 5' and 3' UTR)

FIG. 1

atggcttccctggggcagatcctcttctggagcataattagcatcatcattattc
tggctggagcaattgcactcatcattggctttggtatttcagggagacactcca
tcacagtcactactgtcgcctcagctgggaacattggggaggatggaatcct
gagctgcacttttgaacctgacatcaaactttctgatatcgtgatacaatggct
gaaggaaggtgttttaggcttggtccatgagttcaaagaaggcaaagatga
gctgtcggagcaggatgaaatgttcagaggccggacagcagtgtttgctga
tcaagtgatagttggcaatgcctctttgcggctgaaaaacgtgcaactcaca
gatgctggcacctacaaatgttatatcatcacttctaaaggcaaggggaatg
ctaaccttgagtataaaactggagccttcagcatgccggaagtgaatgtgg
actataatgccagctcagagaccttgcggtgtgaggctccccgatggttccc
ccagcccacagtggtctgggcatcccaagttgaccagggagccaacttctc
ggaagtctccaataccagctttgagctgaactctgagaatgtgaccatgaag
gttgtgtctgtgctctacaatgttacgatcaacaacacatactcctgtatgattg
aaaatgacattgccaaagcaacaggggatatcaaagtgacagaatcgga
gatcaaaaggcggagtcacctacagctgctaaactcaaaggcttctctgtgt
gtctcttctttctttgccatcagctgggcacttctgcctctcagcccttacctgatg
ctaaaa

(SEQ ID No. 2: Human B7-H4 nucleic acid sequence, coding sequence)

FIG. 2

MASLGQILFWSIISIIIILAGAIALIIGFGISGRHSITVTTVA
SAGNIGEDGILSCTFEPDIKLSDIVIQWLKEGVLGLVHE
FKEGKDELSEQDEMFRGRTAVFADQVIVGNASLRLKN
VQLTDAGTYKCYIITSKGKGNANLEYKTGAFSMPEVN
VDYNASSETLRCEAPRWFPQPTVVWASQVDQGANF
SEVSNTSFELNSENVTMKVVSVLYNVTINNTYSCMIEN
DIAKATGDIKVTESEIKRRSHLQLLNSKASLCVSSFFAI
SWALLPLSPYLMLK

(SEQ ID No. 3: Human B7H4 polypeptide sequence; UniProt Accession No.: Q7Z7D3)

FIG. 3

GYYWN

(SEQ ID No. 4: Amino acid sequence of HCDR1 of the anti-B7H4 antibody)

FIG. 4

EINHSGSTSYNPSLKS

(SEQ ID No. 5: Amino acid sequence of HCDR2 of the anti-B7H4 antibody)

FIG. 5

VLYNWNVDS

(SEQ ID No. 6: Amino acid sequence of HCDR3 of the anti-B7H4 antibody)

FIG. 6

RASQDIRNDVG

(SEQ ID No. 7: Amino acid sequence of LCDR1 of the anti-B7H4 antibody)

FIG. 7

AASRLQS

(SEQ ID No. 8: Amino acid sequence of LCDR2 of the anti-B7H4 antibody)

FIG. 8

LQHNSYPRT

(SEQ ID No. 9: Amino acid sequence of LCDR3 of the anti-B7H4 antibody)

FIG. 9

QVQLQQWGAGLLKPSETLSLACTVYGGSFSGYYW
NWIRQPPGKGLEWIGEINHSGSTSYNPSLKSRVTIS
VDTSKNQFSLKLSSVTAADTAVYYCARVLYNWNVD
SWGQGTLVTVSS

(SEQ ID No. 10: Amino acid sequence of the heavy chain variable region of the anti-B7H4 antibody)

FIG. 10

DIQMTQSPSSLSASVGDRVTITCRASQDIRNDVGW
YQQKPGKAPKRLIYAASRLQSGVPSRFSGSGSGTE
FTLTISSLQPEDFATYYCLQHNSYPRTFGQGTKVEIK

(SEQ ID No. 11: Amino acid sequence of the light chain variable region of the anti-B7H4 antibody)

FIG. 11

QVQLQQWGAGLLKPSETLSLACTVYGGSFSGYYW
NWIRQPPGKGLEWIGEINHSGSTSYNPSLKSRVTIS
VDTSKNQFSLKLSSVTAADTAVYYCARVLYNWNVD
SWGQGTLVTVSSASTKGPSVFPLAPSSKSTSGGTA
ALGCLVKDYFPEPVTVSWNSGALTSGVHTFPAVLQ
SSGLYSLSSVVTVPSSSLGTQTYICNVNHKPSNTKV
DKRVEPKSCDKTHTCPPCPAPELLGGPSVFLFPPK
PKDTLMISRTPEVTCVVVDVSHEDPEVKFNWYVDG
VEVHNAKTKPREEQYNSTYRVVSVLTVLHQDWLNG
KEYKCKVSNKALPAPIEKTISKAKGQPREPQVYTLP
PSREEMTKNQVSLTCLVKGFYPSDIAVEWESNGQP
ENNYKTTPPVLDSDGSFFLYSKLTVDKSRWQQGNV
FSCSVMHEALHNHYTQKSLSLSPGK

(SEQ ID No. 12: Amino acid sequence of a heavy chain of the anti-B7H4 antibody)

FIG. 12

DIQMTQSPSSLSASVGDRVTITCRASQDIRNDVGW
YQQKPGKAPKRLIYAASRLQSGVPSRFSGSGSGTE
FTLTISSLQPEDFATYYCLQHNSYPRTFGQGTKVEIK
RTVAAPSVFIFPPSDEQLKSGTASVVCLLNNFYPRE
AKVQWKVDNALQSGNSQESVTEQDSKDSTYSLSS
TLTLSKADYEKHKVYACEVTHQGLSSPVTKSFNRG
EC

(SEQ ID No. 13: Amino acid sequence of a light chain of the anti-B7H4 antibody)

FIG. 13

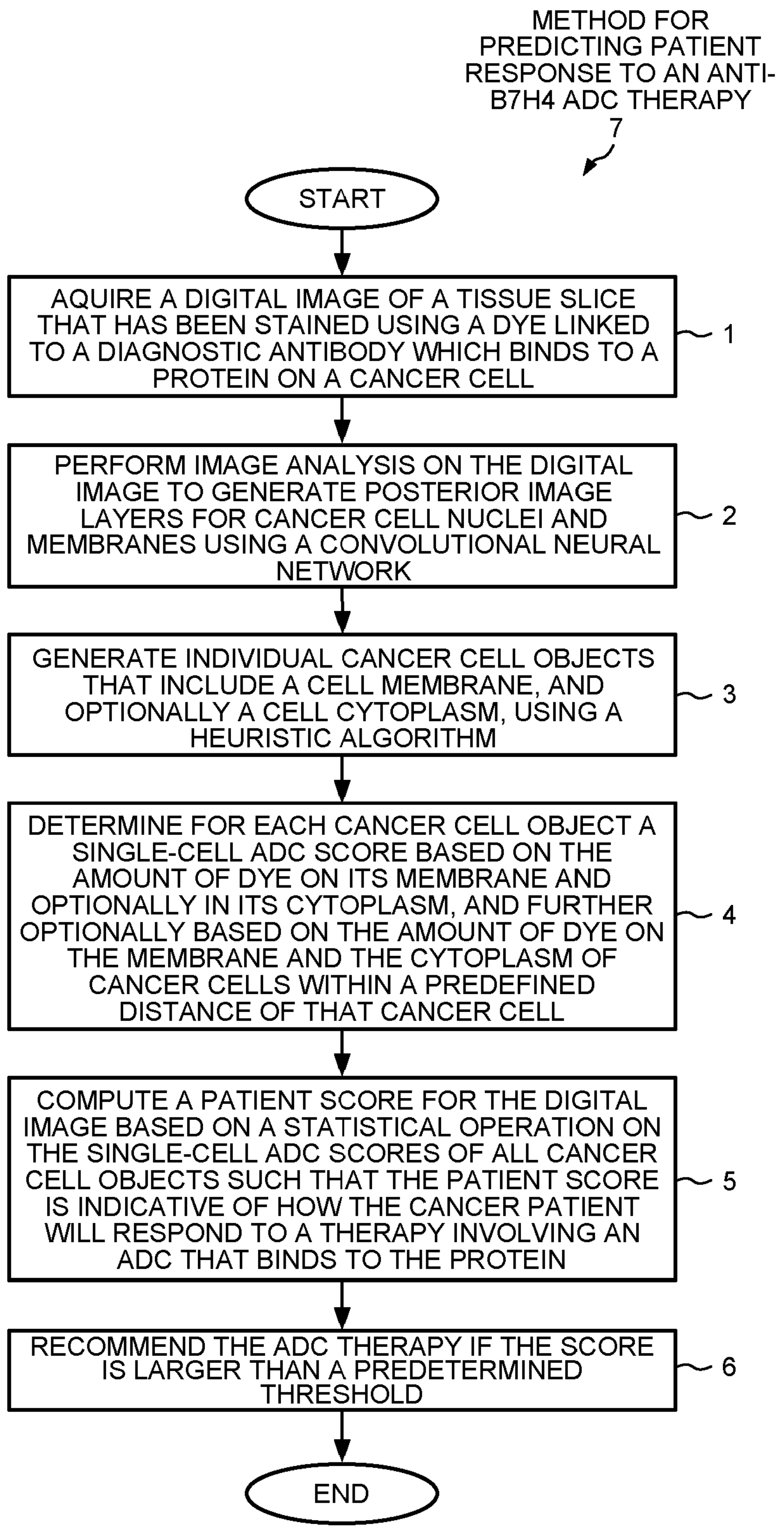
METHOD FOR PREDICTING PATIENT RESPONSE TO AN ANTI-B7H4 ADC THERAPY
7
START
AQUIRE A DIGITAL IMAGE OF A TISSUE SLICE THAT HAS BEEN STAINED USING A DYE LINKED TO A DIAGNOSTIC ANTIBODY WHICH BINDS TO A PROTEIN ON A CANCER CELL
1
PERFORM IMAGE ANALYSIS ON THE DIGITAL IMAGE TO GENERATE POSTERIOR IMAGE LAYERS FOR CANCER CELL NUCLEI AND MEMBRANES USING A CONVOLUTIONAL NEURAL NETWORK
2
GENERATE INDIVIDUAL CANCER CELL OBJECTS THAT INCLUDE A CELL MEMBRANE, AND OPTIONALLY A CELL CYTOPLASM, USING A HEURISTIC ALGORITHM
3
DETERMINE FOR EACH CANCER CELL OBJECT A SINGLE-CELL ADC SCORE BASED ON THE AMOUNT OF DYE ON ITS MEMBRANE AND OPTIONALLY IN ITS CYTOPLASM, AND FURTHER OPTIONALLY BASED ON THE AMOUNT OF DYE ON THE MEMBRANE AND THE CYTOPLASM OF CANCER CELLS WITHIN A PREDEFINED DISTANCE OF THAT CANCER CELL
4
COMPUTE A PATIENT SCORE FOR THE DIGITAL IMAGE BASED ON A STATISTICAL OPERATION ON THE SINGLE-CELL ADC SCORES OF ALL CANCER CELL OBJECTS SUCH THAT THE PATIENT SCORE IS INDICATIVE OF HOW THE CANCER PATIENT WILL RESPOND TO A THERAPY INVOLVING AN ADC THAT BINDS TO THE PROTEIN
5
RECOMMEND THE ADC THERAPY IF THE SCORE IS LARGER THAN A PREDETERMINED THRESHOLD
6
END

FIG. 14

Digital image of a B7-H4 stained tissue section

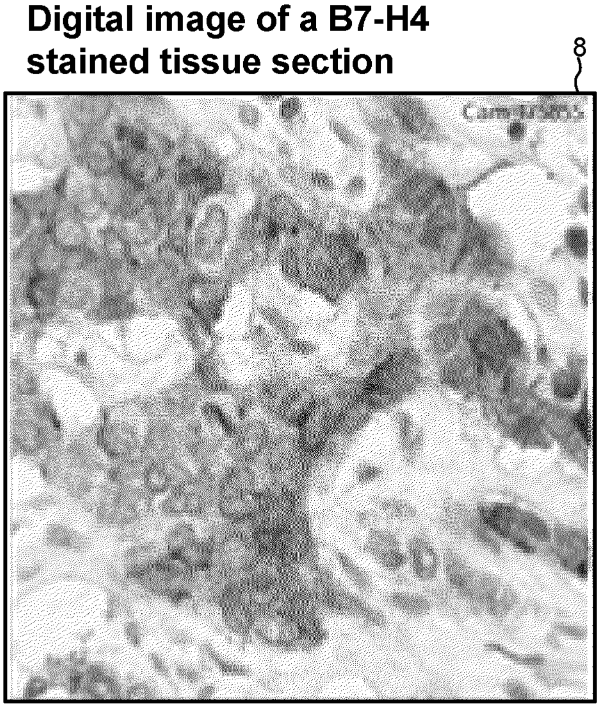


Posterior layer for cell nuclei, generated by a CNN

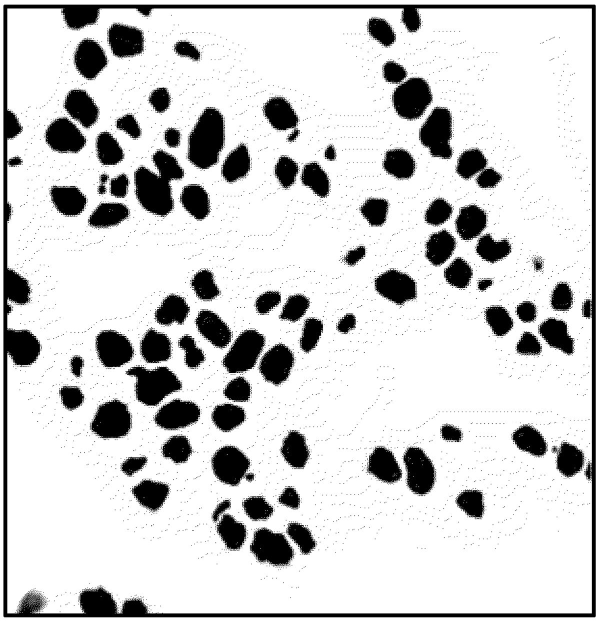

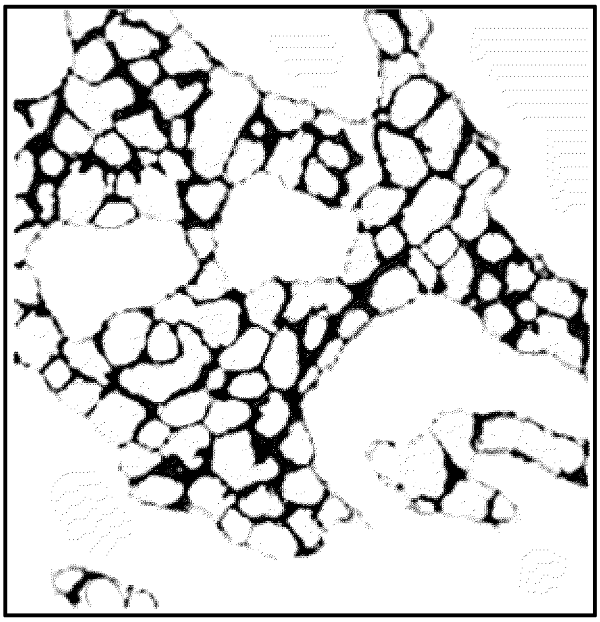

Posterior layer for cell membranes generated by the CNN

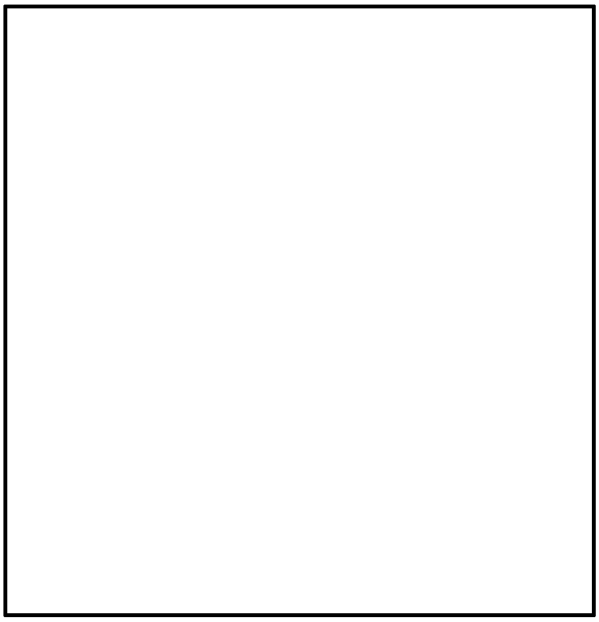

Unique Identifiers for cells, initially empty

In the images, dark corresponds to high probability, and white corresponds to low probability

FIG. 15

Nucleus segmentation results after thresholding and region grow

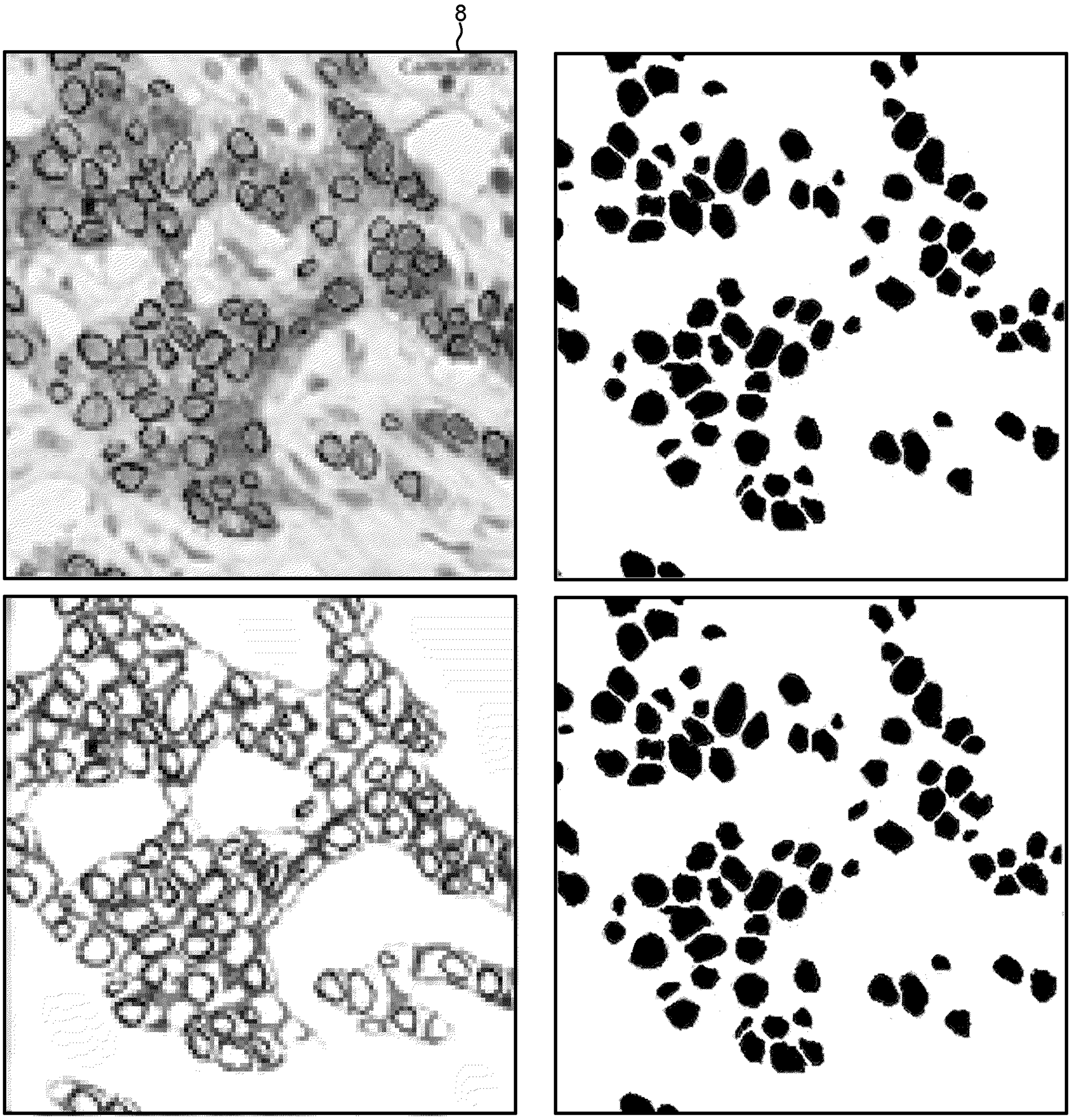


Unique Identifiers for cells at the nuclei pixels

FIG. 16

**Cell segmentation results by region growing of each nucleus with restrictions:**
**(1) not to grow downhill in the membrane posterior,**
**(2) and use a minimal object density constraint for the cell border**

8

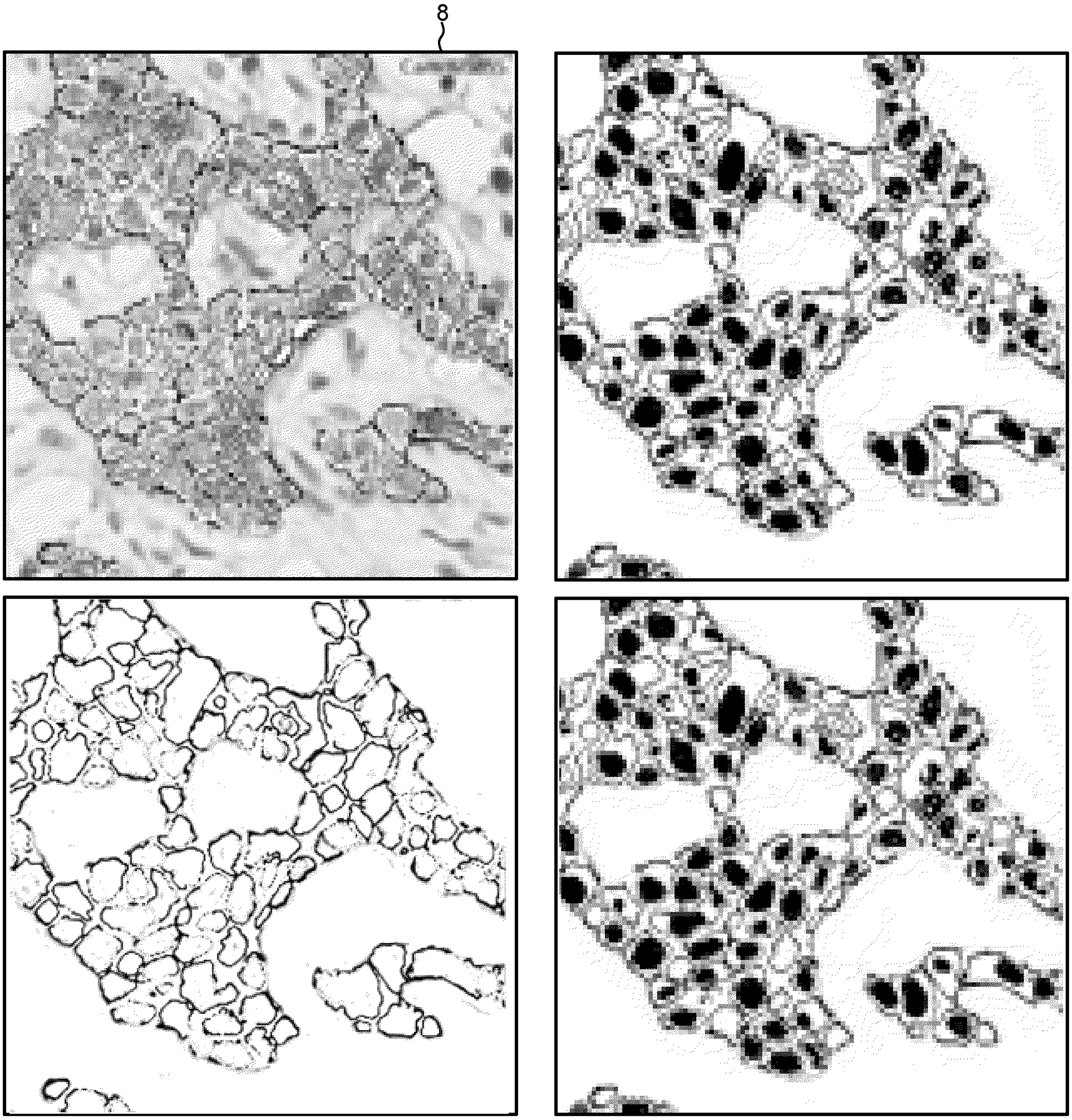

**The unique identifier (UID) for each membrane object is the same as that of the associated nucleus object.**

FIG. 17

Cytoplasm and membrane segmentation results by identification of cell border pixels as membrane, and then region growing of the membrane into pixels with membrane posterior >20%.

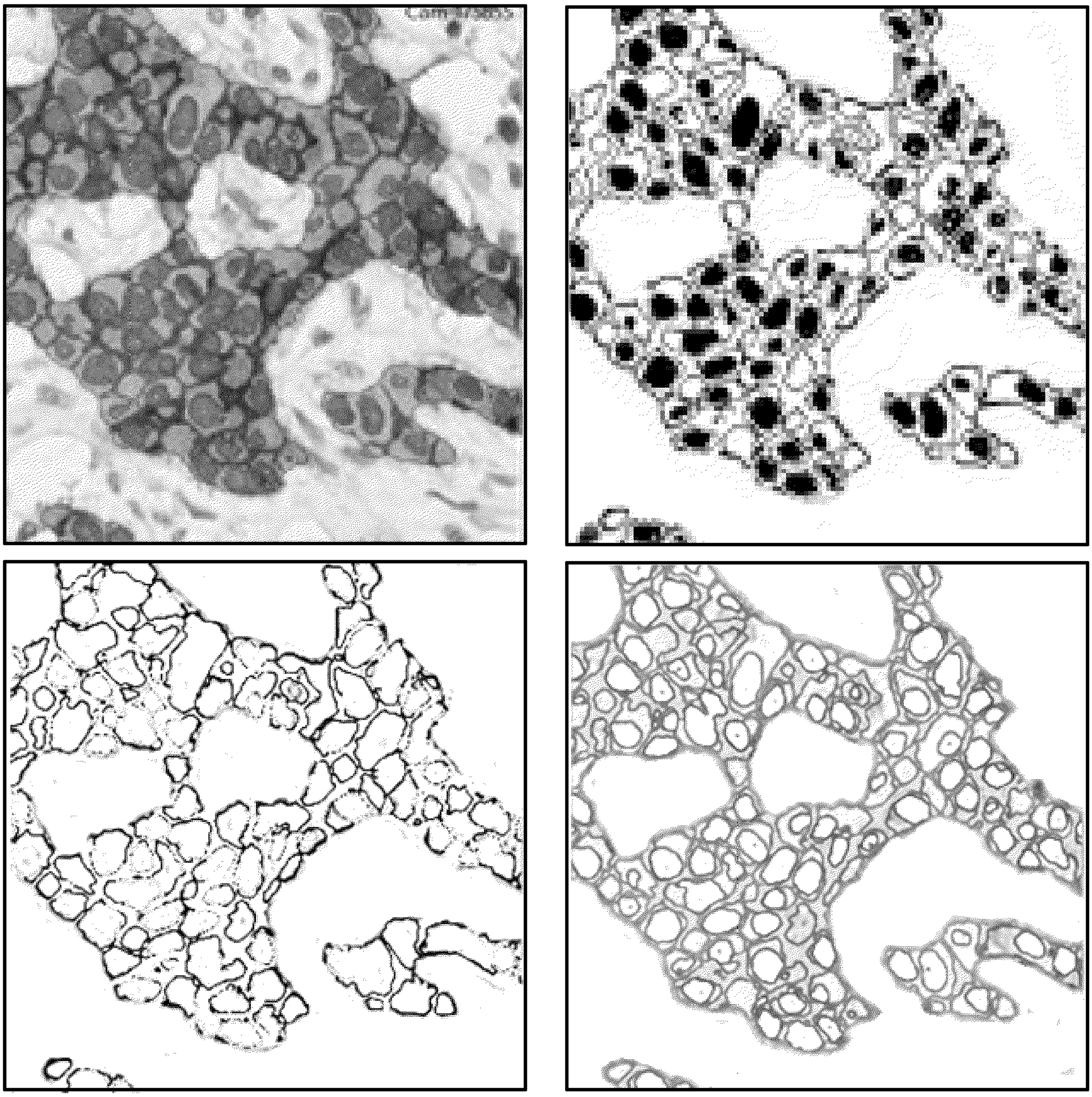

Unique Identifiers for cells at the nuclei, cytoplasm and membrane pixels

FIG. 18

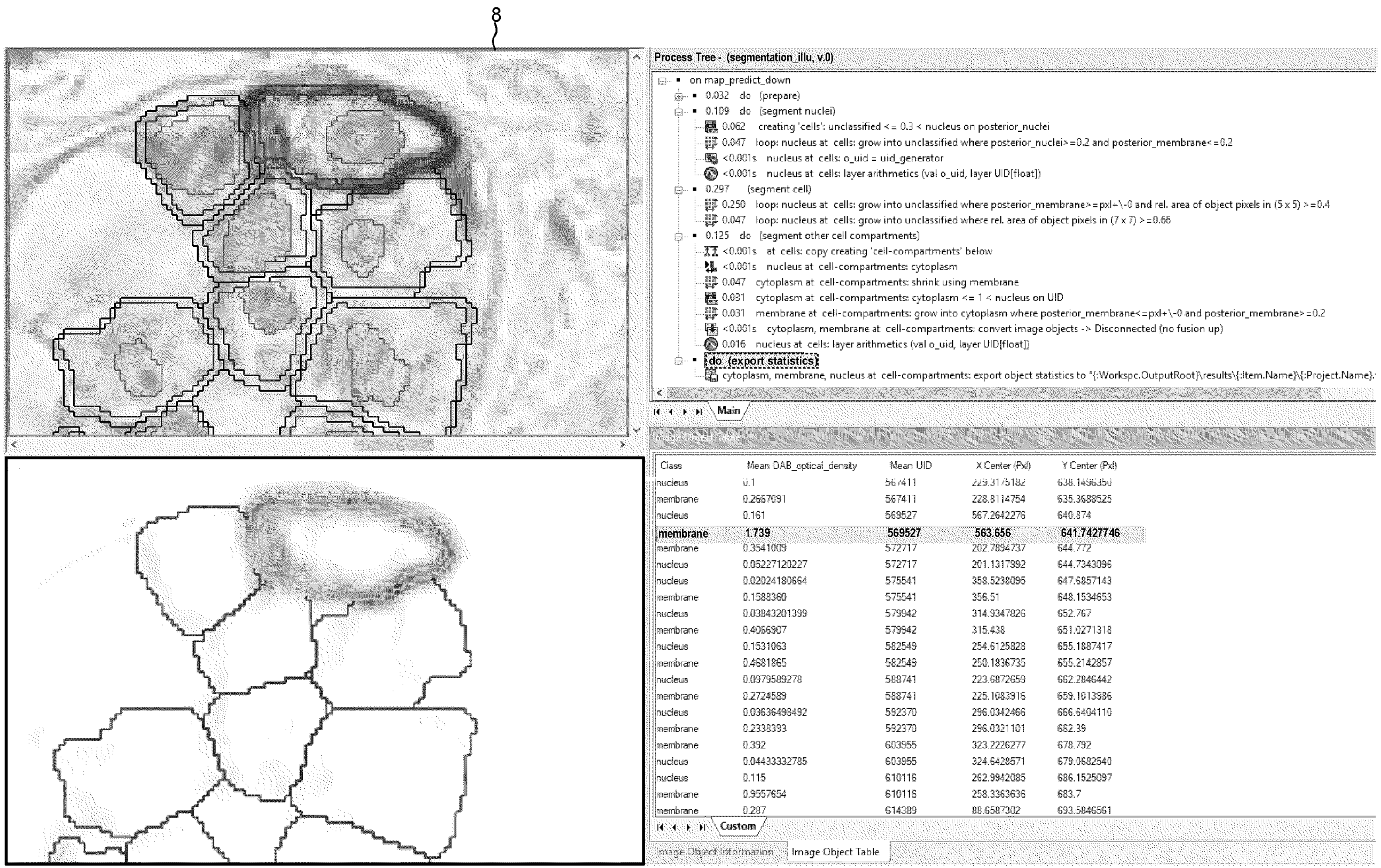


**In the lower image, dark corresponds to high optical density, and bright corresponds to low optical density**

FIG. 19

COGNITION NETWORK LANGUAGE SCRIPT IS USED TO SEGMENT CELLS INTO SEPARATE NUCLEUS, CYTOPLASM AND MEMBRANE.

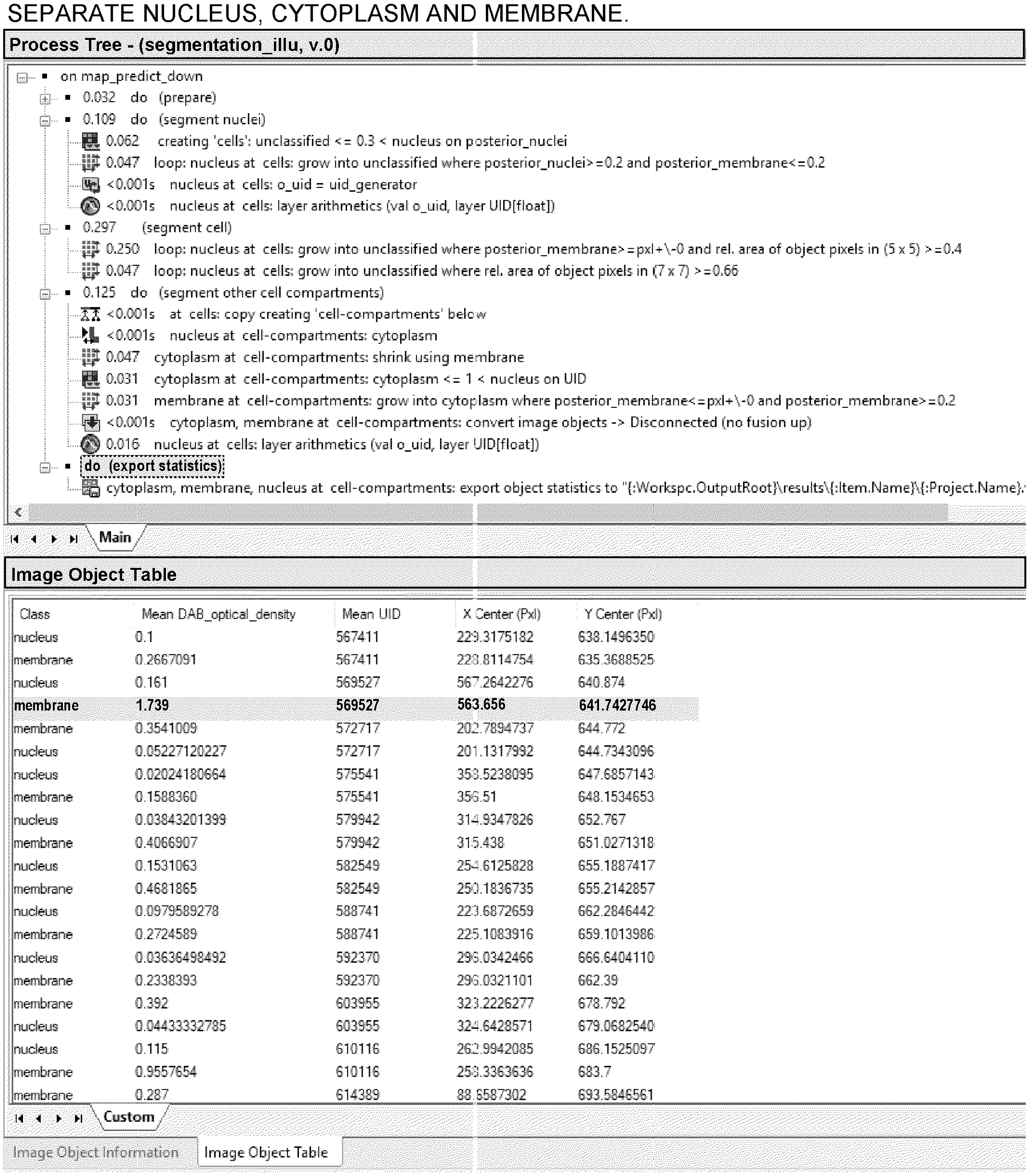

**Process Tree - (segmentation_illu, v.0)**

- on map_predict_down
  - 0.032 do (prepare)
  - 0.109 do (segment nuclei)
    - 0.062 creating 'cells': unclassified <= 0.3 < nucleus on posterior_nuclei
    - 0.047 loop: nucleus at cells: grow into unclassified where posterior_nuclei>=0.2 and posterior_membrane<=0.2
    - <0.001s nucleus at cells: o_uid = uid_generator
    - <0.001s nucleus at cells: layer arithmetics (val o_uid, layer UID[float])
  - 0.297 (segment cell)
    - 0.250 loop: nucleus at cells: grow into unclassified where posterior_membrane>=pxl+\-0 and rel. area of object pixels in (5 x 5) >=0.4
    - 0.047 loop: nucleus at cells: grow into unclassified where rel. area of object pixels in (7 x 7) >=0.66
  - 0.125 do (segment other cell compartments)
    - <0.001s at cells: copy creating 'cell-compartments' below
    - <0.001s nucleus at cell-compartments: cytoplasm
    - 0.047 cytoplasm at cell-compartments: shrink using membrane
    - 0.031 cytoplasm at cell-compartments: cytoplasm <= 1 < nucleus on UID
    - 0.031 membrane at cell-compartments: grow into cytoplasm where posterior_membrane<=pxl+\-0 and posterior_membrane>=0.2
    - <0.001s cytoplasm, membrane at cell-compartments: convert image objects -> Disconnected (no fusion up)
    - 0.016 nucleus at cells: layer arithmetics (val o_uid, layer UID[float])
  - do (export statistics)
    - cytoplasm, membrane, nucleus at cell-compartments: export object statistics to "{:Workspc.OutputRoot}\results\{:Item.Name}\{:Project.Name}.

Main

**Image Object Table**

| Class | Mean DAB_optical_density | Mean UID | X Center (Pxl) | Y Center (Pxl) |
|---|---|---|---|---|
| nucleus | 0.1 | 567411 | 229.3175182 | 638.1496350 |
| membrane | 0.2667091 | 567411 | 228.8114754 | 635.3688525 |
| nucleus | 0.161 | 569527 | 567.2642276 | 640.874 |
| **membrane** | **1.739** | **569527** | **563.656** | **641.7427746** |
| membrane | 0.3541009 | 572717 | 202.7894737 | 644.772 |
| nucleus | 0.05227120227 | 572717 | 201.1317992 | 644.7343096 |
| nucleus | 0.02024180664 | 575541 | 353.5238095 | 647.6857143 |
| membrane | 0.1588360 | 575541 | 356.51 | 648.1534653 |
| nucleus | 0.03843201399 | 579942 | 314.9347826 | 652.767 |
| membrane | 0.4066907 | 579942 | 315.438 | 651.0271318 |
| nucleus | 0.1531063 | 582549 | 254.6125828 | 655.1887417 |
| membrane | 0.4681865 | 582549 | 250.1836735 | 655.2142857 |
| nucleus | 0.0979589278 | 588741 | 223.6872659 | 662.2846442 |
| membrane | 0.2724589 | 588741 | 225.1083916 | 659.1013986 |
| nucleus | 0.03636498492 | 592370 | 296.0342466 | 666.6404110 |
| membrane | 0.2338393 | 592370 | 296.0321101 | 662.39 |
| membrane | 0.392 | 603955 | 323.2226277 | 678.792 |
| nucleus | 0.04433332785 | 603955 | 324.6428571 | 679.0682540 |
| nucleus | 0.115 | 610116 | 262.9942085 | 686.1525097 |
| membrane | 0.9557654 | 610116 | 253.3363636 | 683.7 |
| membrane | 0.287 | 614389 | 88.6587302 | 693.5846561 |

Custom

Image Object Information | Image Object Table

THE STATISTICS ARE TABULATED OF THE DAB OPTICAL DENSITY FOR ALL MEMBRANES AND CYTOPLASMS IN THE IMAGE .

FIG. 20

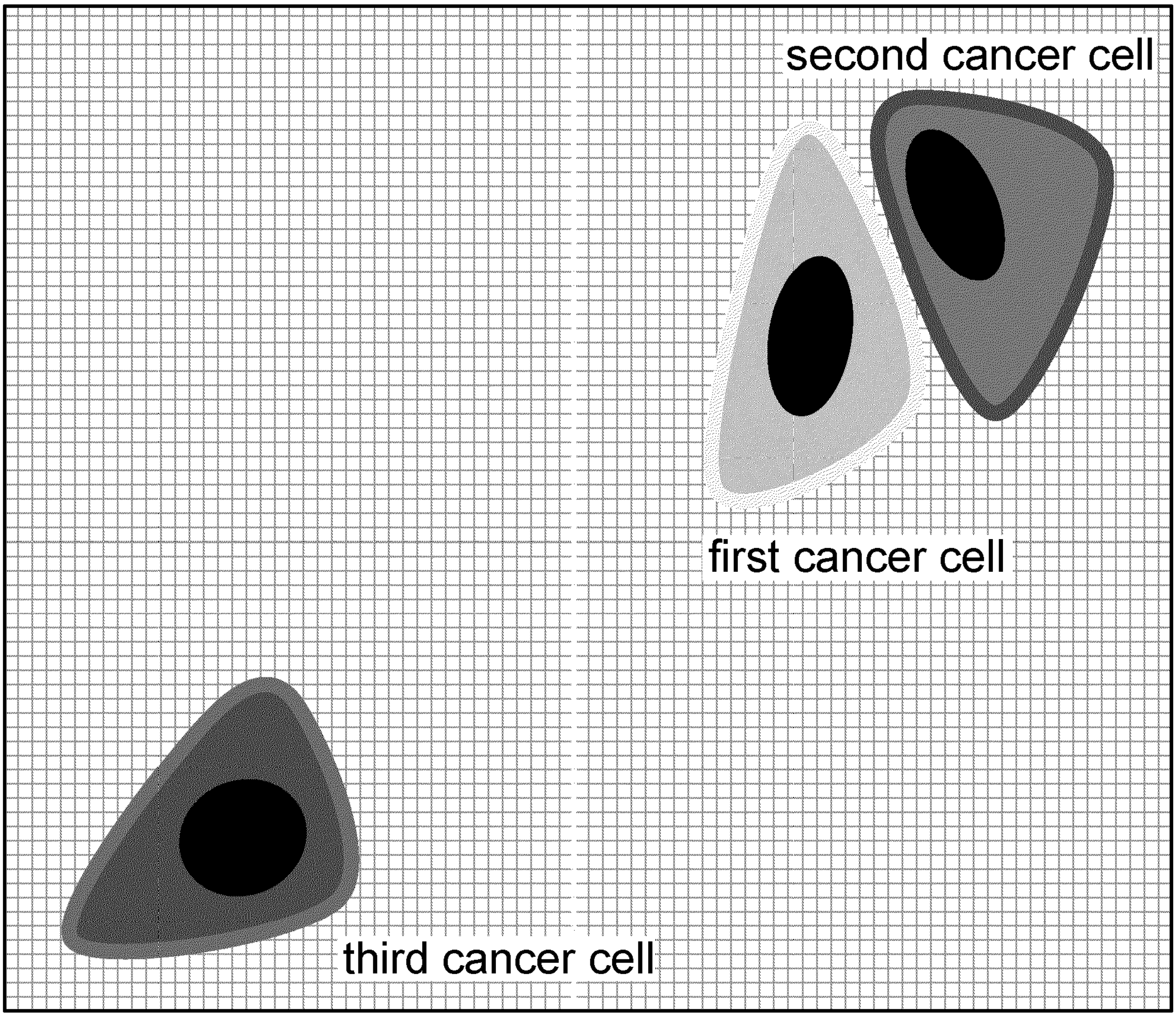


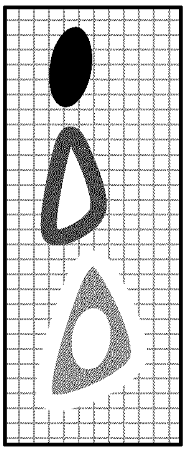

**cell nucleus**

**cell membrane: gray value represents average DAB optical density of all membrane pixels (brighter corresponds to more amount of diagnostic antibody)**

**cell cytoplasm: gray value represents average DAB optical density of all cytoplasm pixels.**

FIG. 21

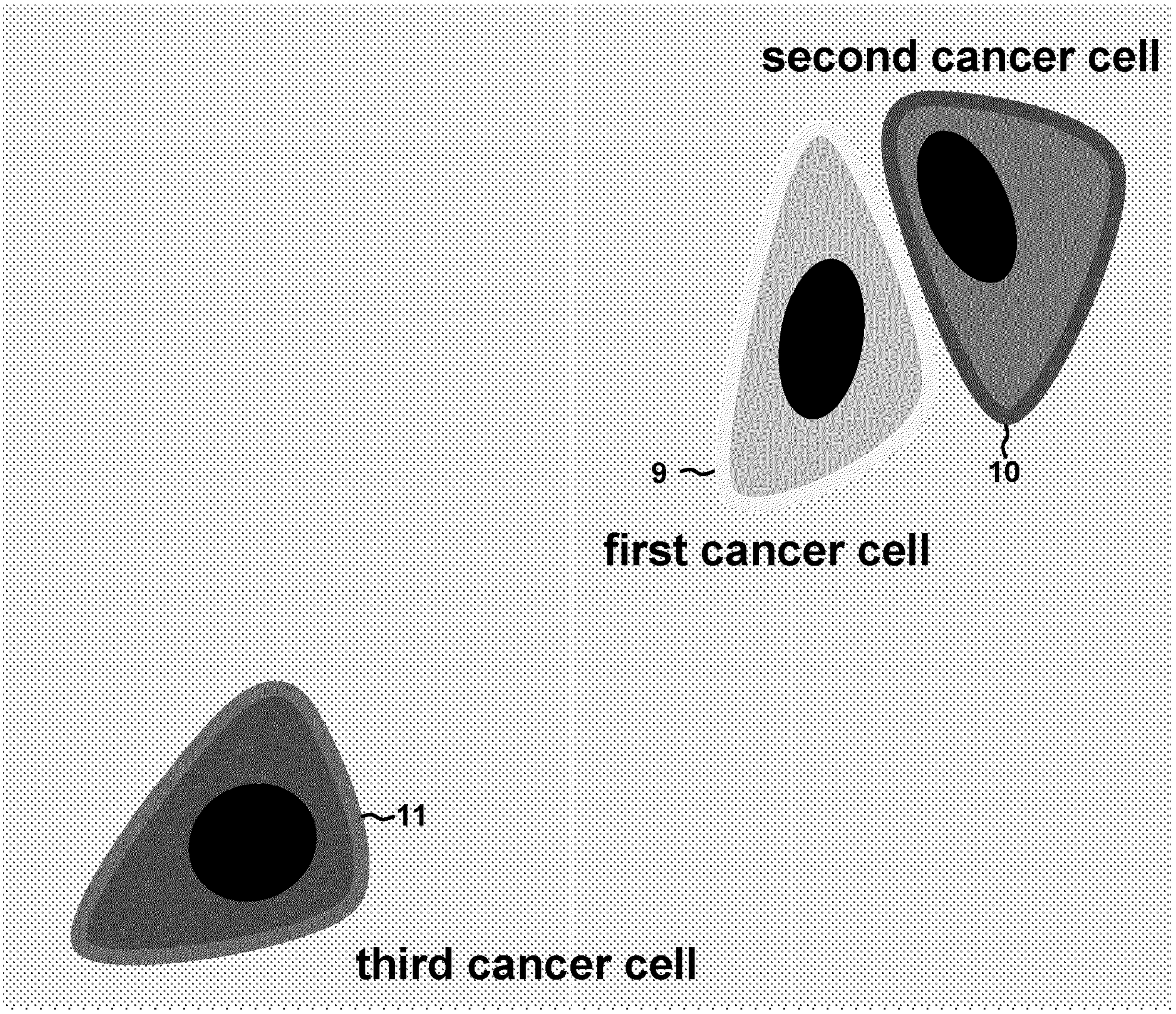


| UID | X [pixels] | Y [pixels] | Optical density membrane (ODM) | Optical density cytoplasm (ODC) |
|---|---|---|---|---|
| 1 | 480 | 190 | 242/255 = .949 | 203/255 = .796 |
| 2 | 590 | 130 | 94/255 = .369 | 136/255 = .533 |
| 3 | 140 | 500 | 127/255 = .498 | 94/255 = .369 |

FIG. 22

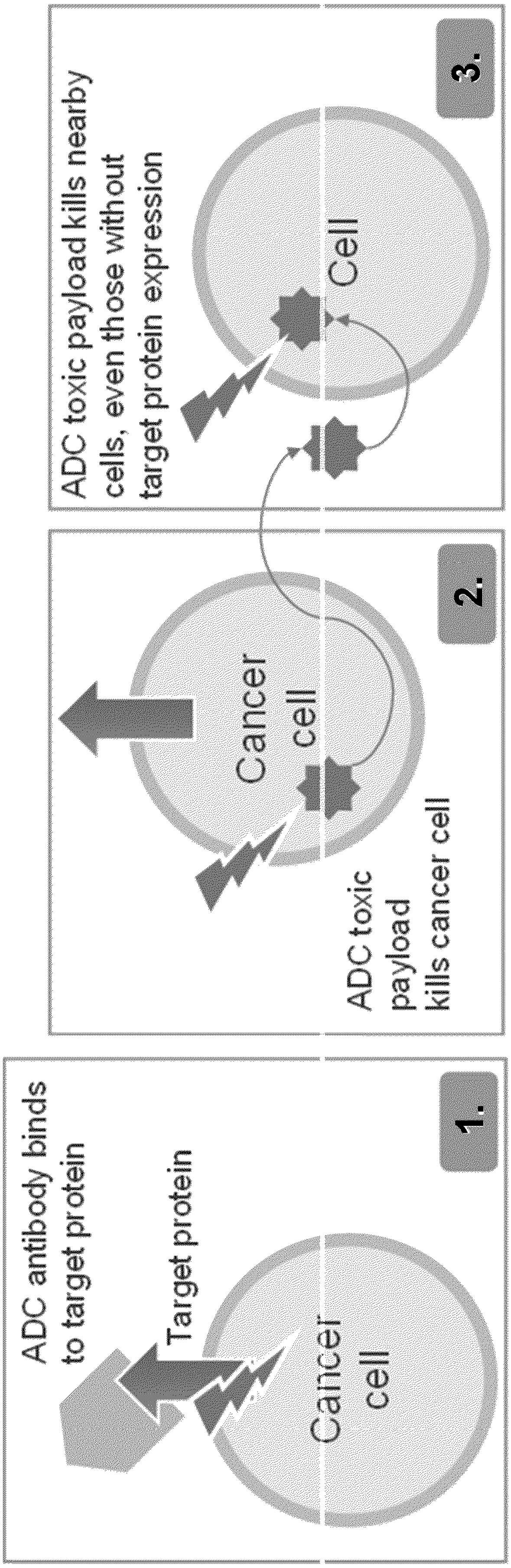
ADC antibody binds to target protein
Target protein
Cancer cell
1.
ADC toxic payload kills cancer cell
Cancer cell
2.
ADC toxic payload kills nearby cells, even those without target protein expression
Cell
3.

FIG. 23

# Cell statistics with single cell ADC scoring

**r_1 – r_i : euclidean distance from center of gravity of the cell with UID 1 to the cell with UID i.**

**w_ij = exp ( - |r_i – r_j | / r_norm ) : exponential weighting factor to include ADC payload uptake from neighboring cells**

**Single cell ADC score for cell i = min (K1 , Sum_j with w_ij < 15% { w_ij x max ( 0, ODM_j x (K2 + ODM_j – ODC_j )) } )**

| UID | X [pixels] | Y [pixels] | Optical density membrane (ODM) | Optical density cytoplasm (ODC) | max(0, ODM x(ODM-ODC)) | r_1-r_i | r_2-r_i | r_3-r_i | exp(-(r_1-r_i)/50) | exp(-(r_2-r_i)/50) | exp(-(r_3-r_i)/50) | Single-Cell ADC Uptake |
|---|---|---|---|---|---|---|---|---|---|---|---|---|
| 1 | 480 | 190 | 0.949019608 | 0.796078431 | 0.145144175 | 0 | 125.2996 | 460.1087 | 1 | 0.08159455 | 0.00010082 | 0.145144175 |
| 2 | 590 | 130 | 0.368627451 | 0.533333333 | 0 | 125.2996 | 0 | 582.5805 | 0.08159455 | 1 | 8.705E-06 | 0.011842974 |
| 3 | 140 | 500 | 0.498039216 | 0.368627451 | 0.064452134 | 460.1087 | 582.5805 | 0 | 0.00010082 | 8.705E-06 | 1 | 0.064452134 |

r_norm=50

FIG. 24

GENERATING A SCORE FOR EACH CELL WHICH REFLECTS THE LIKELIHOOD THAT A CANCER CELL IS DAMAGED IN FUNCTION OR KILLED

Single Cell ADC score = Weighted sum of this cell and neighboring cells of a bilinear combination of the amounts of Target Protein on the cells membrane and cytoplasm, the weights depend on the distance of the neighboring cells to this cell.

Mathematics for cell i with position $r_i$ on the slide and average membrane (cytoplasm) optical density values $ODM_i$ ($ODC_i$)

$$single_cell_score_i = Sum_j \text{ with } |r_j - r_i|<d \{ a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|) \}$$

with the functions $a_{kl}$ depending on the distance $|r_j - r_i|$ of the cell j to the cell i, e.g., $a_{kl}(|r_j - r_i|) = A_{kl} \times \exp(- |r_j - r_i|/r_{norm})$ with $A_{kl}$ and $r_0$ constants determined by optimized therapy success prediction.

FIG. 25

| # | Model | Mean H Score | Indication | Mean % Tumour ... | Mean % Tumour 2... | Mean % Tumour 1... | Mean % Tumour ... | %TG NIP228-SG3932 | %TG INT016-SG3932 | Response category (B7H4-ADC) |
|---|---|---|---|---|---|---|---|---|---|---|
| 1 | CTG-2432 | 7 | Breast | 2.13319 | 7.19308 | 32.86937 | 57.80430 | 82.10000 | 95 | PD |
| 2 | CTG-2308 | 163 | Breast | 2.94132 | 3.83924 | 11.05063 | 82.16880 | 86.70000 | 50.50000 | PD |
| 3 | CTG-1727 | 243 | Breast | 38.32497 | 23.64700 | 22.73467 | 15.29333 | -91.10000 | -60.90000 | R |
| 4 | CTG-1705 | 15 | Breast | 18.10867 | 16.47230 | 29.34750 | 36.07153 | 88.60000 | 91.40000 | PD |
| 5 | CTG-1647 | 256 | Breast | 70.91383 | 15.37857 | 8.96942 | 4.73822 | 88 | -26.40000 | SD |
| 7 | CTG-1408 | 259 | Breast | 78.58367 | 14.59943 | 4.39727 | 2.41959 | 60.20000 | -74.80000 | R |
| 8 | CTG-1242 | 137 | Breast | 34.64913 | 17.49317 | 23.42373 | 24.43390 | -96 | -79.80000 | R |
| 9 | CTG-1211 | 54 | Breast | 1.24608 | 1.93807 | 7.78568 | 89.03017 | 38 | 24.10000 | SD |
| 10 | CTG-1153 | 185 | Breast | 46.61777 | 27.55307 | 19.52799 | 6.30119 | 33.40000 | -80.80000 | R |
| 11 | CTG-1124 | 160 | Breast | 6.59805 | 8.14635 | 20.92987 | 64.32573 | -57.30000 | -76.40000 | R |
| 12 | CTG-1106 | 69 | Breast | 71.31187 | 14.57053 | 9.71658 | 4.40100 | 111.30000 | 71 | PD |
| 13 | CTG-1018 | 2 | Breast | 0.5844 | 0.9539 | 4.94309 | 93.51867 | 40.10000 | 44.20000 | PD |
| 14 | CTG-0052 | 28 | Breast | 26.93974 | 12.98232 | 15.29437 | 44.78359 | -56.20000 | -31.30000 | R |
| 15 | CTG-0017 | 269 | Breast | 71.46083 | 16.77000 | 7.98098 | 3.78816 | -67.70000 | -73.50000 | R |
| 16 | CTG-0012 | 258 | Breast | 88.03390 | 6.25682 | 3.17673 | 2.53252 | -86 | -100 | R |

**Correlation between H Score and response category for 15 patients in PDX Breast study**

FIG. 26

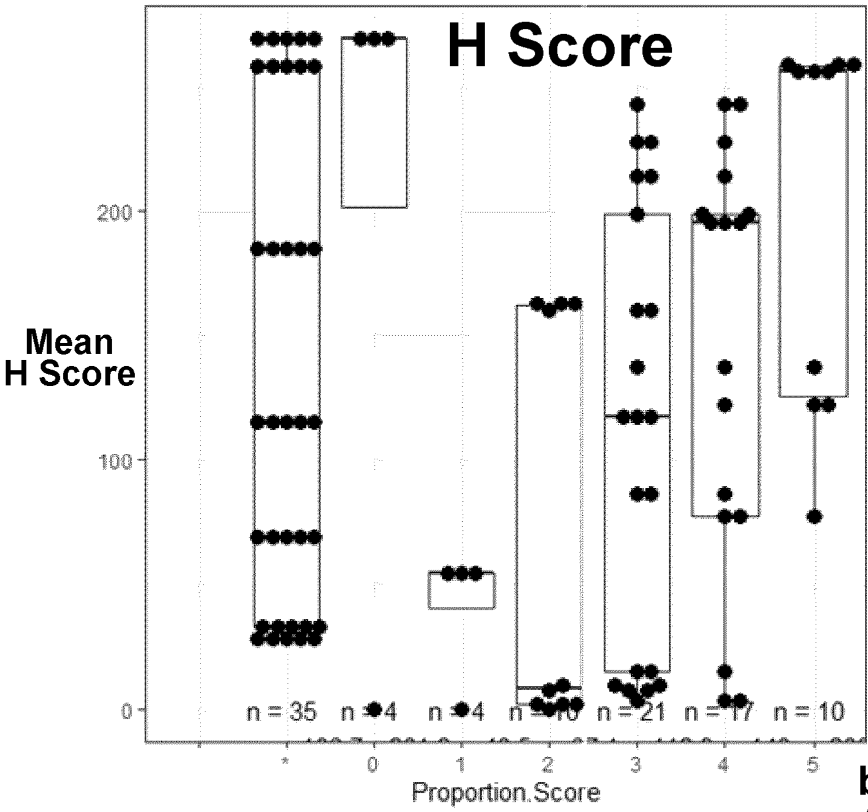


FIG. 27

For the proportion score, there is a strong correlation between QCS score (q50) and manually assessed H Score

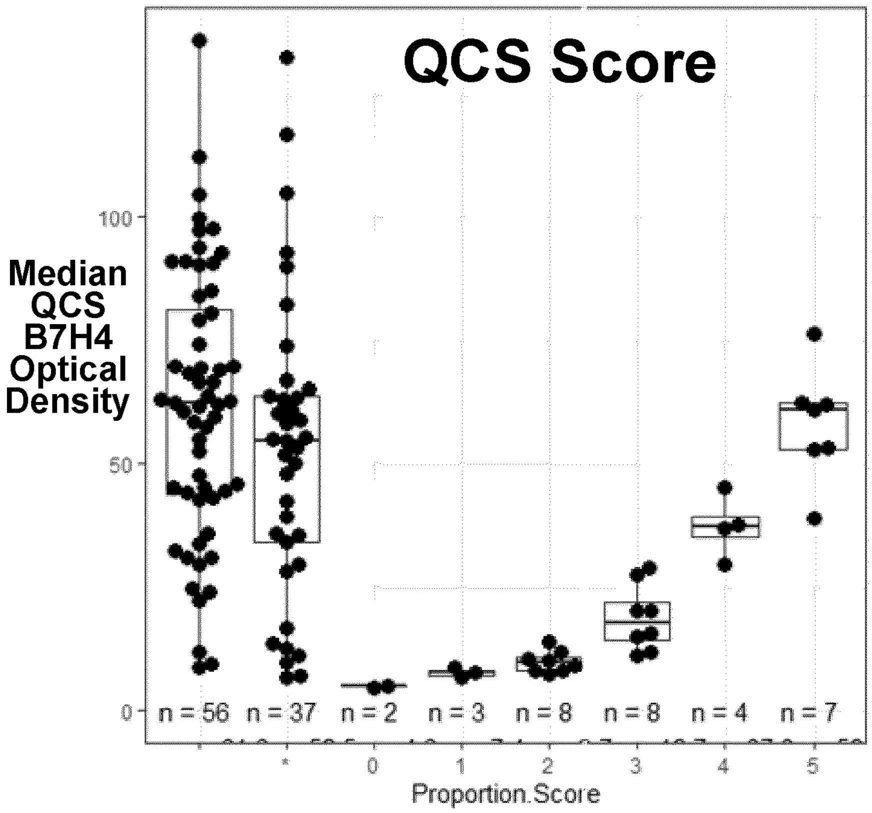


FIG. 28

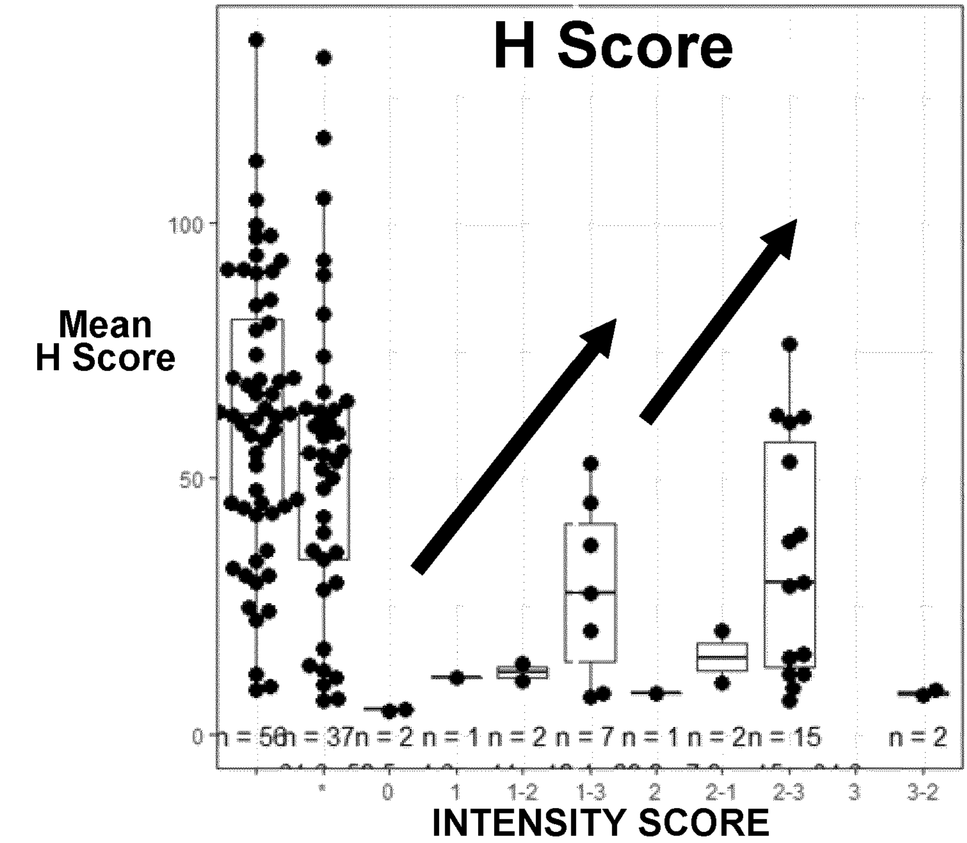
H Score
Mean
H Score
n = 56
n = 37
n = 2
n = 1
n = 2
n = 7
n = 1
n = 2
n = 15
n = 2
INTENSITY SCORE

FIG. 29

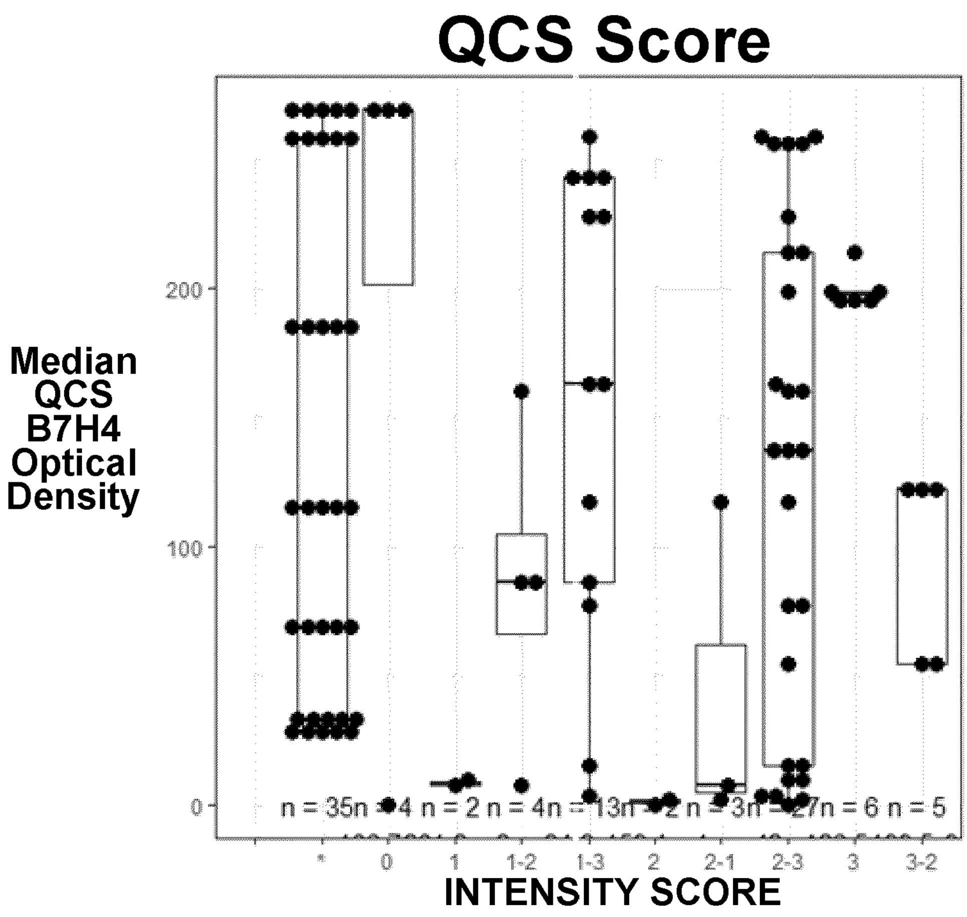
QCS Score
Median
QCS
B7H4
Optical
Density
n = 35
n = 4
n = 2
n = 4
n = 13
n = 2
n = 3
n = 27
n = 6
n = 5
INTENSITY SCORE

FIG. 30

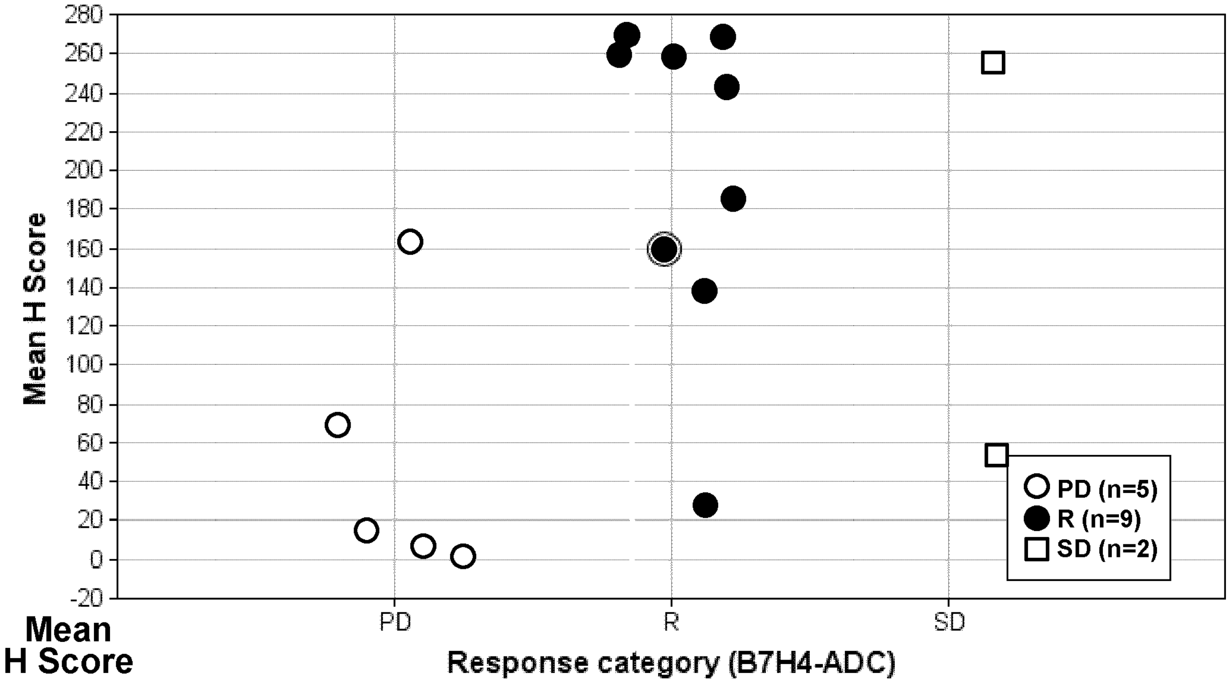

280
260
240
220
200
180
160
140
120
100
80
60
40
20
0
-20
Mean H Score
PD (n=5)
R (n=9)
SD (n=2)
PD
R
SD
Mean
H Score
Response category (B7H4-ADC)


FIG. 31

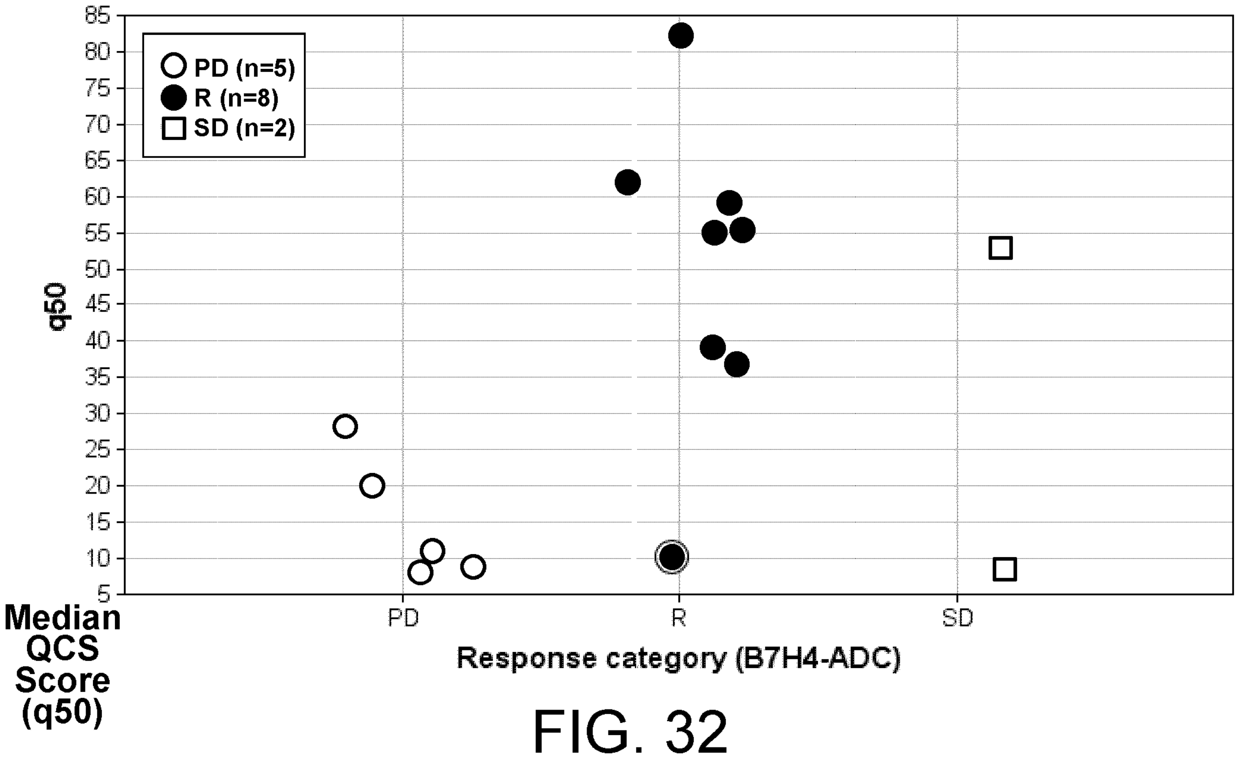

85
80
75
70
65
60
55
50
45
40
35
30
25
20
15
10
5
q50
PD (n=5)
R (n=8)
SD (n=2)
PD
R
SD
Median
QCS
Score
(q50)
Response category (B7H4-ADC)


FIG. 32

Mean
H Score

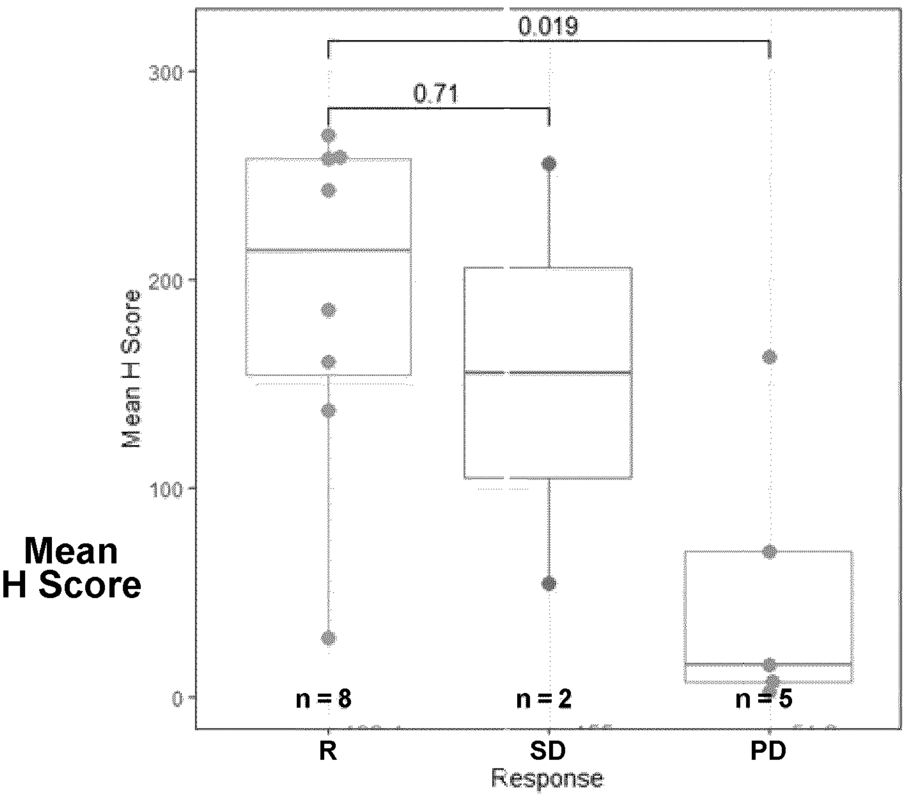


FIG. 33

Median
QCS
Score

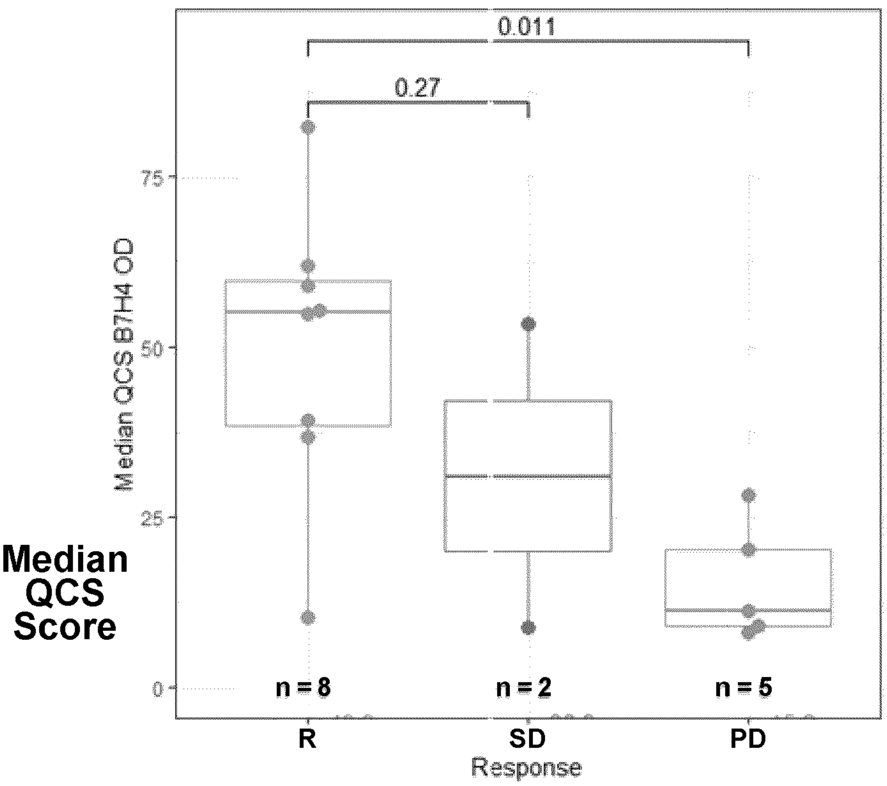


FIG. 34

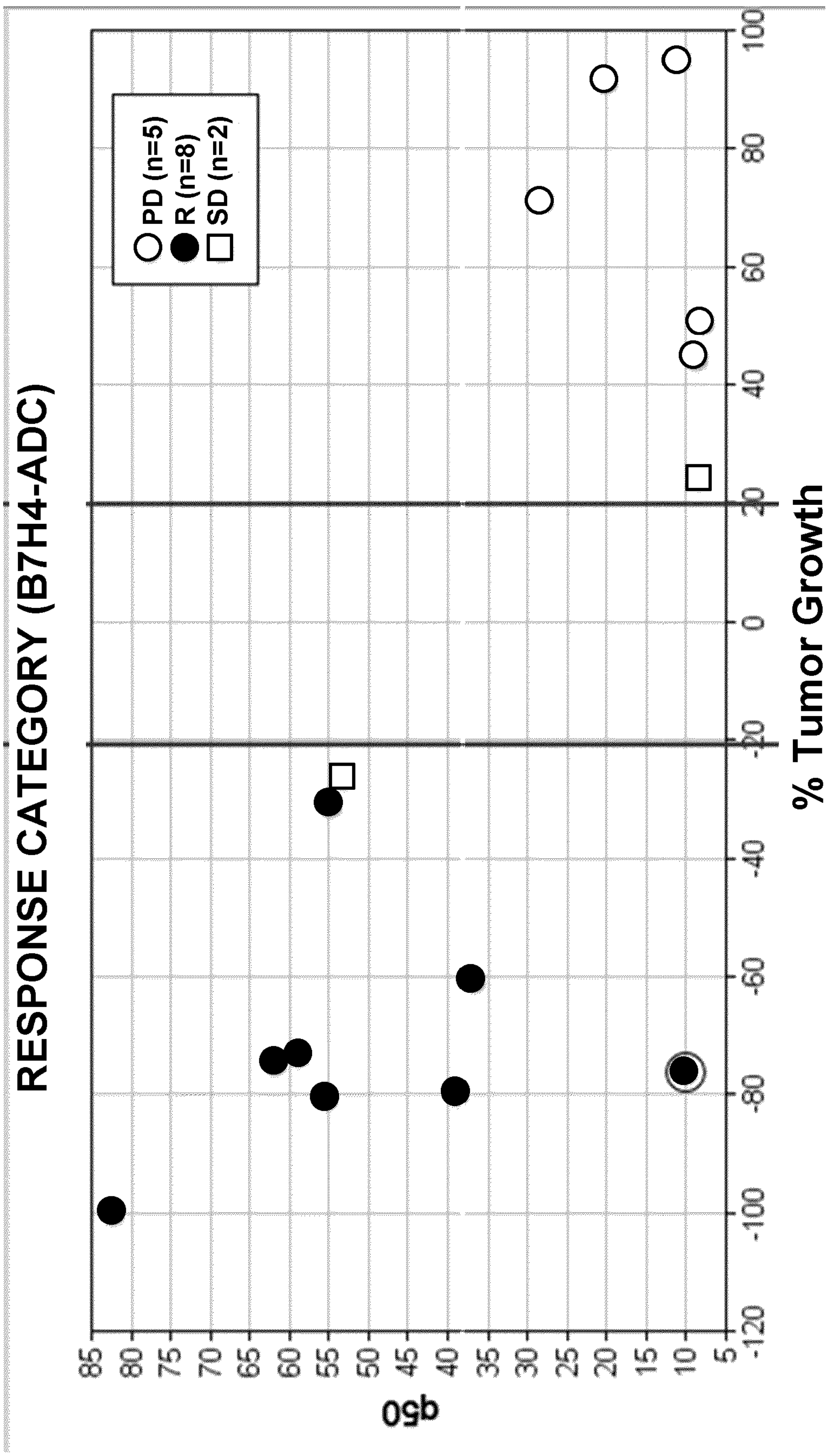
RESPONSE CATEGORY (B7H4-ADC)
PD (n=5)
R (n=8)
SD (n=2)
q50
% Tumor Growth
85
80
75
70
65
60
55
50
45
40
35
30
25
20
15
10
5
-120
-100
-80
-60
-40
-20
0
20
40
60
80
100

FIG. 35

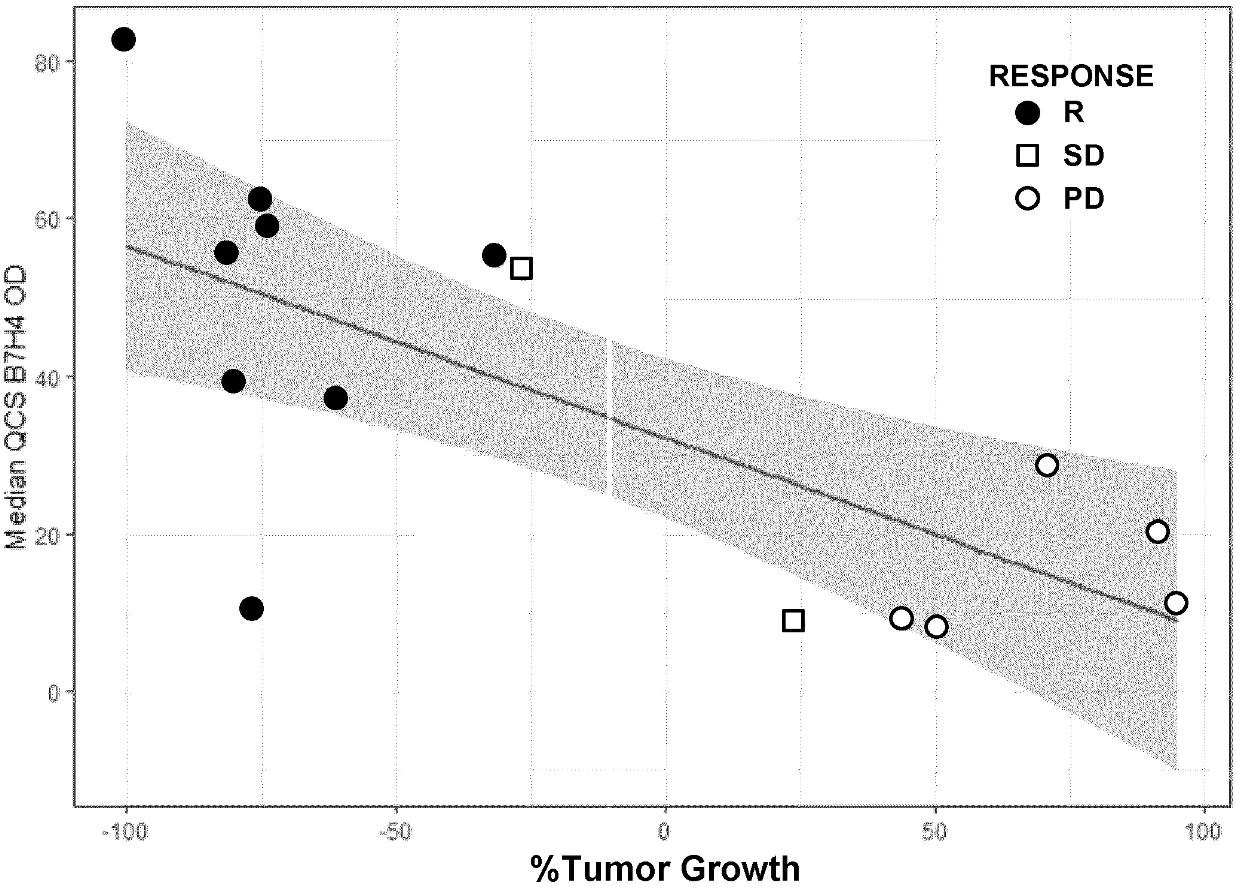


Correlation between QCS Score and tumor growth

FIG. 36

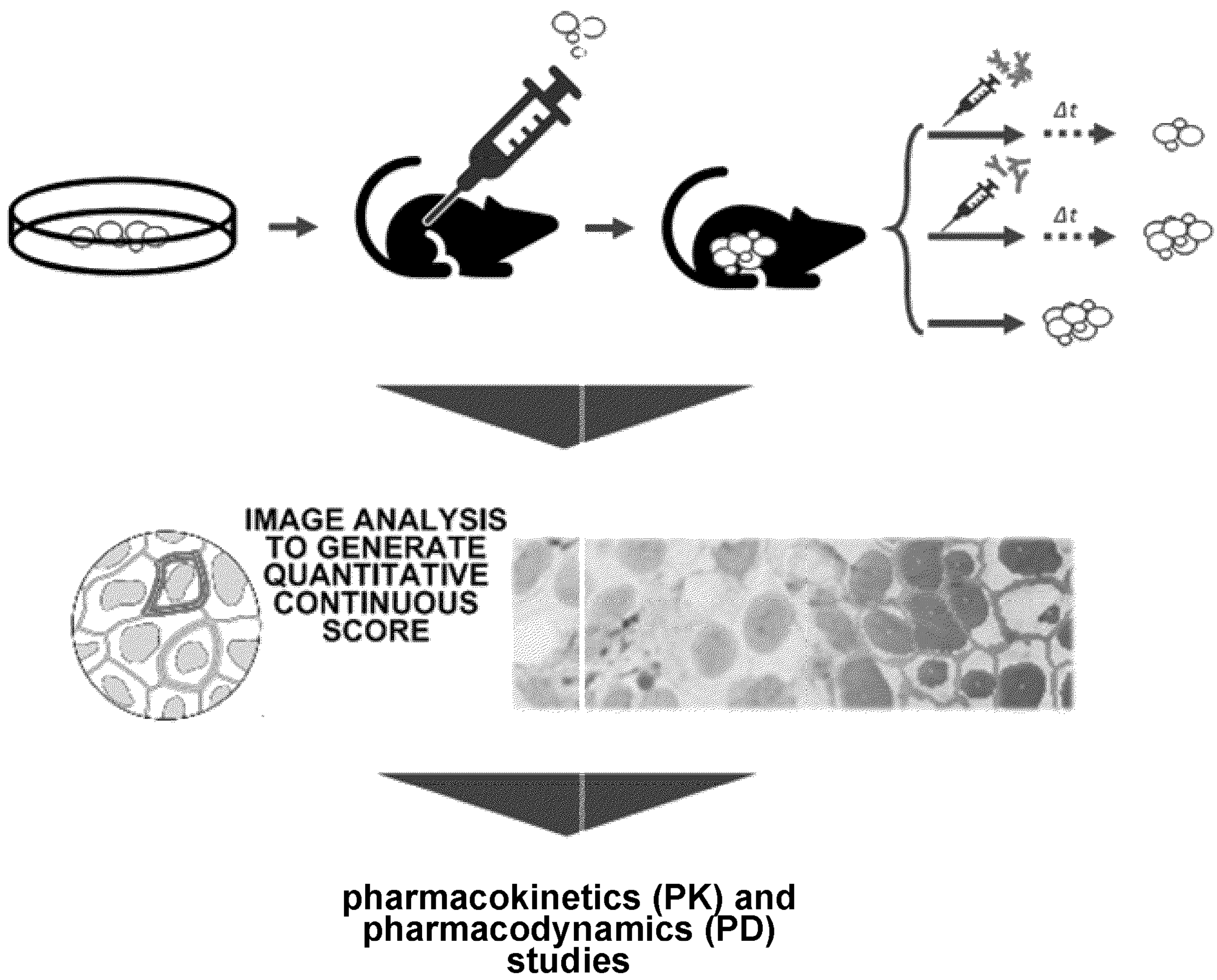
Δt
Δt
IMAGE ANALYSIS
TO GENERATE
QUANTITATIVE
CONTINUOUS
SCORE
pharmacokinetics (PK) and
pharmacodynamics (PD)
studies

FIG. 37

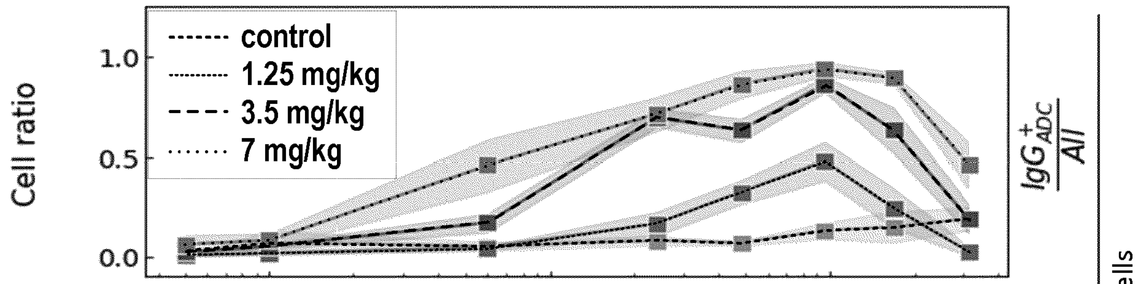


The ratio of epithelial cells staining for ADC-IgG with ADC-IgG bound ($OD_{membrane} \geq 5$, which is antibody staining above the maximum value observed in the control) over all epithelial cells.

FIG. 38A

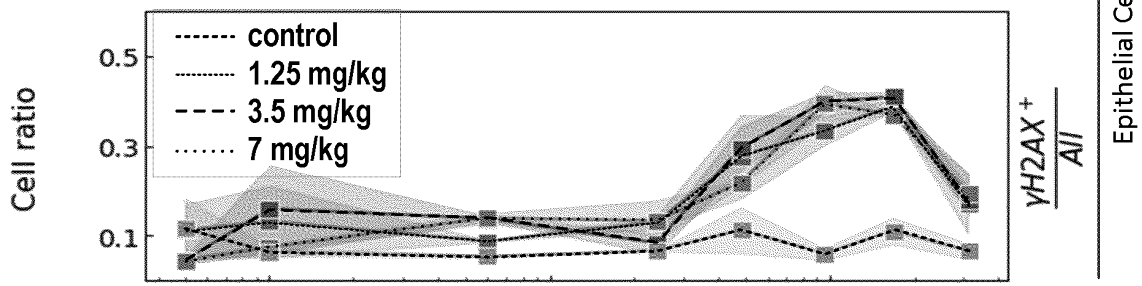


Fraction of epithelial cells found positive for foci in the γH2AX assay. Slides were analyzed with HALO AI (CPSS).

FIG. 38B

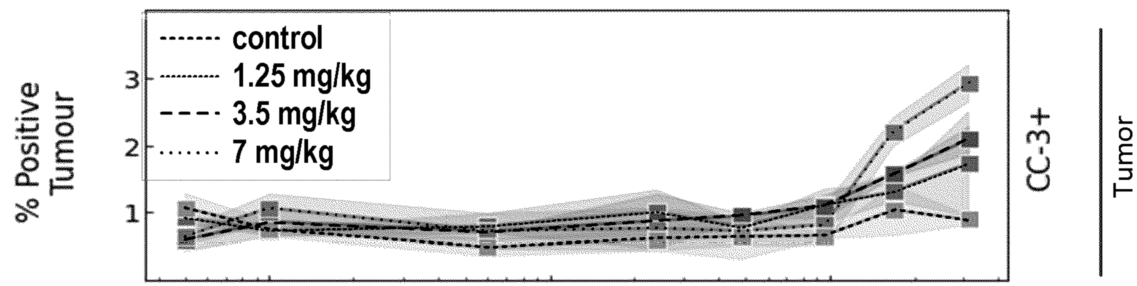


The percent of cleaved Caspase -3 positive tumor. Slides were analyzed with HALO AI (CPSS).

FIG. 38C

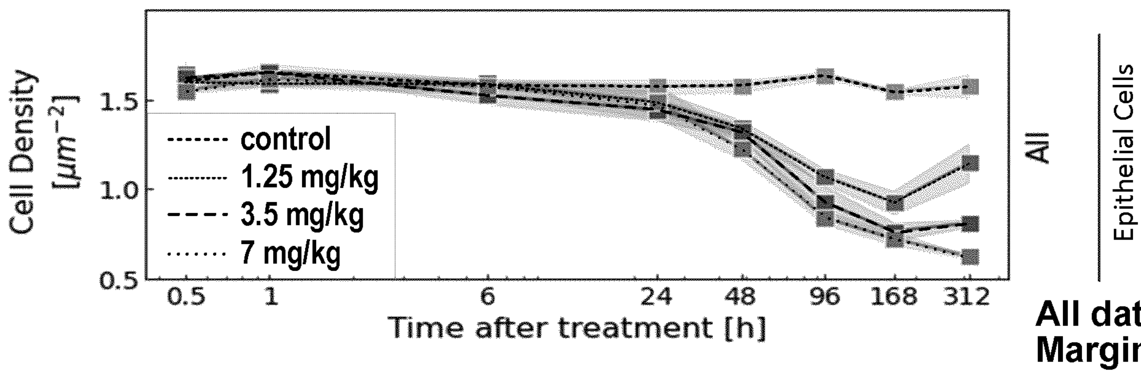


Cell density of all epithelial cells based on QCS analysis. Treatment with E02-GL-SG3932 results in a decrease in cell number, indicating cell death, compared to isotype control ADC.

FIG. 38D

All data shown with logarithmic x-axis. Margin indicates 90% confidence interval.

FIG. 39B

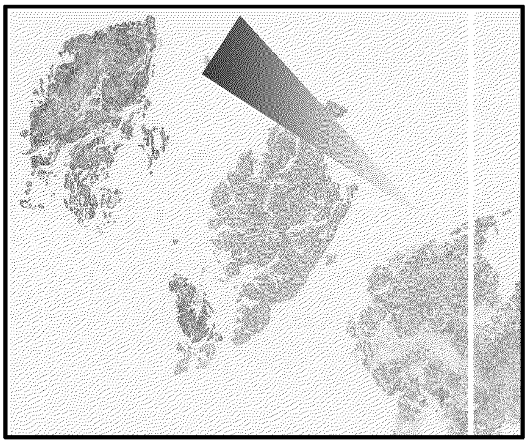

Outlier #1

FIG. 39C

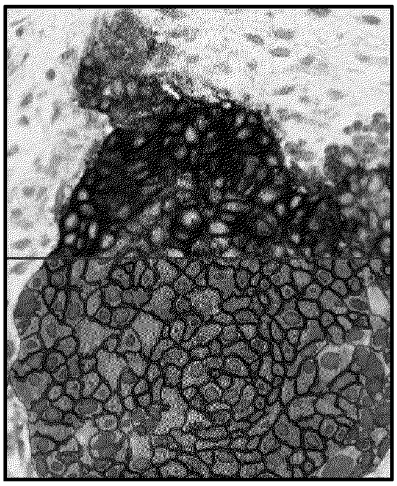

Outlier #2

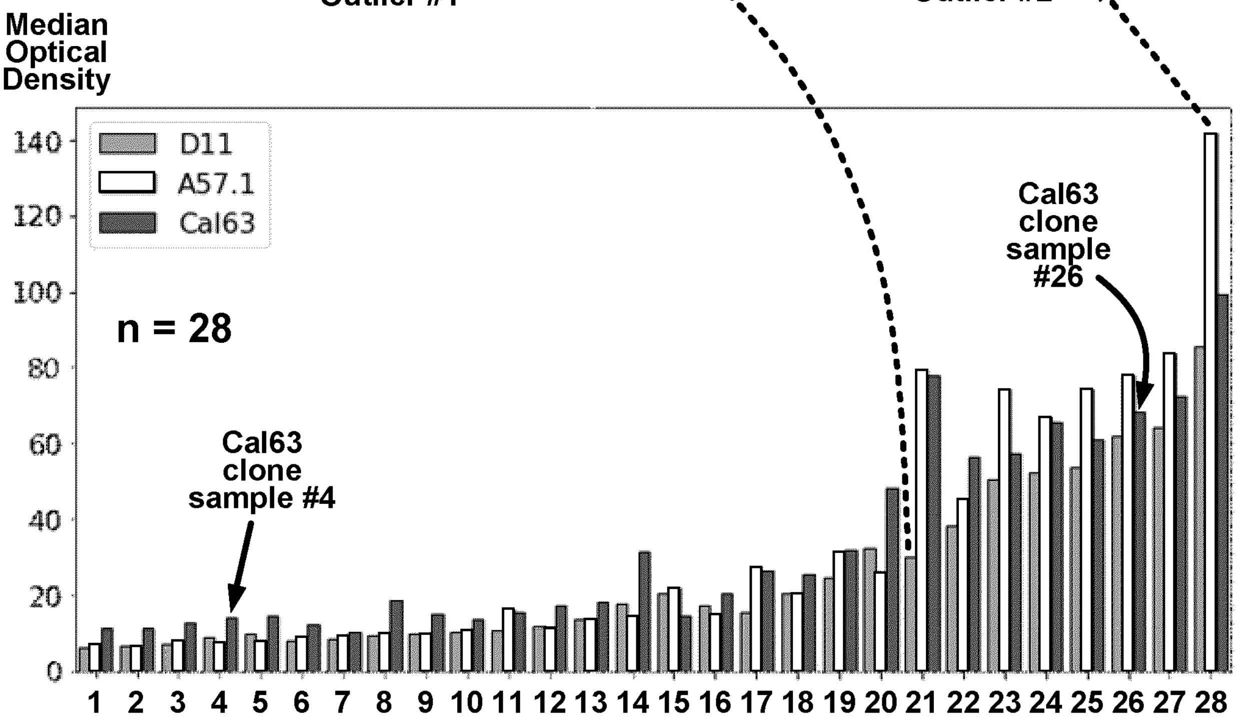


Optical density readouts of D11, A57.1 and Cal63 stained serial sections are highly correlated

FIG. 39A

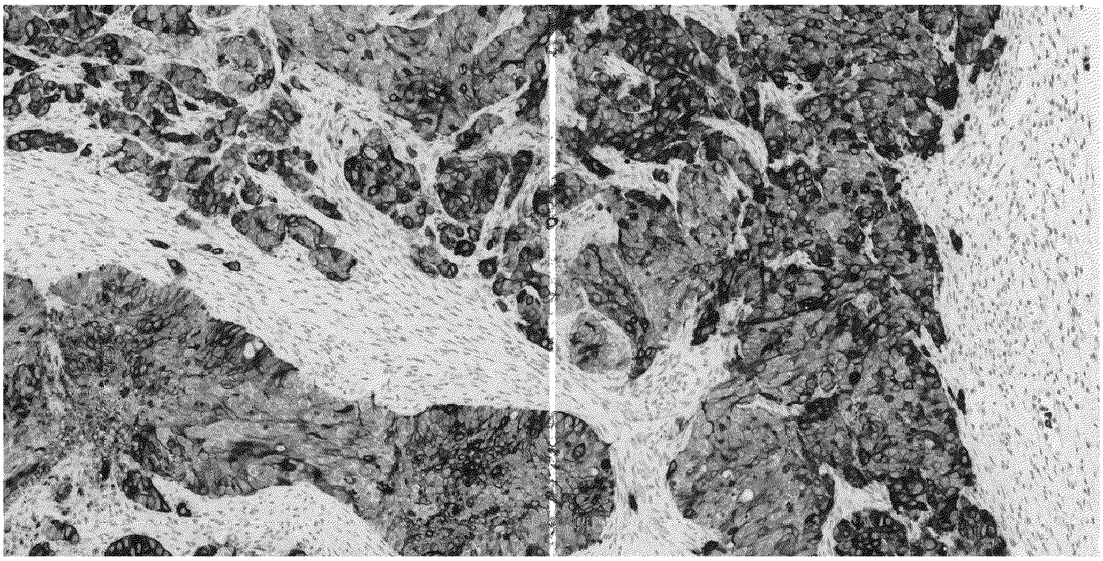

Assay: Cal63

Clone sample #26

FIG. 40A

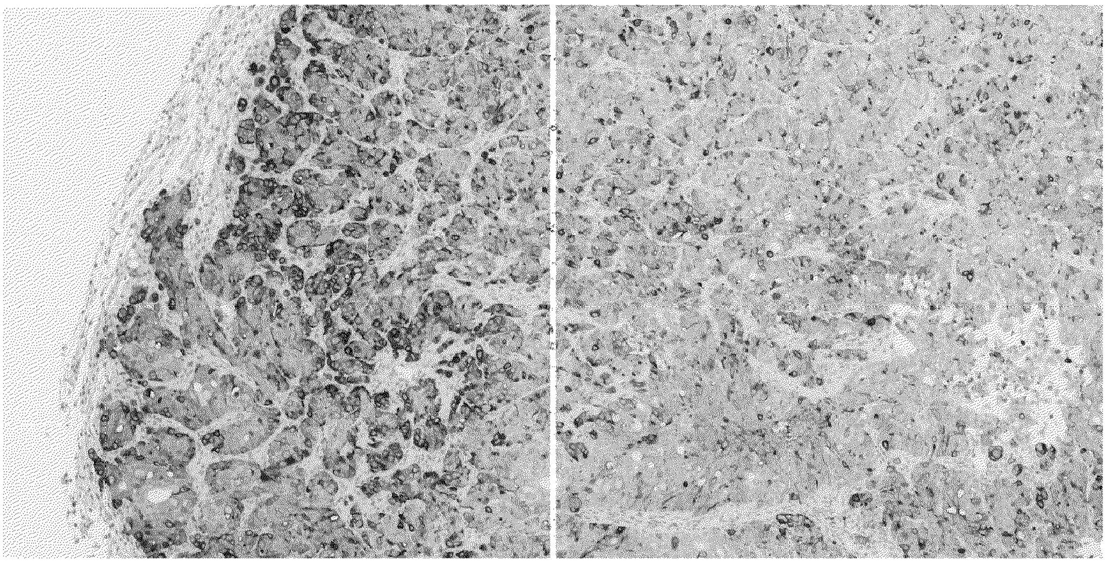

Assay: Cal63

Clone sample #4

FIG. 40B

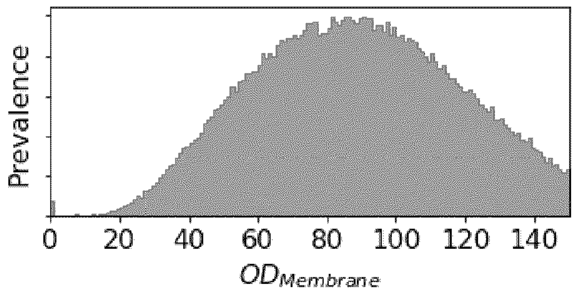


Baseline per-cell distribution of membrane optical density

| | |
|---|---|
| mean OD | 89.2 |
| median OD | 88.4 |
| mean abs. dev. | 25.0 |

B7H4 at time zero, no treatment, n = 3

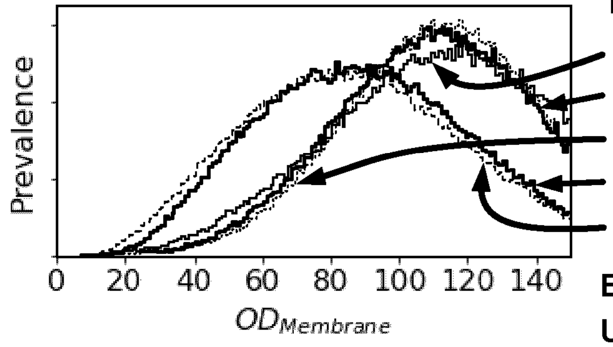


Per-cell distribution of membrane optical density

- 1.25 mg/kg ADC w/o payload
- 3.5 mg/kg ADC w/o payload
- 7 mg/kg ADC w/o payload
- untreated
- isotype antibody

B7H4 at t = 168 h, n = 3

Untreated sample from t = 0 h

Clone sample #26

FIG. 41A

**The median optical density of the membrane staining of clone sample #4 is about 60% that of clone sample #26.**

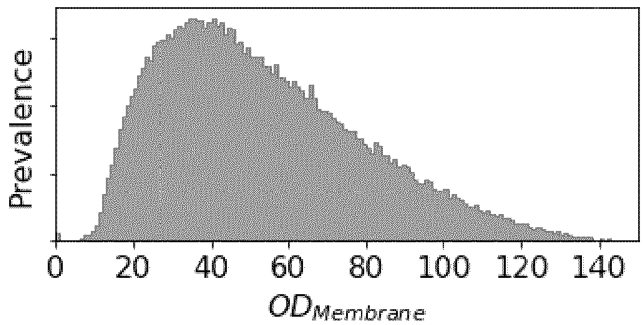


Baseline per-cell distribution of membrane optical density

| | |
|---|---|
| mean OD | 53.3 |
| median OD | 48.9 |
| mean abs. dev. | 20.8 |

B7H4 at time zero, no treatment, n = 3

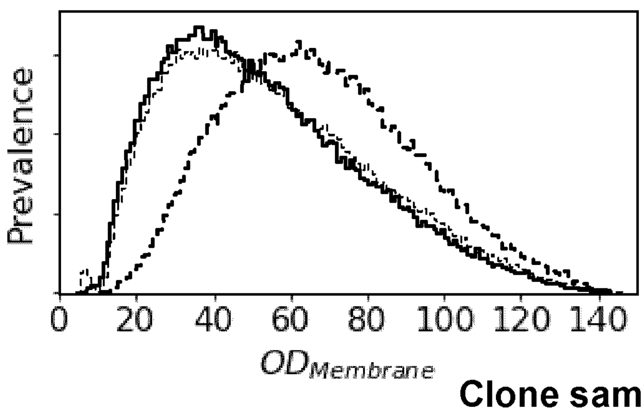


Per-cell distribution of membrane optical density

B7H4 at t = 168 h, n = 3

Untreated sample from t = 0 h

- untreated
- 7 mg/kg isotype antibody
- 7 mg/kg E02-GL-SG3932

Clone sample #4

FIG. 41B

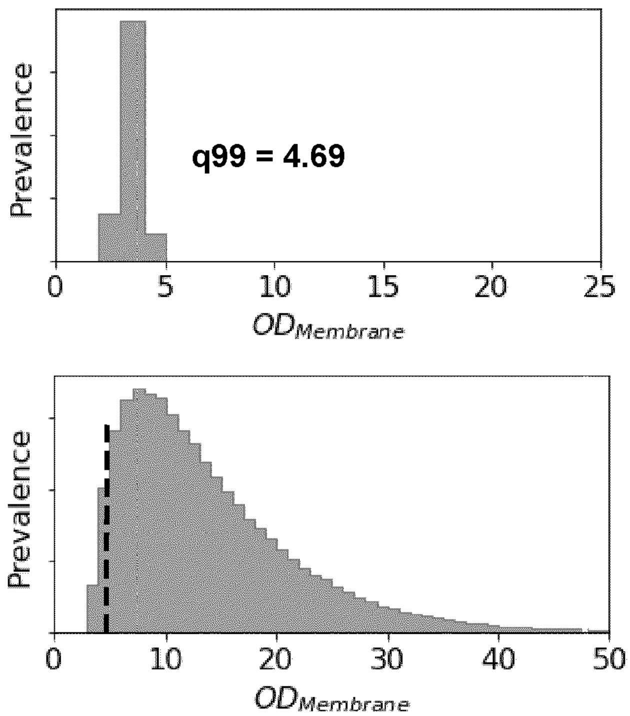


**Baseline per-cell distribution of bound IgG**

**at time zero, no treatment, n = 3**

**Maximum per-cell distribution of bound IgG**

**at 7 mg/kg E02-GL-SG3932, n = 3**

**Clone sample #26**

FIG. 42A

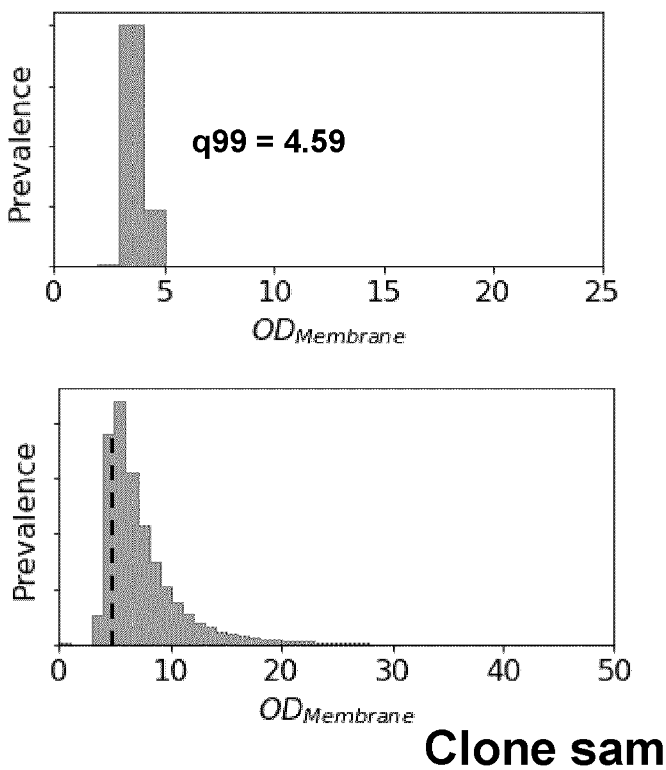


**Baseline per-cell distribution of bound IgG**

**at time zero, no treatment, n = 3**

**Maximum per-cell distribution of bound IgG**

**at 7 mg/kg E02-GL-SG3932, n = 3**

**Clone sample #4**

FIG. 42B

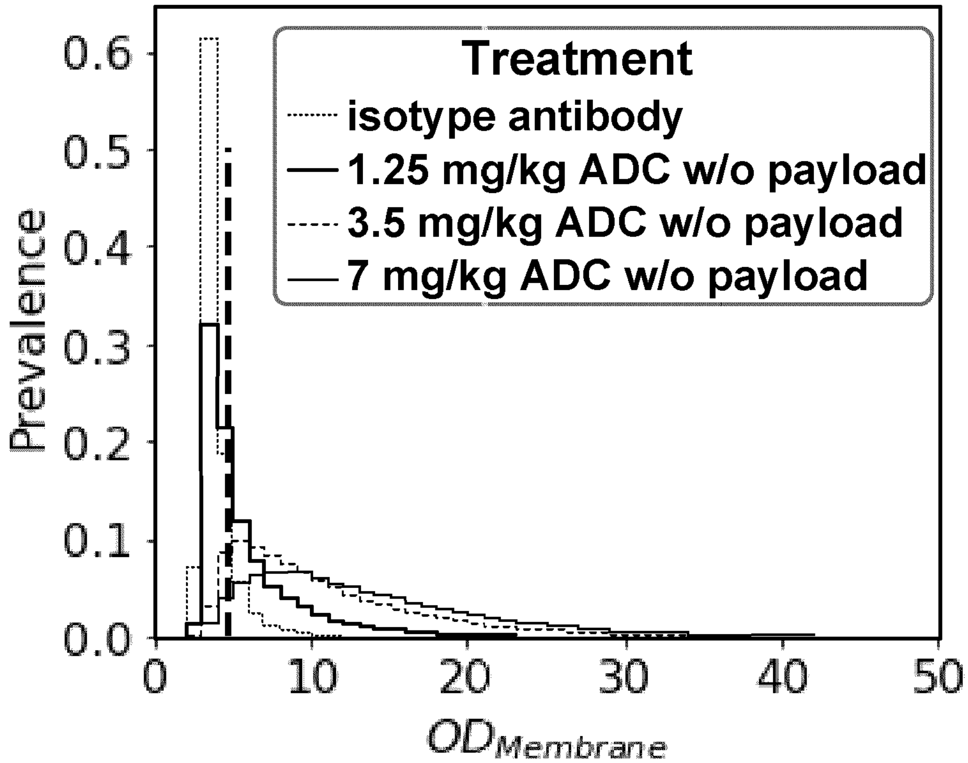


Maximum membrane optical density of bound IgG at various dose levels of E02-GL-SG3932

Clone sample #26

FIG. 42C

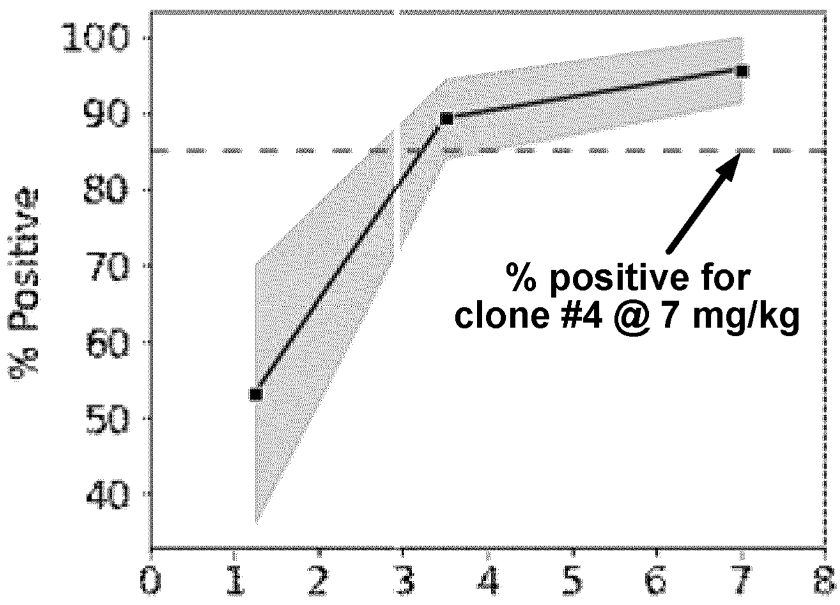


Clone sample #26

FIG. 42D

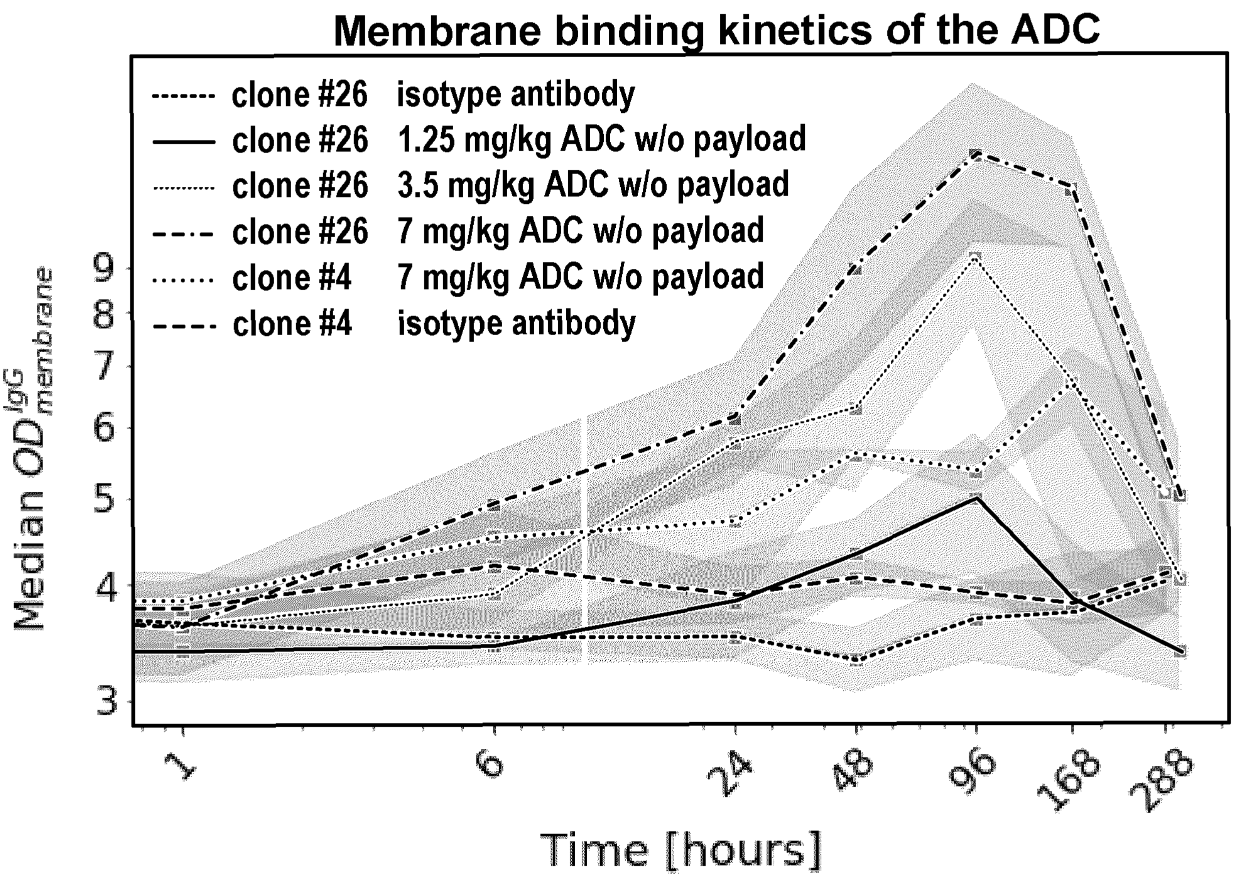


FIG. 43A

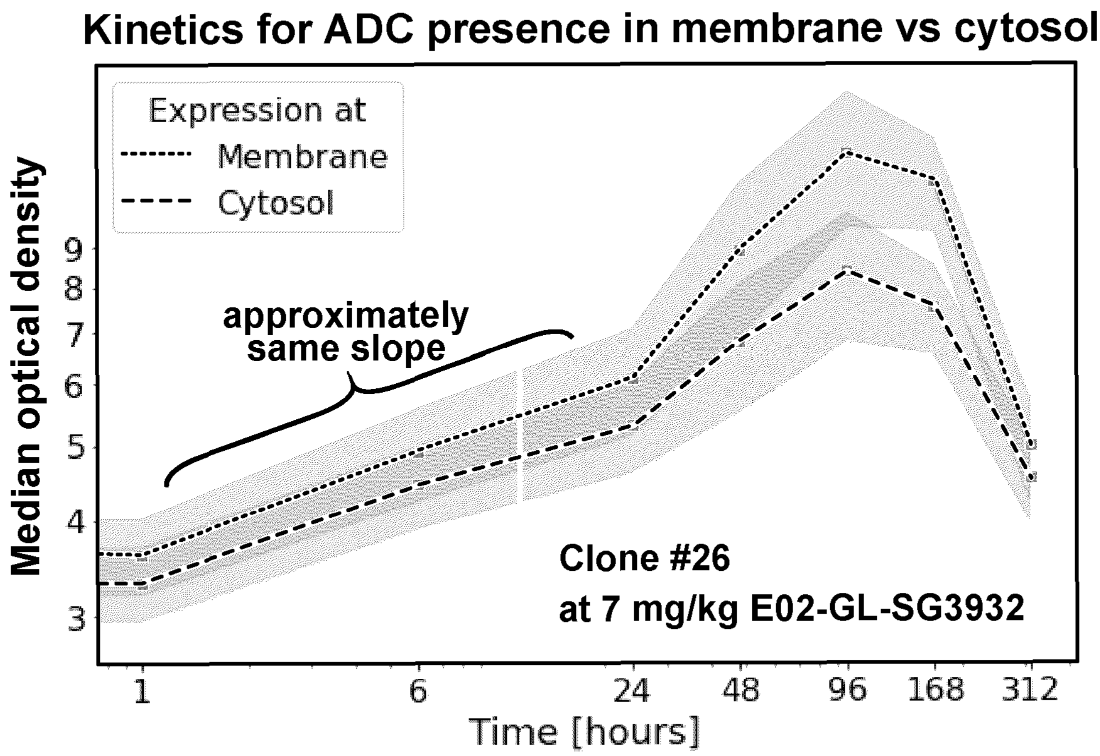


FIG. 43B

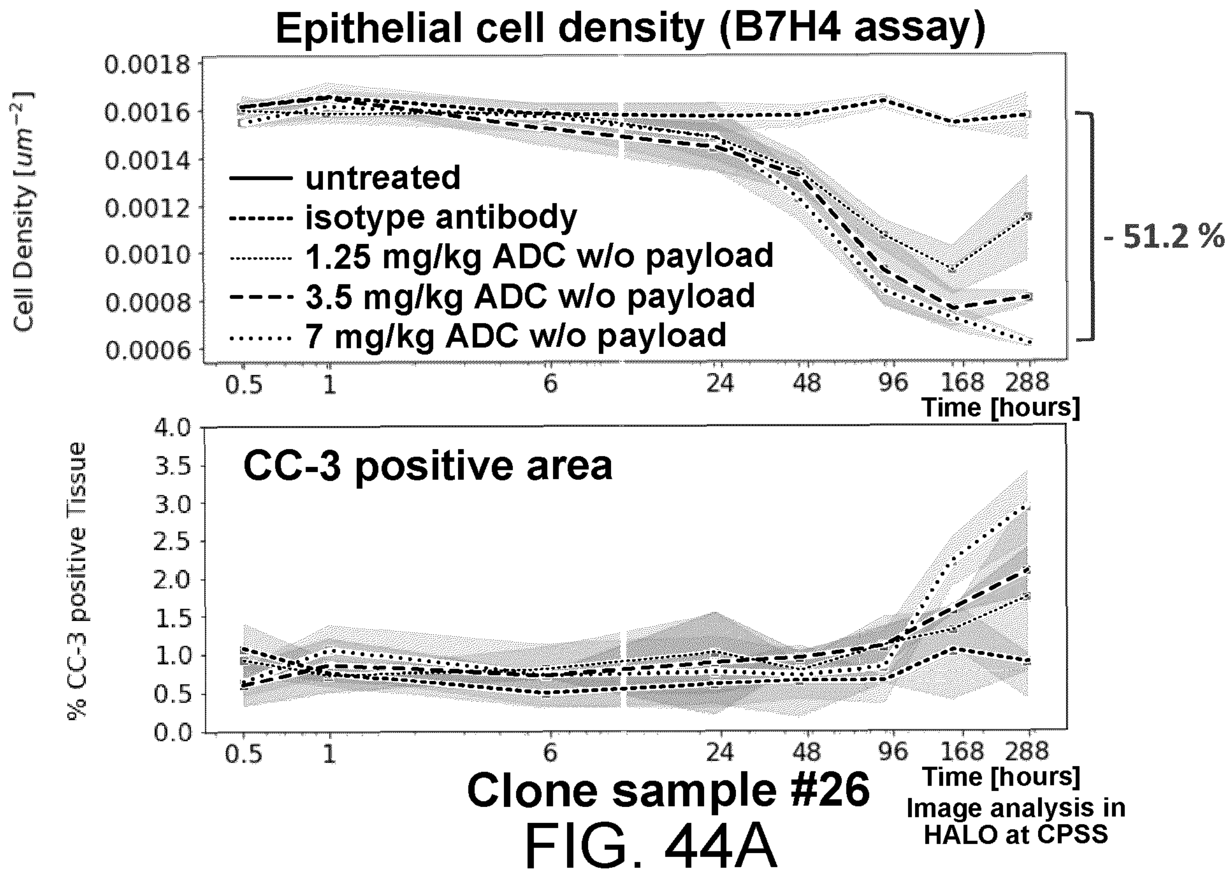


Clone sample #26

Image analysis in HALO at CPSS

FIG. 44A

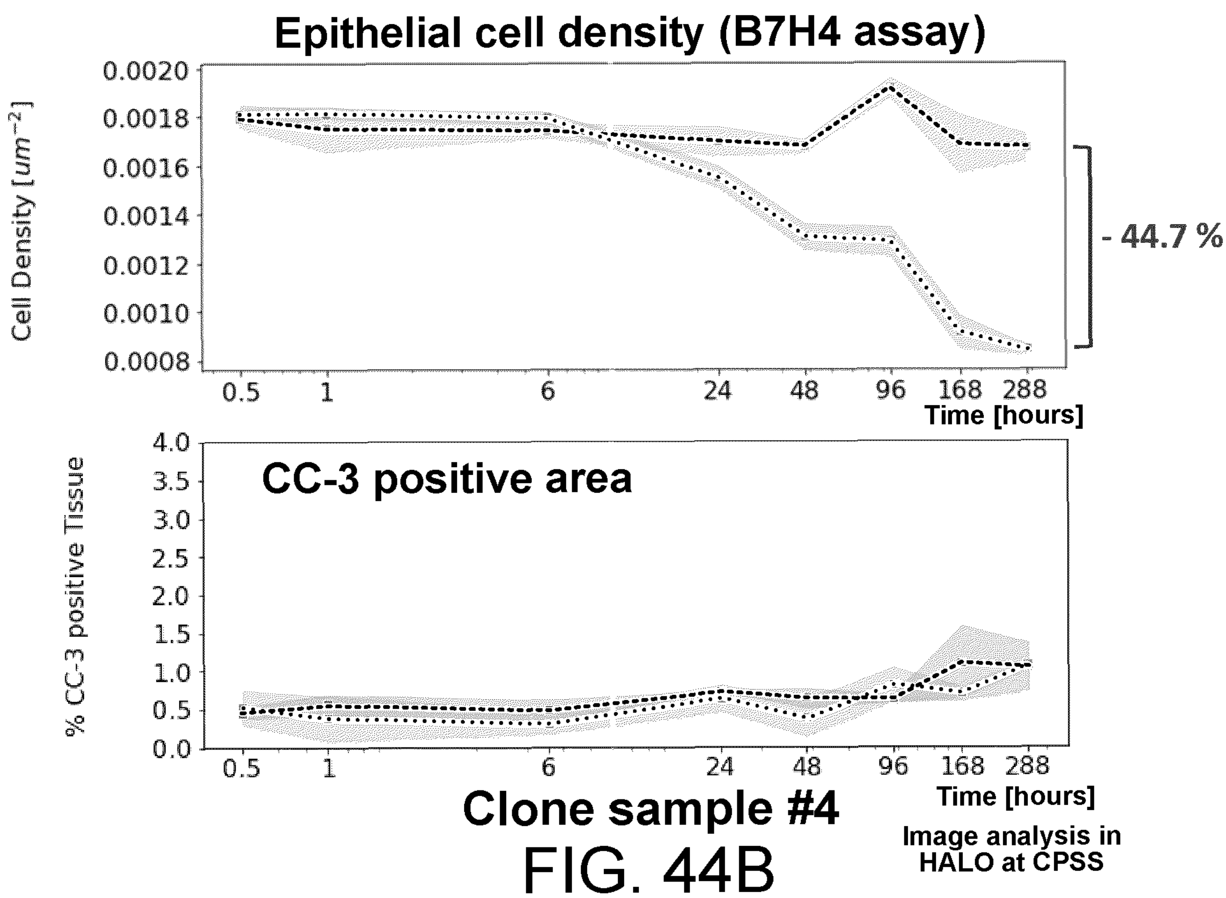


Clone sample #4

Image analysis in HALO at CPSS

FIG. 44B

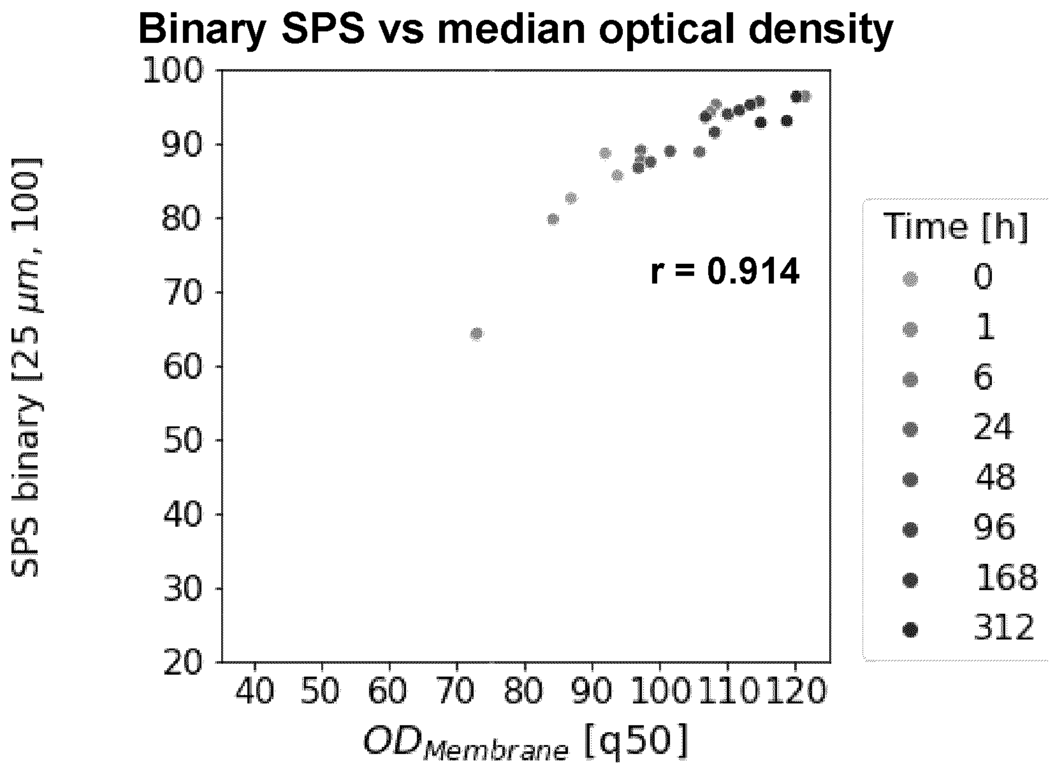


Clone sample #26

FIG. 45A

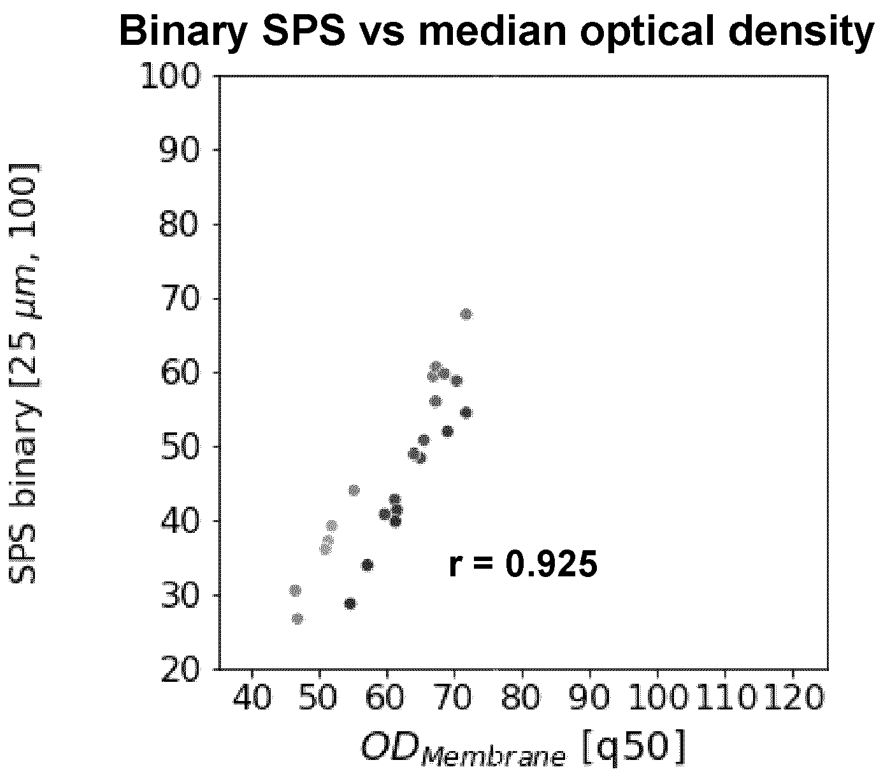


Clone sample #4

FIG. 45B

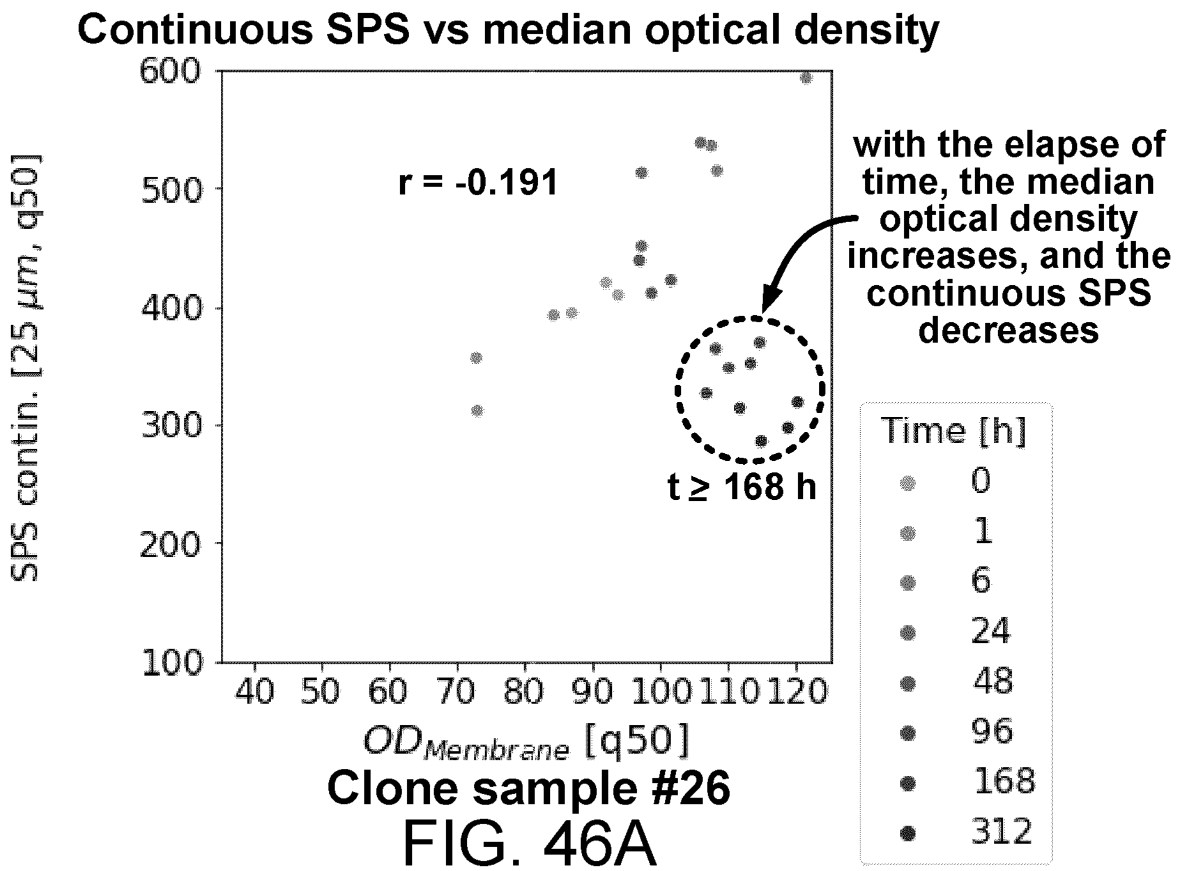


Clone sample #26

FIG. 46A

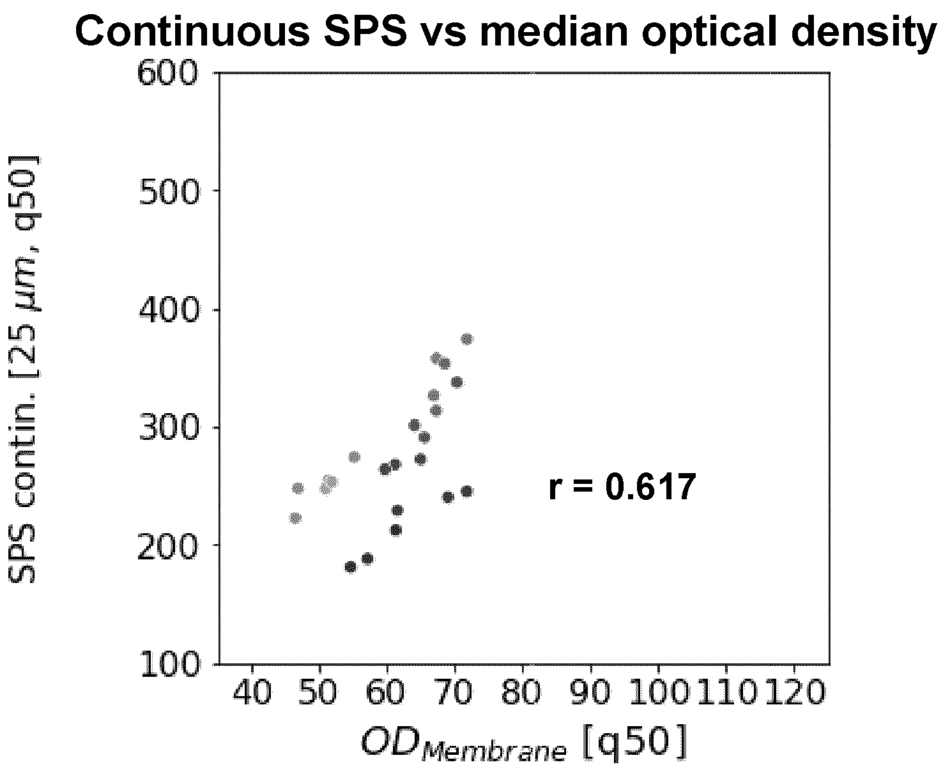


Clone sample #4

FIG. 46B

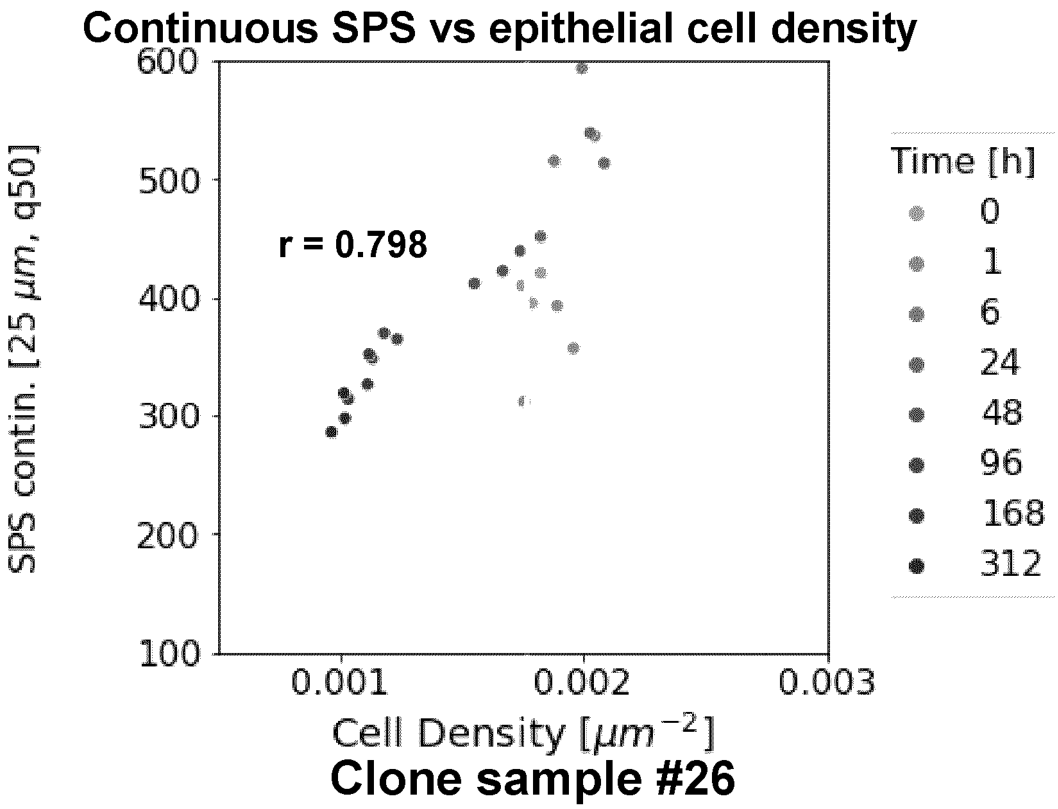


Clone sample #26

FIG. 47A

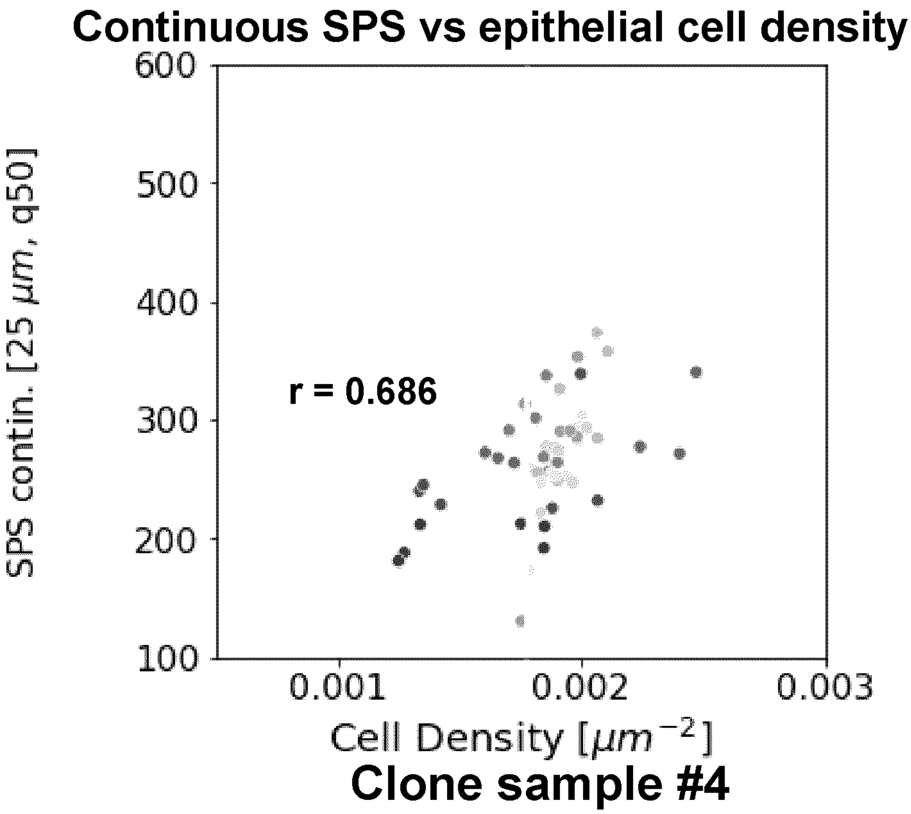


Clone sample #4

FIG. 47B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63077607
- WO 2015124777 A
- US 4816567 A
- US 5750373 A
- US 5969108 A
- US 6172197 B
- US 5885793 A
- US 6521404 B
- US 6544731 B
- US 6555313 B
- US 6582915 B
- US 6593081 B
- US 6300064 B
- US 6653068 B
- US 6706484 B
- US 7264963 B
- US 5639641 A
- US 5723323 A
- US 5976862 A
- US 5824514 A
- US 5817483 A
- US 5814476 A
- US 5763192 A
- US 5766886 A
- US 5714352 A
- US 6204023 B
- US 6180370 B
- US 5693762 A
- US 5530101 A
- US 5585089 A
- US 5225539 A
- US 7557189 B
- US 7538195 B
- US 7342110 B
- US 9816280 W
- US 9618978 W
- US 9109630 W
- US 9105939 W
- US 9401234 W
- GB 8901334 W
- GB 9101134 W
- GB 9201755 W
- WO 9014443 A
- WO 9014424 A
- WO 9014430 A
- EP 229246 A
- US 5545807 A
- US 5545806 A
- US 5569825 A
- US 5625126 A
- US 5633425 A
- US 5661016 A
- US 5641870 A
- US 4946778 A
- US 5693761 A


### Non-patent literature cited in the description


- **DUBOWCHIK et al.** *Bioconjugate Chemistry*, 2002, vol. 13, 855-869
- Greene's Protecting Groups in Organic Synthesis. John Wiley & Sons, 2007, 696-871
- **KABAT, E.A. et al.** Sequences of Proteins of Immunological Interest. U.S. Department of Health and Human Services, NIH Publication, 1991
- **CHOTHIA, C. et al.** *J. Mol. Biol.*, 1987, vol. 196, 901-917
- **WARD et al.** *Nature*, 1989, vol. 341, 544-546
- **BIRD et al.** *Science 1Al-ATi-Alβ*, 1988
- **HUSTON et al.** *Proc. Natl. Acad. ScL USA*, 1988, vol. 85, 5879-5883
- **GC HOWARD**. Making and Using Antibodies: A Practical Handbook. CRC Books., 2006
- **KOHLER** ; **MILSTEIN**. *Nature*, 1975, vol. 256, 495
- **GODING**. Monoclonal Antibodies: Principles and Practice. Academic Press, 1986
- **MCCAFFERTY et al.** *Nature*, 1990, vol. 348, 552-554
- **CLACKSON et al.** *Nature*, 1991, vol. 352, 624-628
- **MARKS et al.** *J. Mol. Biol.*, 1991, vol. 222, 581-597
- **COLE** ; **ALAN R. LISS et al.** *Monoclonal Antibodies and Cancer Therapy*, 1985, 77
- **BOEMER et al.** *J. Immunol.*, 1991, vol. 147 (1), 86-95
- **VAUGHAN et al.** *Nat. Biotech.*, 1996, vol. 14, 309-314
- **SHEETS et al.** *Proc. Natl. Acad. Sci. USA*, 1998, vol. 95, 6157-6162

- **HOOGENBOOM** ; **WINTER**. *J. Mol. Biol.*, 1991, vol. 227, 381
- **MARKS et al.** *J. Mol. Biol.*, 1991, vol. 222, 581
- **ROTHE et al.** *J. Molec. Biol.*, 2008, vol. 376, 1182-1200
- **MARKS et al.** *BioTechnology*, 1992, vol. 10, 779-783
- **JONES et al.** *Nature*, 1986, vol. 321, 522
- **RIECHMANN et al.** *Nature*, 1988, vol. 332, 323
- **VERHOEYEN et al.** *Science*, 1988, vol. 239, 1534
- **SIMS et al.** *J. Immunol.*, 1993, vol. 151, 2296
- **CHOTHIA** ; **LESK**. *J. Mol. Biol.*, 1987, vol. 196, 901
- **CARTER et al.** *Proc. Natl. Acad. Sci. USA*, 1992, vol. 89, 4285
- **PRESTA et al.** *J. Immunol.*, 1993, vol. 151, 2623
- **MORIMOTO et al.** *J. Biochem. Biophys. Meth.*, 1993, vol. 24, 107-117
- **BRENNAN et al.** *Science*, 1985, vol. 229, 81
- **HUSE et al.** *Science*, 1989, vol. 246, 1275-1281
- Remington's Pharmaceutical Sciences. 2012